(19) Europäisches Patentamt
European Patent Office
Office européen des brevets

(11) EP 1 789 561 B1

(12) **EUROPEAN PATENT SPECIFICATION**

(45) Date of publication and mention of the grant of the patent:
**06.01.2010 Bulletin 2010/01**

(21) Application number: **05776204.9**

(22) Date of filing: **14.07.2005**

(51) Int Cl.:
***C12N 15/82*** *(2006.01)*

(86) International application number:
**PCT/EP2005/053398**

(87) International publication number:
**WO 2006/008271 (26.01.2006 Gazette 2006/04)**

(54) **PLANTS HAVING IMPROVED GROWTH CHARACTERISTICS AND METHOD FOR MAKING THE SAME**

PFLANZEN MIT VERBESSERTEN WACHSTUMSEIGENSCHAFTEN UND VERFAHREN ZU DEREN HERSTELLUNG

PLANTES PRÉSENTANT DES CARACTÉRISTIQUES DE CROISSANCE MODIFIÉS ET PROCÉDÉ DE FABRICATION ASSOCIÉ

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.07.2004 EP 04103421**
**21.07.2004 US 589765 P**

(43) Date of publication of application:
**30.05.2007 Bulletin 2007/22**

(73) Proprietor: **CropDesign N.V.**
**9052 Zwijnaarde-Gent (BE)**

(72) Inventors:
- **FRANKARD, Valerie**
**B-1640 Sint-Genesius-Rode (BE)**
- **MIRONOV, Vladimir**
**B-9000 Gent (BE)**

(74) Representative: **Mistry, Meeta**
**BASF AG**
**Global Intellectual Property**
**GVX - C006**
**67056 Ludwigshafen (DE)**

(56) References cited:
**WO-A-01/02541 WO-A-01/07570**
**WO-A-98/05760**

- **COWAN A KEITH ET AL: "Fruit size: Towards an understanding of the metabolic control of fruit growth using avocado as a model system" PHYSIOLOGIA PLANTARUM, vol. 111, no. 2, February 2001 (2001-02), pages 127-136, XP008038987 ISSN: 0031-9317**
- **DICKINSON J R ET AL: "A CELL CYCLE ROLE FOR A PLANT SUCROSE NONFERMENTING-1-RELATED PROTEIN KINASE (SNRK1) IS INDICATED BY EXPRESSION IN YEAST" PLANT GROWTH REGULATION, DORDRECHT, NL, vol. 28, no. 3, July 1999 (1999-07), pages 169-174, XP008038990 ISSN: 0167-6903**
- **LAURIE S ET AL: "THE ROLE OF PROTEIN KINASES IN THE REGULATION OF PLANT GROWTH AND DEVELOPMENT" PLANT GROWTH REGULATION, DORDRECHT, NL, vol. 34, no. 3, July 2001 (2001-07), pages 253-265, XP009034127 ISSN: 0167-6903**
- **KOBAYASHI YUHKO ET AL: "Differential activation of the rice sucrose nonfermenting1-related protein kinase2 family by hyperosmotic stress and abscisic acid" PLANT CELL, vol. 16, no. 5, May 2004 (2004-05), pages 1163-1177, XP002305913 ISSN: 1040-4651 cited in the application**
- **HALFORD N G ET AL: "SNF1-RELATED PROTEIN KINASES: GLOBAL REGULATORS OF CARBON METABOLISM IN PLANTS?" PLANT MOLECULAR BIOLOGY, NIJHOFF PUBLISHERS, DORDRECHT, NL, vol. 37, no. 5, 1998, pages 735-748, XP000910411 ISSN: 0167-4412**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MUSTILLI ANNA-CHIARA ET AL: "Arabidopsis OST1 protein kinase mediates the regulation of stomatal aperture by abscisic acid and acts upstream of reactive oxygen species production." PLANT CELL, vol. 14, no. 12, December 2002 (2002-12), pages 3089-3099, XP002305914 ISSN: 1040-4651 cited in the application
- ANDERBERG ROBERT J ET AL: "Isolation of a wheat cDNA clone for an abscisic acid-inducible transcript with homology to protein kinases" PROCEEDINGS OF THE NATIONAL ACADEMY OF SCIENCES OF THE UNITED STATES OF AMERICA, vol. 89, no. 21, 1992, pages 10183-10187, XP002918247 ISSN: 0027-8424 cited in the application
- PARK Y S ET AL: "TWO PUTATIVE PROTEIN KINASES FROM ARABIDOPSIS THALIANA CONTAIN HIGHLY ACIDIC DOMAINS" PLANT MOLECULAR BIOLOGY, NIJHOFF PUBLISHERS, DORDRECHT, NL, vol. 22, 1993, pages 615-624, XP002060429 ISSN: 0167-4412 cited in the application & DATABASE EMBL SEQUENCE LIBRARY EBI,HINXTON; 1 November 1995 (1995-11-01), PARK, Y.S., ET AL.: "Two putative protein kinases from Arabidopsis thaliana contain highly acidic domains" retrieved from EBI.AC.UK Database accession no. P43291
- HRABAK ESTELLE M ET AL: "The arabidopsis CDPK-SnRK superfamily of liprotein kinases." PLANT PHYSIOLOGY (ROCKVILLE), vol. 132, no. 2, June 2003 (2003-06), pages 666-680, XP002305916 ISSN: 0032-0889

SEQUENCE LISTING

<110> CropDesign N.V.

<120> Plants having modified growth characteristics and method for making the same

<130> CD-119-prio

<150> EP 04103421.6
<151> 2004-07-16

<150> US 60/589, 765
<151> 2004-07-21

<160> 55

<170> PatentIn version 3.3

<210> 1
<211> 1092
<212> DNA
<213> Arabidopsis thaliana

<400> 1

```
atggacaagt acgagctggt gaaagacata ggtgctggga attttggagt tgccaggctc      60
atgaaggtca aaaactctaa ggaacttgtt gccatgaagt acatcgagcg tggtcctaag     120
attgatgaga atgtggcaag agagatcatt aatcacagat cacttcgcca tccgaatata     180
atccggttca aggaggtggt gttgactcca acccatcttg ccattgccat ggaatatgct     240
gctggtggtg aactattcga gcgtatatgc agtgctggaa gatttagtga ggatgaggcg     300
agatatttct tccagcagct tatatcaggt gttagctatt gccatgctat gcaaatatgc     360
catagagatc tgaagctcga gaatacgctc ttggatggaa gtcctgctcc acgtctgaaa     420
atctgtgatt ttggttattc caagtcctct ctgctgcact ctaggcccaa atcaacagtt     480
ggaactccag catatattgc acctgaggtc ctttctcgaa gagaatatga tggcaagatg     540
gctgatgtat ggtcttgtgg tgtgactctt tatgtcatgc tggttggagc atacccattt     600
gaagaccagg aagaccccaa gaacttcagg aaaacaatac aaaaaataat ggctgtccag     660
tacaagatcc cggactacgt ccatatctca caggattgta aaaatctcct ttcccgtata     720
tttgtcgcca attcactcaa gaggatcacc attgcagaaa tcaagaaaca ttcatggttc     780
ctaaagaatt tgccaaggga actcacagag acagctcaag ctgcatattt caagaaagag     840
aacccaacct tctcccttca gaccgttgaa gagatcatga agatagtggc tgacgccaaa     900
acaccgcctc ctgtttcccg atccatcgga ggttttggct ggggaggaaa tggggatgca     960
gatggaaaag aggaagatgc agaagacgtg gaggaggaag aggaggaggt ggaagaagag    1020
gaagacgatg aggatgaata cgataagact gtaaaggaag tacacgcaag tggagaagtg    1080
agaataagtt ga                                                        1092
```

<210> 2
<211> 363
<212> PRT
<213> Arabidopsis thaliana

<400> 2

```
Met Asp Lys Tyr Glu Leu Val Lys Asp Ile Gly Ala Gly Asn Phe Gly
1               5                   10                  15

Val Ala Arg Leu Met Lys Val Lys Asn Ser Lys Glu Leu Val Ala Met
            20                  25                  30

Lys Tyr Ile Glu Arg Gly Pro Lys Ile Asp Glu Asn Val Ala Arg Glu
        35                  40                  45
```

```
Ile Ile Asn His Arg Ser Leu Arg His Pro Asn Ile Ile Arg Phe Lys
    50                  55                  60

Glu Val Val Leu Thr Pro Thr His Leu Ala Ile Ala Met Glu Tyr Ala
65                  70                  75                  80

Ala Gly Gly Glu Leu Phe Glu Arg Ile Cys Ser Ala Gly Arg Phe Ser
                85                  90                  95

Glu Asp Glu Ala Arg Tyr Phe Phe Gln Gln Leu Ile Ser Gly Val Ser
            100                 105                 110

Tyr Cys His Ala Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
145                 150                 155                 160

Gly Thr Pro Ala Tyr Ile Ala Pro Glu Val Leu Ser Arg Arg Glu Tyr
                165                 170                 175

Asp Gly Lys Met Ala Asp Val Trp Ser Cys Gly Val Thr Leu Tyr Val
            180                 185                 190

Met Leu Val Gly Ala Tyr Pro Phe Glu Asp Gln Glu Asp Pro Lys Asn
        195                 200                 205

Phe Arg Lys Thr Ile Gln Lys Ile Met Ala Val Gln Tyr Lys Ile Pro
    210                 215                 220

Asp Tyr Val His Ile Ser Gln Asp Cys Lys Asn Leu Leu Ser Arg Ile
225                 230                 235                 240

Phe Val Ala Asn Ser Leu Lys Arg Ile Thr Ile Ala Glu Ile Lys Lys
                245                 250                 255

His Ser Trp Phe Leu Lys Asn Leu Pro Arg Glu Leu Thr Glu Thr Ala
            260                 265                 270

Gln Ala Ala Tyr Phe Lys Lys Glu Asn Pro Thr Phe Ser Leu Gln Thr
        275                 280                 285

Val Glu Glu Ile Met Lys Ile Val Ala Asp Ala Lys Thr Pro Pro Pro
    290                 295                 300

Val Ser Arg Ser Ile Gly Gly Phe Gly Trp Gly Gly Asn Gly Asp Ala
305                 310                 315                 320

Asp Gly Lys Glu Glu Asp Ala Glu Asp Val Glu Glu Glu Glu Glu Glu
                325                 330                 335

Val Glu Glu Glu Glu Asp Asp Glu Asp Glu Tyr Asp Lys Thr Val Lys
            340                 345                 350

Glu Val His Ala Ser Gly Glu Val Arg Ile Ser
```

355 360

<210> 3
<211> 3121
<212> DNA
<213> Arabidopsis thaliana

<400> 3

```
caaacatgta tggatcgcat ggaaacttgt gggtccctct ttcttttaaa aaatctcgta 60
ttaattaaat aaatggaaaa aaaaacatga tgggagtttg tttaggcagg ggagattctt 120
cttcattctc atcattattt ctctattaat ttcaccccaa aaaagaaaaa agaaaaattc 180
caacaagaaa aaaaaaagaa aaagaaagtt gattcttcgc ttaggcttga aatctctcca 240
atccaaatct caaattaacc ttccatcgtc atctctttcc cttttttttt cccactttct 300
ttgcgaatcg cgagatctcg gaatcgcatc cttgattttg ggatactgtt tttttttttt 360
ttaatcttgt ttcattttca cgtgaaattc ttagctgcta gaactggact tgaatttcaa 420
cgagaatttt ggagattttt tttttgtttg ggtttttcct ttctgttttg tgtgtttgga 480
attagggttg tcgagcgaga atggacaagt acgagctggt gaaagacata ggtgctggga 540
attttggagt tgccaggctc atgaaggtca aaaactctaa ggaacttgtt gccatgaagt 600
acatcgagcg tggtcctaag gtatattcct ctctgttttt gtgttttcat tgctctccat 660
gagctggtga tcctataccc agatatgcat aattggaatg aattgctatt aagcagaaga 720
gtcgattttt ttttgtaaat ttcttatcgt tagctgattg ggtgtttaac gtaacgttag 780
ttatcttgta gttgtaatat ttttcctgaa aagatttgta caatgagtat ttgctttgtt 840
tgtttttttt gatacagatt gatgagaatg tggcaagaga gatcattaat cacagatcac 900
ttcgccatcc gaatataatc cggttcaagg aggttagtga atttcttgtt gcttgacatg 960
ggtggtgttc ttgctatgaa aaagtttgtt gataatctct tatatctttc atcttgcatt 1020
ccttgttggg tttatggatt ttataggtgg tgttgactcc aacccatctt gccattgcca 1080
tggaatatgc tgctggtggt gaactattcg agcgtatatg cagtgctgga agatttagtg 1140
aggatgaggt gagcttgcca tttgaaaaat tgtgctgtgc ttttgcgaat atgaaattac 1200
tagtatttag gataatctgc atggtctttg gaaagattag gaggaaggga acaagagaaa 1260
acatgtgaac ctccttttat ttagtatcag gcattaaaca gttagggtct acgttctaat 1320
cctttctctc ttttccaggc gagatatttc ttccagcagc ttatatcagg tgttagctat 1380
tgccatgcta tggtaatgta gagacaatga cttaagcaaa atttacttat ccattggctg 1440
tttgaagtcg ttttttttta atcatgtgtt gactattttg ttgcagcaaa tatgccatag 1500
agatctgaag ctcgagaata cgctcttgga tggaagtcct gctccacgtc tgaaaatctg 1560
tgattttggt tattccaagg tctgacacta aaaaaaaatc caagttcccc ccttgtcgac 1620
gagatcctct tttgtgattt gttattctct tttttttagt cctctctgct gcactctagg 1680
cccaaatcaa cagttggaac tccagcatat attgcacctg aggtcctttc tcgaagagaa 1740
tatgatggca aggtaatcaa gcatcatgca caatgcaatg aacttccata aacccatgag 1800
tatttatgat attgtcatgc tctttacatt tttacttttg aatttaaaaa gtcatctttg 1860
tggaagtcgc taagatttga agcatttttt cttctttcag atggctgatg tatggtcttg 1920
tggtgtgact ctttatgtca tgctggttgg agcataccca tttgaagacc aggaagaccc 1980
caagaacttc aggaaaacaa tacaagtagg tttctttttt gaagccatgt atctgcatat 2040
ctcgctttcg ccacatccta ttcgtcaatg tgtgatcttg ttatacagaa aataatggct 2100
gtccagtaca agatcccgga ctacgtccat atctcacagg attgtaaaaa tctccctttcc 2160
cgtatatttg tcgccaattc actcaaggta tacatcaatc aactgaacta aatgttttca 2220
aagatgcctt ttgatttttc tgaacaattg agctacttgt tgtttcgtag aggatcacca 2280
ttgcagaaat caagaaacat tcatggttcc taaagaattt gccaagggaa ctcacagaga 2340
cagctcaagc tgcatatttc aagaaagaga acccaacctt ctcccttcag accgttgaag 2400
agatcatgaa gatagtggct gacgccaaaa caccgcctcc tgtttcccga tccatcggag 2460
gttttggctg gggaggaaat ggggatgcag atggaaaaga ggaagatgca gaagacgtgg 2520
aggaggaaga ggaggaggtg gaagaagagg aagacgatga ggatgaatac gataagactg 2580
taaaggaagt acacgcaagt ggagaagtga gaataagttg atattttggt ttttggtctg 2640
tgtaagaaag aagtcgtcgt tggtttgttg aaactgaaaa gtctctgttc tcgtgtttgc 2700
ctttacaatg ctttggctaa ggttttggtt ctggttttgg agatttgtaa aatttgcagt 2760
ataagatgaa caaacagaga ggttgatgat gagaatgagt cctttgctac gcatggtact 2820
atgaacattg tgacctccaa taaatatttt tgtaaattag attttatttt ccgaaaagat 2880
tcatgtattt gatttttgga tttcttattt ttattttttt tcgttcctta tcattttttt 2940
gaaaatgcaa atctataaaa tacaaatgtc aacaaaaaat caaattgaaa tgttcggaat 3000
tcaaaaataa ttgttttctt ttgttttttt gtttctgatg cgaaatgtga atatattaga 3060

gggaaaatat cccgccatta ggaaaccgga taatcttcta cggccttgag ctcaagtcgg 3120
t 3121
```

<210> 4
<211> 54
<212> DNA
<213> Artificial sequence

<220>
<223> forward primer: prm02295

<400> 4
ggggacaagt ttgtacaaaa aagcaggctt cacaatggac aagtacgagc tggt 54

<210> 5
<211> 51
<212> DNA
<213> Artificial sequence

<220>
<223> reverse primer: prm02296

<400> 5
ggggaccact ttgtacaaga aagctgggtc gacgacttct ttcttacaca g 51

<210> 6
<211> 9
<212> PRT
<213> Artificial sequence

<220>
<223> conserved signature sequence

<220>
<221> MISC FEATURE
<222> (2)..(2)
<223> Xaa can be Phe or Tyr

<220>
<221> MISC_FEATURE

<222> (3)..(3)
<223> Xaa can be Leu, Met or Thr

<220>
<221> MISC FEATURE
<222> (5)..(5)
<223> Xaa can be Asn, Gly or Arg

<220>
<221> MISC_FEATURE
<222> (6)..(6)
<223> Xaa can be Leu, Pro or Ile

<220>
<221> MISC FEATURE
<222> (7)..(7)
<223> Xaa can be Pro or Leu

<220>
<221> MISC FEATURE
<222> (8)..(8)
<223> Xaa can be Ala, Gly, Val, Arg, Lys or Ile

<220>
<221> MISC FEATURE

<222> (9)..(9)
<223> Xaa can be Asp, Glu or Val

<400> 6

```
Trp Xaa Xaa Lys Xaa Xaa Xaa Xaa Xaa
1               5
```

<210> 7
<211> 1086
<212> DNA
<213> Arabidopsis thaliana

<400> 7

```
atggacaagt acgagcttgt taaagacatc ggtgctggga attttggagt ggcgaggctc     60
atgagagtca aaaactccaa ggaactcgtt gctatgaagt acatcgagcg tggacctaag    120
attgatgaga acgtggcgag agagattatt aaccacagat cacttcgtca tcccaatatt    180
atccggttta aggaggtggt tttgacacca acgcacatcg ccattgctat ggaatatgct    240
gctggcggtg agctatttga gcgtatatgt agcgctggaa gattcagtga ggatgaggca    300
agatactttt tccagcagct tatctcagga gtcagctatt gtcatgctat gcaaatatgc    360
cacagagatc tgaagcttga aaataccctc ttagatggaa gtcctgctcc acgcctgaag    420
atctgtgatt ttggttattc caagtcctca ctgttgcact ctatgcccaa atcaactgtt    480
ggaactccag catatattgc acctgaggtt ctttctcgcg gagagtatga tggcaagatg    540
gctgatgtat ggtcttgtgg tgtgactctt tatgtcatgc tggtgggagc atacccattt    600
gaagaccaag aggatcccaa aaacttcaaa aaaacaatac aaagaataat ggctgtcaag    660
tacaagatcc cggactatgt ccatatctca caagattgca aacatctcct ctcccgtata    720
tttgtcacca actcgaataa gaggattacg ataggtgaca tcaagaaaca tccatggttc    780
ctaaagaacc tgccaaggga acttacagaa atagctcaag ctgcatactt caggaaagag    840
aacccgacat tctcactcca aagcgtcgaa gagataatga agattgtgga agaggcaaaa    900
actccagctc gtgtttctcg gtcgattgga gcatttgggt ggggaggagg agaagatgcc    960
gagggcaagg aggaagatgc agaggaagaa gttgaggaag tagaagaaga agaagacgaa   1020
gaagatgagt atgataagac ggtgaagcaa gtgcatgcta gcatgggaga agtccgagtc   1080
agttaa                                                              1086
```

<210> 8
<211> 361
<212> PRT
<213> Arabidopsis thaliana

<400> 8

```
Met Asp Lys Tyr Glu Leu Val Lys Asp Ile Gly Ala Gly Asn Phe Gly
1               5                   10                  15

Val Ala Arg Leu Met Arg Val Lys Asn Ser Lys Glu Leu Val Ala Met
            20                  25                  30

Lys Tyr Ile Glu Arg Gly Pro Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Val Leu Thr Pro Thr His Ile Ala Ile Ala Met Glu Tyr Ala
65                  70                  75                  80
```

```
Ala Gly Gly Glu Leu Phe Glu Arg Ile Cys Ser Ala Gly Arg Phe Ser
                85                  90                  95

Glu Asp Glu Ala Arg Tyr Phe Phe Gln Gln Leu Ile Ser Gly Val Ser
            100                 105                 110

Tyr Cys His Ala Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Met Pro Lys Ser Thr Val
145                 150                 155                 160

Gly Thr Pro Ala Tyr Ile Ala Pro Glu Val Leu Ser Arg Gly Glu Tyr
                165                 170                 175

Asp Gly Lys Met Ala Asp Val Trp Ser Cys Gly Val Thr Leu Tyr Val
            180                 185                 190

Met Leu Val Gly Ala Tyr Pro Phe Glu Asp Gln Glu Asp Pro Lys Asn
        195                 200                 205

Phe Lys Lys Thr Ile Gln Arg Ile Met Ala Val Lys Tyr Lys Ile Pro
    210                 215                 220

Asp Tyr Val His Ile Ser Gln Asp Cys Lys His Leu Leu Ser Arg Ile
225                 230                 235                 240

Phe Val Thr Asn Ser Asn Lys Arg Ile Thr Ile Gly Asp Ile Lys Lys
                245                 250                 255

His Pro Trp Phe Leu Lys Asn Leu Pro Arg Glu Leu Thr Glu Ile Ala
            260                 265                 270

Gln Ala Ala Tyr Phe Arg Lys Glu Asn Pro Thr Phe Ser Leu Gln Ser
        275                 280                 285

Val Glu Glu Ile Met Lys Ile Val Glu Glu Ala Lys Thr Pro Ala Arg
    290                 295                 300

Val Ser Arg Ser Ile Gly Ala Phe Gly Trp Gly Gly Gly Glu Asp Ala
305                 310                 315                 320

Glu Gly Lys Glu Glu Asp Ala Glu Glu Glu Val Glu Glu Val Glu Glu
                325                 330                 335

Glu Glu Asp Glu Glu Asp Glu Tyr Asp Lys Thr Val Lys Gln Val His
            340                 345                 350

Ala Ser Met Gly Glu Val Arg Val Ser
        355                 360
```

<210> 9
<211> 1083
<212> DNA
<213> Arabidopsis thaliana

<400> 9

```
atggacaagt atgaggttgt gaaggatttg ggagctggaa attttggtgt ggctcgtctt     60
cttagacaca aagagaccaa agagctcgtt gctatgaaat acattgagag aggtcgcaag    120
attgatgaga atgtggcaag agagattatc aatcatagat cacttaggca tcctaatatc    180
atcagattca aggaggtgat tctgactcca actcatcttg caattgtaat ggagtatgct    240
tctggaggag agctctttga aagaatctgt aatgctggta gattcagtga agctgaggct    300
agatacttct ttcagcagct gatttgtggc gtggattact gtcattcact gcaaatatgt    360
catagagatt tgaagcttga gaatacactg cttgatggta gtccagcccc gcttttgaaa    420
atctgtgatt ttggttactc caagtcatct ctgcttcact ctagacctaa atcaactgtt    480
ggtactccag cttatatcgc acctgaagtt ctttcccgaa gagaatatga cggaaagcat    540
gcggatgttt ggtcctgtgg tgtgactctt tatgtgatgt tagttggagg ttatccgttt    600
gaagacccgg atgatccgag aaacttcagg aaaacaatcc aacgtataat ggctgtccag    660
tacaagatcc cggattacgt tcatatatcg caggagtgca gacaccttct ctctcgcata    720
tttgtcacta attcagctaa gagaatcaca cttaaagaga tcaagaagca tccatggtac    780
ttaaagaact tgccaaagga gcttacagag cctgctcaag cggcgtacta caagagagaa    840
accccaagct tttccctcca aagcgtagag gacataatga agatcgttgg agaagccagg    900
aatccagctc cgtcttctaa tgccgtcaag ggctttgatg atgatgagga agatgtggag    960
gacgaggttg aagaagaaga agaagaagaa gaagaagagg aggaagaaga ggaagaggaa   1020
gaagatgaat acgagaagca tgttaaagag gcccattctt gtcaagagcc tcccaaagct   1080
taa                                                                 1083
```

<210> 10
<211> 360
<212> PRT
<213> Arabidopsis thaliana

<400> 10

```
Met Asp Lys Tyr Glu Val Val Lys Asp Leu Gly Ala Gly Asn Phe Gly
1               5                   10                  15

Val Ala Arg Leu Leu Arg His Lys Glu Thr Lys Glu Leu Val Ala Met
            20                  25                  30

Lys Tyr Ile Glu Arg Gly Arg Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Ile Leu Thr Pro Thr His Leu Ala Ile Val Met Glu Tyr Ala
65                  70                  75                  80

Ser Gly Gly Glu Leu Phe Glu Arg Ile Cys Asn Ala Gly Arg Phe Ser
                85                  90                  95

Glu Ala Glu Ala Arg Tyr Phe Phe Gln Gln Leu Ile Cys Gly Val Asp
            100                 105                 110

Tyr Cys His Ser Leu Gln Ile Cys His Arg Asp Leu Lys Leu Glu Asn
        115                 120                 125

Thr Leu Leu Asp Gly Ser Pro Ala Pro Leu Leu Lys Ile Cys Asp Phe
    130                 135                 140

Gly Tyr Ser Lys Ser Ser Leu Leu His Ser Arg Pro Lys Ser Thr Val
145                 150                 155                 160

Gly Thr Pro Ala Tyr Ile Ala Pro Glu Val Leu Ser Arg Arg Glu Tyr
```

```
                    165                     170                     175

Asp Gly Lys His Ala Asp Val Trp Ser Cys Gly Val Thr Leu Tyr Val
            180                     185                     190

Met Leu Val Gly Gly Tyr Pro Phe Glu Asp Pro Asp Asp Pro Arg Asn
        195                     200                     205

Phe Arg Lys Thr Ile Gln Arg Ile Met Ala Val Gln Tyr Lys Ile Pro
    210                     215                     220

Asp Tyr Val His Ile Ser Gln Glu Cys Arg His Leu Leu Ser Arg Ile
225                     230                     235                     240

Phe Val Thr Asn Ser Ala Lys Arg Ile Thr Leu Lys Glu Ile Lys Lys
                245                     250                     255

His Pro Trp Tyr Leu Lys Asn Leu Pro Lys Glu Leu Thr Glu Pro Ala
            260                     265                     270

Gln Ala Ala Tyr Tyr Lys Arg Glu Thr Pro Ser Phe Ser Leu Gln Ser
        275                     280                     285

Val Glu Asp Ile Met Lys Ile Val Gly Glu Ala Arg Asn Pro Ala Pro
    290                     295                     300

Ser Ser Asn Ala Val Lys Gly Phe Asp Asp Asp Glu Glu Asp Val Glu
305                     310                     315                     320

Asp Glu Val Glu Glu Glu Glu Glu Glu Glu Glu Glu Glu Glu Glu Glu
                325                     330                     335

Glu Glu Glu Glu Glu Asp Glu Tyr Glu Lys His Val Lys Glu Ala His
            340                     345                     350

Ser Cys Gln Glu Pro Pro Lys Ala
        355                     360
```

<210> 11
<211> 1062
<212> DNA
<213> Arabidopsis thaliana

<400> 11

```
atggacaagt atgacgttgt caaggatctg ggagctggaa atttcggtgt ggctcgcctt     60
ctcaggcaca aggacaccaa agagcttgtt gccatgaaat acatcgagag aggtcgcaag    120
atagatgaga acgtggcgag agagattatt aatcacagat cacttaaaca tcctaatatc    180
atccggttca aggaggtgat cctgacacct actcatcttg ctattgtgat ggagtatgct    240
tctggaggag agctctttga tcgaatctgt actgccggta gatttagtga agctgaggct    300
aggtacttct ttcaacagct gatttgtggt gttgattact gccattcctt gcaaatatgt    360
catagagacc tgaagcttga gaacacactg ctcgatggga gccctgctcc gcttttgaaa    420
atctgtgatt ttggttactc taagtcatct atactacatt ctaggcctaa atcaactgtt    480
ggaactccag cttacatagc acctgaagtt ctttcacgga gagaatatga tggcaagcac    540
gcggatgtgt ggtcatgtgg agtaaccctt tatgtgatgc tggtgggagc ttacccgttt    600
gaggacccta atgatccaaa aaacttcagg aaaacaatcc agcgcataat ggctgtacaa    660
tacaagatcc cggactatgt tcacatatct caggaatgca aacatcttct ctctcgcata    720
ttcgtcacta actctgctaa gagaatcaca cttaaggaga tcaagaatca tccgtggtac    780
ttgaagaatt tgccaaagga gctgctagag tcggctcaag cggcgtatta caagagagac    840
acaagcttct ctcttcaaag cgtagaggac ataatgaaga tagttggaga agcaaggaat    900

ccagctccat caactagtgc tgtcaaaagc tcgggctcag gagctgatga agaagaggaa    960
gaggacgttg aagctgaagt ggaagaggaa gaagatgatg aagacgaata cgagaagcat   1020
gtcaaagagg cacagtcttg tcaagagtct gacaaagctt aa                      1062
```

<210> 12
<211> 353
<212> PRT
<213> Arabidopsis thaliana

<400> 12

```
Met Asp Lys Tyr Asp Val Val Lys Asp Leu Gly Ala Gly Asn Phe Gly
1               5                   10                  15

Val Ala Arg Leu Leu Arg His Lys Asp Thr Lys Glu Leu Val Ala Met
            20                  25                  30

Lys Tyr Ile Glu Arg Gly Arg Lys Ile Asp Glu Asn Val Ala Arg Glu
        35                  40                  45

Ile Ile Asn His Arg Ser Leu Lys His Pro Asn Ile Ile Arg Phe Lys
    50                  55                  60

Glu Val Ile Leu Thr Pro Thr His Leu Ala Ile Val Met Glu Tyr Ala
65                  70                  75                  80

Ser Gly Gly Glu Leu Phe Asp Arg Ile Cys Thr Ala Gly Arg Phe Ser
                85                  90                  95

Glu Ala Glu Ala Arg Tyr Phe Phe Gln Gln Leu Ile Cys Gly Val Asp
            100                 105                 110

Tyr Cys His Ser Leu Gln Ile Cys His Arg Asp Leu Lys Leu Glu Asn
        115                 120                 125

Thr Leu Leu Asp Gly Ser Pro Ala Pro Leu Leu Lys Ile Cys Asp Phe
    130                 135                 140

Gly Tyr Ser Lys Ser Ser Ile Leu His Ser Arg Pro Lys Ser Thr Val
145                 150                 155                 160

Gly Thr Pro Ala Tyr Ile Ala Pro Glu Val Leu Ser Arg Arg Glu Tyr
                165                 170                 175

Asp Gly Lys His Ala Asp Val Trp Ser Cys Gly Val Thr Leu Tyr Val
            180                 185                 190

Met Leu Val Gly Ala Tyr Pro Phe Glu Asp Pro Asn Asp Pro Lys Asn
        195                 200                 205

Phe Arg Lys Thr Ile Gln Arg Ile Met Ala Val Gln Tyr Lys Ile Pro
    210                 215                 220

Asp Tyr Val His Ile Ser Gln Glu Cys Lys His Leu Leu Ser Arg Ile
225                 230                 235                 240

Phe Val Thr Asn Ser Ala Lys Arg Ile Thr Leu Lys Glu Ile Lys Asn
                245                 250                 255

His Pro Trp Tyr Leu Lys Asn Leu Pro Lys Glu Leu Leu Glu Ser Ala
```

260 265 270

Gln Ala Ala Tyr Tyr Lys Arg Asp Thr Ser Phe Ser Leu Gln Ser Val
275 280 285

Glu Asp Ile Met Lys Ile Val Gly Glu Ala Arg Asn Pro Ala Pro Ser
290 295 300

Thr Ser Ala Val Lys Ser Ser Gly Ser Gly Ala Asp Glu Glu Glu Glu
305 310 315 320

Glu Asp Val Glu Ala Glu Val Glu Glu Glu Glu Asp Asp Glu Asp Glu
325 330 335

Tyr Glu Lys His Val Lys Glu Ala Gln Ser Cys Gln Glu Ser Asp Lys
340 345 350

Ala

<210> 13
<211> 1089
<212> DNA
<213> Arabidopsis thaliana

<400> 13

```
atggatcgac cagcagtgag tggtccaatg gatttgccga ttatgcacga tagtgatagg     60
tatgaactcg tcaaggatat tggctccggt aattttggag ttgcgagatt gatgagagac    120
aagcaaagta atgagcttgt tgctgttaaa tatatcgaga gaggtgagaa gatagatgaa    180
aatgtaaaaa gggagataat caaccacagg tccttaagac atcccaatat cgttagattc    240
aaagaggtta tattaacacc aacccattta gccattgtta tggaatatgc atctggagga    300
gaacttttcg agcgaatctg caatgcaggc cgcttcagcg aagacgaggc gaggtttttc    360
ttccagcaac tcatttcagg agttagttac tgtcatgcta tgcaagtatg tcaccgagac    420
ttaaagctcg agaatacgtt attagatggt agcccggccc ctcgtctaaa gatatgtgat    480
ttcggatatt ctaagtcatc agtgttacat tcgcaaccaa aatcaactgt tggaactcct    540
gcttacatcg ctcctgaggt tttactaaag aaagaatatg atggaaaggt tgcagatgtt    600
tggtcttgtg ggttactct gtatgtcatg ctggttggag catatccttt cgaagatccc    660
gaggaaccaa agaatttcag gaaaactata catagaatcc tgaatgttca gtatgctatt    720
ccggattatg ttcacatatc tcctgaatgt cgccatttga tctccagaat atttgttgct    780
gaccctgcaa agaggatatc aattcctgaa ataaggaacc atgaatggtt tctaaagaat    840
ctaccggcag atctaatgaa cgataacacg atgaccactc agtttgatga atcggatcaa    900
ccgggccaaa gcatagaaga aattatgcag atcattgcag aagcaactgt tcctcctgca    960
ggcactcaga atctgaacca ttacctcaca ggaagcttgg acatagatga cgatatggag   1020
gaagacttag agagcgacct tgatgatctt gacatcgaca gtagcggaga gattgtgtac   1080
gcaatgtga                                                           1089
```

<210> 14
<211> 362
<212> PRT
<213> Arabidopsis thaliana

<400> 14

```
Met Asp Arg Pro Ala Val Ser Gly Pro Met Asp Leu Pro Ile Met His
1               5                   10                  15

Asp Ser Asp Arg Tyr Glu Leu Val Lys Asp Ile Gly Ser Gly Asn Phe
            20                  25                  30

Gly Val Ala Arg Leu Met Arg Asp Lys Gln Ser Asn Glu Leu Val Ala
```

35 40 45

Val Lys Tyr Ile Glu Arg Gly Glu Lys Ile Asp Glu Asn Val Lys Arg
50 55 60

Glu Ile Ile Asn His Arg Ser Leu Arg His Pro Asn Ile Val Arg Phe
65 70 75 80

Lys Glu Val Ile Leu Thr Pro Thr His Leu Ala Ile Val Met Glu Tyr
85 90 95

Ala Ser Gly Gly Glu Leu Phe Glu Arg Ile Cys Asn Ala Gly Arg Phe
100 105 110

Ser Glu Asp Glu Ala Arg Phe Phe Phe Gln Gln Leu Ile Ser Gly Val
115 120 125

Ser Tyr Cys His Ala Met Gln Val Cys His Arg Asp Leu Lys Leu Glu
130 135 140

Asn Thr Leu Leu Asp Gly Ser Pro Ala Pro Arg Leu Lys Ile Cys Asp
145 150 155 160

Phe Gly Tyr Ser Lys Ser Ser Val Leu His Ser Gln Pro Lys Ser Thr
165 170 175

Val Gly Thr Pro Ala Tyr Ile Ala Pro Glu Val Leu Leu Lys Lys Glu
180 185 190

Tyr Asp Gly Lys Val Ala Asp Val Trp Ser Cys Gly Val Thr Leu Tyr
195 200 205

Val Met Leu Val Gly Ala Tyr Pro Phe Glu Asp Pro Glu Glu Pro Lys
210 215 220

Asn Phe Arg Lys Thr Ile His Arg Ile Leu Asn Val Gln Tyr Ala Ile
225 230 235 240

Pro Asp Tyr Val His Ile Ser Pro Glu Cys Arg His Leu Ile Ser Arg
245 250 255

Ile Phe Val Ala Asp Pro Ala Lys Arg Ile Ser Ile Pro Glu Ile Arg
260 265 270

Asn His Glu Trp Phe Leu Lys Asn Leu Pro Ala Asp Leu Met Asn Asp
275 280 285

Asn Thr Met Thr Thr Gln Phe Asp Glu Ser Asp Gln Pro Gly Gln Ser
290 295 300

Ile Glu Glu Ile Met Gln Ile Ile Ala Glu Ala Thr Val Pro Pro Ala
305 310 315 320

Gly Thr Gln Asn Leu Asn His Tyr Leu Thr Gly Ser Leu Asp Ile Asp
325 330 335

Asp Asp Met Glu Glu Asp Leu Glu Ser Asp Leu Asp Asp Leu Asp Ile
340 345 350

```
Asp Ser Ser Gly Glu Ile Val Tyr Ala Met
        355                 360
```

<210> 15
<211> 1020
<212> DNA
<213> Arabidopsis thaliana

<400> 15

```
atggagaagt atgagatggt gaaggattta ggatttggta atttcggatt ggctcggctt      60
atgcgtaata agcaaacaaa cgagcttgtg gctgtcaaat tcatcgatcg aggctacaag     120
atagatgaga acgttgcaag agaaataatc aatcatagag ctctcaacca tccgaatatt     180
gttcggttta aagaggttgt tttaactccg acacatcttg gaatagtaat ggagtatgca     240
gctggaggag aactgttcga gcggatatct agcgtgggtc gatttagcga agctgaggca     300
agatatttct ttcaacaact catttgtgga gtccattact tacatgcatt gcaaatatgc     360
catagagatc tgaaattaga aaacacattg cttgatggaa gcccagcacc acgtttaaaa     420
atttgtgatt ttggctactc aaagtcttct gttctgcact ccaacccaaa atcaacggtg     480
ggaactccgg catatatagc accggaagtt ttttgtcgat cggaatacga cggaaagtca     540
gttgatgtgt ggtcttgtgg agtggccctc tatgttatgt tggtaggagc ttatccattc     600
gaagacccta aagaccctcg caatttccga aaaactgttc agaaaataat ggccgtaaac     660
tacaagattc caggatatgt tcacatatcc gaagactgca gaaagttact atctcgtata     720
tttgttgcca atccgttaca tagaagtacg cttaaagaga ttaagagtca tgcatggttc     780
ctaaagaatt tgccaagaga attaaaggag ccagcacaag caatctatta ccaaaggaat     840
gttaatctta ttaatttttc tcctcaaaga gtagaggaga ttatgaagat agttggtgag     900
gcaagaacca ttccaaacct ttctcgcccg gtcgaatcgc ttggatcaga taaaaaagat     960
gatgatgaag aagaatattt ggatgctaat gatgaagaat ggtatgatga ttacgcatag    1020
```

<210> 16
<211> 339
<212> PRT
<213> Arabidopsis thaliana

<400> 16

```
Met Glu Lys Tyr Glu Met Val Lys Asp Leu Gly Phe Gly Asn Phe Gly
1               5                   10                  15

Leu Ala Arg Leu Met Arg Asn Lys Gln Thr Asn Glu Leu Val Ala Val
            20                  25                  30

Lys Phe Ile Asp Arg Gly Tyr Lys Ile Asp Glu Asn Val Ala Arg Glu
        35                  40                  45

Ile Ile Asn His Arg Ala Leu Asn His Pro Asn Ile Val Arg Phe Lys
    50                  55                  60

Glu Val Val Leu Thr Pro Thr His Leu Gly Ile Val Met Glu Tyr Ala
65                  70                  75                  80

Ala Gly Gly Glu Leu Phe Glu Arg Ile Ser Ser Val Gly Arg Phe Ser
                85                  90                  95

Glu Ala Glu Ala Arg Tyr Phe Phe Gln Gln Leu Ile Cys Gly Val His
            100                 105                 110

Tyr Leu His Ala Leu Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140
```

```
Gly Tyr Ser Lys Ser Ser Val Leu His Ser Asn Pro Lys Ser Thr Val
145                 150                 155                 160

Gly Thr Pro Ala Tyr Ile Ala Pro Glu Val Phe Cys Arg Ser Glu Tyr
                165                 170                 175

Asp Gly Lys Ser Val Asp Val Trp Ser Cys Gly Val Ala Leu Tyr Val
            180                 185                 190

Met Leu Val Gly Ala Tyr Pro Phe Glu Asp Pro Lys Asp Pro Arg Asn
        195                 200                 205

Phe Arg Lys Thr Val Gln Lys Ile Met Ala Val Asn Tyr Lys Ile Pro
    210                 215                 220

Gly Tyr Val His Ile Ser Glu Asp Cys Arg Lys Leu Leu Ser Arg Ile
225                 230                 235                 240

Phe Val Ala Asn Pro Leu His Arg Ser Thr Leu Lys Glu Ile Lys Ser
                245                 250                 255

His Ala Trp Phe Leu Lys Asn Leu Pro Arg Glu Leu Lys Glu Pro Ala
            260                 265                 270

Gln Ala Ile Tyr Tyr Gln Arg Asn Val Asn Leu Ile Asn Phe Ser Pro
        275                 280                 285

Gln Arg Val Glu Glu Ile Met Lys Ile Val Gly Glu Ala Arg Thr Ile
    290                 295                 300

Pro Asn Leu Ser Arg Pro Val Glu Ser Leu Gly Ser Asp Lys Lys Asp
305                 310                 315                 320

Asp Asp Glu Glu Glu Tyr Leu Asp Ala Asn Asp Glu Glu Trp Tyr Asp
                325                 330                 335

Asp Tyr Ala
```

<210> 17
<211> 1086
<212> DNA
<213> Arabidopsis thaliana

<400> 17

```
atggatcgag ctccggtgac cacaggaccg ttggatatgc cgattatgca cgacagtgat     60
cgatatgact tcgttaagga tattggttct ggtaatttcg gtgttgctcg tcttatgaga    120
gataaactca ctaaagagct tgttgctgtc aagtacatcg agagaggaga caagattgat    180
gaaaatgttc aaagggagat cattaaccac aggtcactaa ggcatcctaa tattgtcaga    240
tttaaagagg tcattttgac gccgactcat ctggctatca taatggaata tgcttctggc    300
ggtgaacttt acgagcggat ttgcaatgca ggacggttta gtgaagatga ggctcggttc    360
ttctttcagc agcttctatc tggagtcagt tattgtcatt cgatgcaaat ttgccatcgt    420
gacctgaagc tagagaatac attgttggat ggaagtcctg ctcctcgatt aaaaatttgt    480
gattttggat attcaaagtc ttctgttctt cattcacaac caaagtcaac tgttggtact    540
cctgcataca tcgctccaga ggtactgctt cgtcaggaat atgatggcaa gattgcagat    600
gtatggtcat gtggtgtgac cttatacgtc atgttggttg gagcgtatcc gttcgaagat    660
ccagaagagc caagagacta tcggaaaaca atacagagaa tccttagcgt taaatactca    720
atccctgatg acatacggat atcacctgaa tgctgtcatc ttatttcaag aatcttcgtg    780

gctgatcccg ctaccagaat aagcatacca gagatcaaaa cccatagttg gttcttgaag    840
aatctccctg ctgatctaat gaacgagagc aacacaggaa gccagttcca ggagcctgaa    900
caaccaatgc aaagccttga cacaatcatg caaatcatct ctgaagccac aattcccgct    960
gttcgaaacc gttgcctaga cgatttcatg actgacaatc ttgatcttga cgatgacatg   1020
gatgactttg actctgaatc tgaaatcgac attgacagta gcggagagat agtttacgct   1080
ctctaa                                                              1086
```

<210> 18
<211> 361
<212> PRT
<213> Arabidopsis thaliana

<400> 18

```
Met Asp Arg Ala Pro Val Thr Thr Gly Pro Leu Asp Met Pro Ile Met
1               5                   10                  15

His Asp Ser Asp Arg Tyr Asp Phe Val Lys Asp Ile Gly Ser Gly Asn
            20                  25                  30

Phe Gly Val Ala Arg Leu Met Arg Asp Lys Leu Thr Lys Glu Leu Val
        35                  40                  45

Ala Val Lys Tyr Ile Glu Arg Gly Asp Lys Ile Asp Glu Asn Val Gln
    50                  55                  60

Arg Glu Ile Ile Asn His Arg Ser Leu Arg His Pro Asn Ile Val Arg
65                  70                  75                  80

Phe Lys Glu Val Ile Leu Thr Pro Thr His Leu Ala Ile Ile Met Glu
                85                  90                  95

Tyr Ala Ser Gly Gly Glu Leu Tyr Glu Arg Ile Cys Asn Ala Gly Arg
            100                 105                 110

Phe Ser Glu Asp Glu Ala Arg Phe Phe Phe Gln Gln Leu Leu Ser Gly
        115                 120                 125

Val Ser Tyr Cys His Ser Met Gln Ile Cys His Arg Asp Leu Lys Leu
    130                 135                 140

Glu Asn Thr Leu Leu Asp Gly Ser Pro Ala Pro Arg Leu Lys Ile Cys
145                 150                 155                 160

Asp Phe Gly Tyr Ser Lys Ser Ser Val Leu His Ser Gln Pro Lys Ser
                165                 170                 175

Thr Val Gly Thr Pro Ala Tyr Ile Ala Pro Glu Val Leu Leu Arg Gln
            180                 185                 190

Glu Tyr Asp Gly Lys Ile Ala Asp Val Trp Ser Cys Gly Val Thr Leu
        195                 200                 205

Tyr Val Met Leu Val Gly Ala Tyr Pro Phe Glu Asp Pro Glu Glu Pro
    210                 215                 220

Arg Asp Tyr Arg Lys Thr Ile Gln Arg Ile Leu Ser Val Lys Tyr Ser
225                 230                 235                 240

Ile Pro Asp Asp Ile Arg Ile Ser Pro Glu Cys Cys His Leu Ile Ser
```

```
                245                 250                 255

Arg Ile Phe Val Ala Asp Pro Ala Thr Arg Ile Ser Ile Pro Glu Ile
            260                 265                 270

Lys Thr His Ser Trp Phe Leu Lys Asn Leu Pro Ala Asp Leu Met Asn
        275                 280                 285

Glu Ser Asn Thr Gly Ser Gln Phe Gln Glu Pro Glu Gln Pro Met Gln
    290                 295                 300

Ser Leu Asp Thr Ile Met Gln Ile Ile Ser Glu Ala Thr Ile Pro Ala
305                 310                 315                 320

Val Arg Asn Arg Cys Leu Asp Asp Phe Met Thr Asp Asn Leu Asp Leu
                325                 330                 335

Asp Asp Asp Met Asp Asp Phe Asp Ser Glu Ser Glu Ile Asp Ile Asp
            340                 345                 350

Ser Ser Gly Glu Ile Val Tyr Ala Leu
        355                 360
```

<210> 19
<211> 1032
<212> DNA
<213> Arabidopsis thaliana

<400> 19

```
atggagaggt acgaaatagt gaaggatatt gggtctggta acttcggagt agcaaagctt      60
gttcgtgaca aattttccaa agagcttttc gctgttaagt tcatcgagcg aggccaaaag     120
attgatgaac atgtacaaag agaaatcatg aaccataggt cgctgatcca tcccaatata     180
ataagattca aggaggtttt attgacggca acacatttgg cgttagtaat ggaatacgcc     240
gccggaggag aactgttcgg aagaatctgc agcgccggaa gattcagtga agacgaggca     300
aggtttttct ttcagcagct tatatcagga gttaattact gtcacagtct tcaaatatgc     360
catagagatt taaagctaga gaacacgtta cttgatggaa gcgaagcgcc acgtgtaaag     420
atttgcgact ttggatattc aaaatcagga gttcttcatt cgcaaccaaa gacaacagta     480
ggaacacctg cttacattgc acctgaagtg ctctccacga aagagtatga cggcaaaatc     540
gctgatgttt ggtcttgtgg agtcactttg tatgttatgc ttgttggtgc ttatcctttt     600
gaagatcctt ctgatcctaa agattttcgg aagacgatcg gtcggattct caaagctcag     660
tatgctattc ctgattatgt tcgagtttcg gatgaatgca gacatcttct ctctcggata     720
ttcgttgcca accctgaaaa gagaataaca atagaggaga taaagaatca ttcttggttt     780
ctcaagaact tgccggtaga gatgtatgaa ggatcattga tgatgaatgg tccatcgact     840
cagacagtag aagagatagt gtggatcatt gaagaagctc ggaaacctat caccgtagct     900
actggactcg caggtgctgg tggctctggt ggaagcagta atggtgccat tggaagtagc     960
agtatggatc tcgatgactt ggacacagat ttcgacgaca tcgataccgc tgatctcctt    1020
tccccttttgt ga                                                        1032
```

<210> 20
<211> 343
<212> PRT
<213> Arabidopsis thaliana

<400> 20

```
Met Glu Arg Tyr Glu Ile Val Lys Asp Ile Gly Ser Gly Asn Phe Gly
1               5                   10                  15

Val Ala Lys Leu Val Arg Asp Lys Phe Ser Lys Glu Leu Phe Ala Val
            20                  25                  30
```

```
Lys Phe Ile Glu Arg Gly Gln Lys Ile Asp Glu His Val Gln Arg Glu
        35                  40                  45

Ile Met Asn His Arg Ser Leu Ile His Pro Asn Ile Ile Arg Phe Lys
    50                  55                  60

Glu Val Leu Leu Thr Ala Thr His Leu Ala Leu Val Met Glu Tyr Ala
65                  70                  75                  80

Ala Gly Gly Glu Leu Phe Gly Arg Ile Cys Ser Ala Gly Arg Phe Ser
                85                  90                  95

Glu Asp Glu Ala Arg Phe Phe Phe Gln Gln Leu Ile Ser Gly Val Asn
            100                 105                 110

Tyr Cys His Ser Leu Gln Ile Cys His Arg Asp Leu Lys Leu Glu Asn
        115                 120                 125

Thr Leu Leu Asp Gly Ser Glu Ala Pro Arg Val Lys Ile Cys Asp Phe
    130                 135                 140

Gly Tyr Ser Lys Ser Gly Val Leu His Ser Gln Pro Lys Thr Thr Val
145                 150                 155                 160

Gly Thr Pro Ala Tyr Ile Ala Pro Glu Val Leu Ser Thr Lys Glu Tyr
                165                 170                 175

Asp Gly Lys Ile Ala Asp Val Trp Ser Cys Gly Val Thr Leu Tyr Val
            180                 185                 190

Met Leu Val Gly Ala Tyr Pro Phe Glu Asp Pro Ser Asp Pro Lys Asp
        195                 200                 205

Phe Arg Lys Thr Ile Gly Arg Ile Leu Lys Ala Gln Tyr Ala Ile Pro
    210                 215                 220

Asp Tyr Val Arg Val Ser Asp Glu Cys Arg His Leu Leu Ser Arg Ile
225                 230                 235                 240

Phe Val Ala Asn Pro Glu Lys Arg Ile Thr Ile Glu Glu Ile Lys Asn
                245                 250                 255

His Ser Trp Phe Leu Lys Asn Leu Pro Val Glu Met Tyr Glu Gly Ser
            260                 265                 270

Leu Met Met Asn Gly Pro Ser Thr Gln Thr Val Glu Glu Ile Val Trp
        275                 280                 285

Ile Ile Glu Glu Ala Arg Lys Pro Ile Thr Val Ala Thr Gly Leu Ala
    290                 295                 300

Gly Ala Gly Gly Ser Gly Gly Ser Ser Asn Gly Ala Ile Gly Ser Ser
305                 310                 315                 320

Ser Met Asp Leu Asp Asp Leu Asp Thr Asp Phe Asp Asp Ile Asp Thr
                325                 330                 335

Ala Asp Leu Leu Ser Pro Leu
```

340

<210> 21
<211> 1089
<212> DNA
<213> Arabidopsis thaliana

<400> 21

```
atggatccgg cgactaattc accgattatg ccgattgatt taccgattat gcacgacagt      60
gatcgttacg acttcgttaa agatattggc tctggtaatt tcggcgttgc tcgtctcatg     120
accgatagag tcaccaagga gcttgttgct gttaaataca tcgagagagg agaaaagatt     180
gatgaaaatg ttcagaggga gattatcaat catagatcat tgagacatcc taatattgtt     240
aggtttaaag aggtgatttt gacgccttcc catttggcta ttgttatgga atatgctgct     300
ggtggagaac tttatgagcg gatttgtaat gccggacggt ttagtgaaga tgaggctcgg     360
ttcttctttc agcagcttat atctggagtt agctattgtc atgcaatgca aatatgccat     420
cgggatctga agctggaaaa tacattgtta gatggaagtc cggcacctcg tttgaaaata     480
tgtgattttg gttattccaa gtcttctgtt cttcattccc aaccaaagtc aactgttggt     540
actcctgcat acattgcacc agagattctt cttcgacagg aatatgatgg caagcttgca     600
gatgtatggt cttgcggtgt aacattatat gtaatgttgg ttggagctta tccattcgag     660
gatccacagg agccacgaga ttatcgaaag acaatacaaa gaatccttag tgtcacatac     720
tcgatcccag aggacttaca cctctcacca gaatgtcgcc atctaatatc gaggatcttc     780
gtggctgatc cggcaacaag aatcactatt ccggagatca catccgataa atggttcttg     840
aagaatctac caggtgattt gatggatgag aaccgaatgg gaagtcagtt tcaagagcct     900
gagcagccaa tgcagagcct tgacacgatt atgcagataa tatcggaggc tacgattccg     960
actgttcgta atcgttgcct cgatgatttc atggcggata atcttgatct agacgatgac    1020
atggatgact ttgattccga atctgagatt gatgttgaca gtagtggaga gatagtttat    1080
gctctctga                                                            1089
```

<210> 22
<211> 362
<212> PRT
<213> Arabidopsis thaliana

<400> 22

```
Met Asp Pro Ala Thr Asn Ser Pro Ile Met Pro Ile Asp Leu Pro Ile
1               5                   10                  15

Met His Asp Ser Asp Arg Tyr Asp Phe Val Lys Asp Ile Gly Ser Gly
            20                  25                  30

Asn Phe Gly Val Ala Arg Leu Met Thr Asp Arg Val Thr Lys Glu Leu
        35                  40                  45

Val Ala Val Lys Tyr Ile Glu Arg Gly Glu Lys Ile Asp Glu Asn Val
    50                  55                  60

Gln Arg Glu Ile Ile Asn His Arg Ser Leu Arg His Pro Asn Ile Val
65                  70                  75                  80

Arg Phe Lys Glu Val Ile Leu Thr Pro Ser His Leu Ala Ile Val Met
                85                  90                  95

Glu Tyr Ala Ala Gly Gly Glu Leu Tyr Glu Arg Ile Cys Asn Ala Gly
            100                 105                 110

Arg Phe Ser Glu Asp Glu Ala Arg Phe Phe Phe Gln Gln Leu Ile Ser
        115                 120                 125

Gly Val Ser Tyr Cys His Ala Met Gln Ile Cys His Arg Asp Leu Lys
```

```
    130                 135                 140

Leu Glu Asn Thr Leu Leu Asp Gly Ser Pro Ala Pro Arg Leu Lys Ile
145                 150                 155                 160

Cys Asp Phe Gly Tyr Ser Lys Ser Ser Val Leu His Ser Gln Pro Lys
                165                 170                 175

Ser Thr Val Gly Thr Pro Ala Tyr Ile Ala Pro Glu Ile Leu Leu Arg
            180                 185                 190

Gln Glu Tyr Asp Gly Lys Leu Ala Asp Val Trp Ser Cys Gly Val Thr
        195                 200                 205

Leu Tyr Val Met Leu Val Gly Ala Tyr Pro Phe Glu Asp Pro Gln Glu
    210                 215                 220

Pro Arg Asp Tyr Arg Lys Thr Ile Gln Arg Ile Leu Ser Val Thr Tyr
225                 230                 235                 240

Ser Ile Pro Glu Asp Leu His Leu Ser Pro Glu Cys Arg His Leu Ile
                245                 250                 255

Ser Arg Ile Phe Val Ala Asp Pro Ala Thr Arg Ile Thr Ile Pro Glu
            260                 265                 270

Ile Thr Ser Asp Lys Trp Phe Leu Lys Asn Leu Pro Gly Asp Leu Met
        275                 280                 285

Asp Glu Asn Arg Met Gly Ser Gln Phe Gln Glu Pro Glu Gln Pro Met
    290                 295                 300

Gln Ser Leu Asp Thr Ile Met Gln Ile Ile Ser Glu Ala Thr Ile Pro
305                 310                 315                 320

Thr Val Arg Asn Arg Cys Leu Asp Asp Phe Met Ala Asp Asn Leu Asp
                325                 330                 335

Leu Asp Asp Asp Met Asp Asp Phe Asp Ser Glu Ser Glu Ile Asp Val
            340                 345                 350

Asp Ser Ser Gly Glu Ile Val Tyr Ala Leu
        355                 360
```

<210> 23
<211> 1053
<212> DNA
<213> Arabidopsis thaliana

<400> 23

```
atggagagat acgacatctt aagagatctt ggttccggta actttggagt tgctaagctt    60
gtcagagaaa aagccaacgg agagttttac gccgttaaat acatcgaaag aggccttaag   120
attgatgaac atgttcagag agagatcata aaccacagag acttgaagca tcctaatatc   180
atcagattta aagaggtttt tgtaacacca acacatcttg ccatagtaat ggagtatgca   240
gctggtggtg aactttttga aagaatttgc aatgccggta gattcagcga agacgaagga   300
agatattatt tcaaacaact tatctcggga gttagctatt gtcacgctat gcaaatatgt   360
cacagagacc ttaagctcga gaatacactc ttagacggga gcccgtcgtc gcatcttaaa   420
atatgtgatt ttggatactc caagtcatca gttttacact ctcaaccaaa atccaccgtg   480
ggaactccgg cttacgttgc tccggaagtc ttgtcccgga aagaatataa tggaaagatt   540

gcagatgtgt ggtcgtgtgg ggtgacctta tatgtaatgt tagttggtgc ttatcccttt   600
gaagatcccg aagatccacg gaacattaga aacaccattc agaggatatt aagtgtacac   660
tacaccatac cggattacgt caggatttcc tccgagtgca agcatctctt gtctcgtatc   720
tttgtggctg accctgataa gagaataact gtaccggaaa tcgaaaagca cccgtggttc   780
ttgaagggcc ctttggttgt gccgccggag gaagagaaat gcgataatgg agttgaagaa   840
gaagaagaag aagaagagaa gtgtcgacag agtgttgaag agatagtgaa gataatagag   900
gaagcaagaa agggagtaaa tggtacggat aataatggtg gattagggtt aatagatggg   960
agcattgatc ttgatgatat tgatgatgct gatatttatg atgatgttga tgatgatgag  1020
gagagaaatg gtgatttcgt atgtgctcta tga                                1053
```

<210> 24
<211> 350
<212> PRT
<213> Arabidopsis thaliana

<400> 24

```
Met Glu Arg Tyr Asp Ile Leu Arg Asp Leu Gly Ser Gly Asn Phe Gly
1               5                   10                  15

Val Ala Lys Leu Val Arg Glu Lys Ala Asn Gly Glu Phe Tyr Ala Val
            20                  25                  30

Lys Tyr Ile Glu Arg Gly Leu Lys Ile Asp Glu His Val Gln Arg Glu
        35                  40                  45

Ile Ile Asn His Arg Asp Leu Lys His Pro Asn Ile Ile Arg Phe Lys
    50                  55                  60

Glu Val Phe Val Thr Pro Thr His Leu Ala Ile Val Met Glu Tyr Ala
65                  70                  75                  80

Ala Gly Gly Glu Leu Phe Glu Arg Ile Cys Asn Ala Gly Arg Phe Ser
                85                  90                  95

Glu Asp Glu Gly Arg Tyr Tyr Phe Lys Gln Leu Ile Ser Gly Val Ser
            100                 105                 110

Tyr Cys His Ala Met Gln Ile Cys His Arg Asp Leu Lys Leu Glu Asn
        115                 120                 125

Thr Leu Leu Asp Gly Ser Pro Ser Ser His Leu Lys Ile Cys Asp Phe
    130                 135                 140

Gly Tyr Ser Lys Ser Ser Val Leu His Ser Gln Pro Lys Ser Thr Val
145                 150                 155                 160

Gly Thr Pro Ala Tyr Val Ala Pro Glu Val Leu Ser Arg Lys Glu Tyr
                165                 170                 175

Asn Gly Lys Ile Ala Asp Val Trp Ser Cys Gly Val Thr Leu Tyr Val
            180                 185                 190

Met Leu Val Gly Ala Tyr Pro Phe Glu Asp Pro Glu Asp Pro Arg Asn
        195                 200                 205

Ile Arg Asn Thr Ile Gln Arg Ile Leu Ser Val His Tyr Thr Ile Pro
    210                 215                 220

Asp Tyr Val Arg Ile Ser Ser Glu Cys Lys His Leu Leu Ser Arg Ile
```

225 230 235 240

Phe Val Ala Asp Pro Asp Lys Arg Ile Thr Val Pro Glu Ile Glu Lys
245 250 255

His Pro Trp Phe Leu Lys Gly Pro Leu Val Val Pro Pro Glu Glu Glu
260 265 270

Lys Cys Asp Asn Gly Val Glu Glu Glu Glu Glu Glu Glu Glu Lys Cys
275 280 285

Arg Gln Ser Val Glu Glu Ile Val Lys Ile Ile Glu Glu Ala Arg Lys
290 295 300

Gly Val Asn Gly Thr Asp Asn Asn Gly Gly Leu Gly Leu Ile Asp Gly
305 310 315 320

Ser Ile Asp Leu Asp Asp Ile Asp Asp Ala Asp Ile Tyr Asp Asp Val
325 330 335

Asp Asp Asp Glu Glu Arg Asn Gly Asp Phe Val Cys Ala Leu
340 345 350

<210> 25
<211> 1029
<212> DNA
<213> Oryza sativa

<400> 25

```
atggagcggt acgaggtgat gagggacatc gggtccggga acttcggggt ggccaagctc     60
gtccgcgacg tcgccaccaa ccacctcttc gccgtcaagt tcatcgagag gggactcaag    120
attgatgaac atgttcaaag ggagattatg aaccaccgat cactgaagca tccaaacata    180
atccggttca aggaggtcgt gctaactccc acacatttgg caatagttat ggaatatgct    240
gctggtggtg agctatttga aaggatttgc aacgcaggga gattcagtga ggatgaggca    300
aggttcttct tccaacagct gatttctgga gtgagctatt gtcattctat gcaagtatgc    360
catagagatt tgaaactcga aaatactctc ttggatggca gtgtcacacc tcggcttaag    420
atttgtgatt ttggttactc caagtcttct gtcctgcact ctcaaccgaa atcaactgtt    480
ggcacaccgg cttacattgc tccagaggtc ctctctagaa aggaatacga tggaaaggta    540
gctgatgttt ggtcatgtgg ggtaacactc tatgtgatgc ttgttggtgc gtatcctttt    600
gaggaccctg atgacccaag gaacttccgc aagacgatca ctaggatact cagtgtacag    660
tattcaattc cagactacgt tcgagtttca gcggactgca gacatctcct gtcccggatt    720
ttcgttggaa atcctgagca gaggataact atcccagaga tcaagaacca cccatggttc    780
ctgaagaacc tgcccatcga gatgaccgac gagtaccaga ggagcatgca gctggcggac    840
atgaacacgc cgtcgcagag cctggaggag gtcatggcga tcattcagga ggcccggaaa    900
ccgggcgacg ccatgaagct cgccggcgcc gggcaggtcg cctgcctggg gagcatggat    960
ctcgacgaca tcgacgatat cgacgacatt gacatcgaga acagcgggga cttcgtgtgc   1020
gccttgtga                                                           1029
```

<210> 26
<211> 342
<212> PRT
<213> Oryza sativa

<400> 26

Met Glu Arg Tyr Glu Val Met Arg Asp Ile Gly Ser Gly Asn Phe Gly
1 5 10 15

Val Ala Lys Leu Val Arg Asp Val Ala Thr Asn His Leu Phe Ala Val
20 25 30

Lys Phe Ile Glu Arg Gly Leu Lys Ile Asp Glu His Val Gln Arg Glu
35 40 45

Ile Met Asn His Arg Ser Leu Lys His Pro Asn Ile Ile Arg Phe Lys
50 55 60

Glu Val Val Leu Thr Pro Thr His Leu Ala Ile Val Met Glu Tyr Ala
65 70 75 80

Ala Gly Gly Glu Leu Phe Glu Arg Ile Cys Asn Ala Gly Arg Phe Ser
85 90 95

Glu Asp Glu Ala Arg Phe Phe Phe Gln Gln Leu Ile Ser Gly Val Ser
100 105 110

Tyr Cys His Ser Met Gln Val Cys His Arg Asp Leu Lys Leu Glu Asn
115 120 125

Thr Leu Leu Asp Gly Ser Val Thr Pro Arg Leu Lys Ile Cys Asp Phe
130 135 140

Gly Tyr Ser Lys Ser Ser Val Leu His Ser Gln Pro Lys Ser Thr Val
145 150 155 160

Gly Thr Pro Ala Tyr Ile Ala Pro Glu Val Leu Ser Arg Lys Glu Tyr
165 170 175

Asp Gly Lys Val Ala Asp Val Trp Ser Cys Gly Val Thr Leu Tyr Val
180 185 190

Met Leu Val Gly Ala Tyr Pro Phe Glu Asp Pro Asp Asp Pro Arg Asn
195 200 205

Phe Arg Lys Thr Ile Thr Arg Ile Leu Ser Val Gln Tyr Ser Ile Pro
210 215 220

Asp Tyr Val Arg Val Ser Ala Asp Cys Arg His Leu Leu Ser Arg Ile
225 230 235 240

Phe Val Gly Asn Pro Glu Gln Arg Ile Thr Ile Pro Glu Ile Lys Asn
245 250 255

His Pro Trp Phe Leu Lys Asn Leu Pro Ile Glu Met Thr Asp Glu Tyr
260 265 270

Gln Arg Ser Met Gln Leu Ala Asp Met Asn Thr Pro Ser Gln Ser Leu
275 280 285

Glu Glu Val Met Ala Ile Ile Gln Glu Ala Arg Lys Pro Gly Asp Ala
290 295 300

Met Lys Leu Ala Gly Ala Gly Gln Val Ala Cys Leu Gly Ser Met Asp
305 310 315 320

Leu Asp Asp Ile Asp Asp Ile Asp Asp Ile Asp Ile Glu Asn Ser Gly
325 330 335

Asp Phe Val Cys Ala Leu

340

<210> 27
<211> 1020
<212> DNA
<213> Oryza sativa

<400> 27

```
atggagaggt acgaggtgat caaggacata gggtcgggga acttcggcgt ggccaagctt      60
gtccgggatg tgcggaccaa ggagctgttt gccgtcaagt tcatcgagag ggggcagaag     120
atcgacgaga atgtccaaag ggagattatg aaccacaggt cactgaggca tccgaacatt     180
gttagattca aggaggttgt gctaactccc acacatttgg ccatagttat ggaatatgct     240
gctggaggtg agctattcga aaggatttgc agtgctggga ggtttagcga ggatgaggca     300
aggttcttct tccagcagtt gatttcagga gttagctact gtcattccat gcaaatatgt     360
catagagatt tgaaactaga aaatactctc ttggatggga gcatagcacc tcggctcaag     420
atatgtgatt ttggttactc aaagtcctct ttgttgcact ctcaaccgaa atctactgtc     480
ggtactccag cttatatcgc tcctgaggtc cttgctagaa aagaatatga tggaaaggtt     540
gctgacgttt ggtcatgtgg agtaactcta tatgtgatgc ttgttggtgc gtacccccttt    600
gaggaccctg acgaaccaag aaacttccgc aagacaatta ctcggatact aagcgtacaa     660
tacatggttc ctgattatgt tcgagtttcg atggaatgca gacatcttct gtcccggatt     720
ttcgtggcaa acccagagca acgaattacc attcctgaga tcaagaacca cccatggttc     780
ctcaagaacc tgccgatcga gatgactgac gagtaccaga tgagcgtcca gatgaacgac     840
atcaacaccc cgtcacaggg cctggaggag atcatggcca tcatacagga ggcgcggaag     900
ccgggtgatg gctccaaatt ctccgggcag atcccgggcc tagggagcat ggagctcgac     960
gacgttgaca ccgacgacat cgacgtcgag gacagcggcg acttcgtgtg cgcattgtga    1020
```

<210> 28
<211> 339
<212> PRT
<213> Oryza sativa

<400> 28

```
Met Glu Arg Tyr Glu Val Ile Lys Asp Ile Gly Ser Gly Asn Phe Gly
1               5                   10                  15

Val Ala Lys Leu Val Arg Asp Val Arg Thr Lys Glu Leu Phe Ala Val
            20                  25                  30

Lys Phe Ile Glu Arg Gly Gln Lys Ile Asp Glu Asn Val Gln Arg Glu
        35                  40                  45

Ile Met Asn His Arg Ser Leu Arg His Pro Asn Ile Val Arg Phe Lys
    50                  55                  60

Glu Val Val Leu Thr Pro Thr His Leu Ala Ile Val Met Glu Tyr Ala
65                  70                  75                  80

Ala Gly Gly Glu Leu Phe Glu Arg Ile Cys Ser Ala Gly Arg Phe Ser
                85                  90                  95

Glu Asp Glu Ala Arg Phe Phe Phe Gln Gln Leu Ile Ser Gly Val Ser
            100                 105                 110

Tyr Cys His Ser Met Gln Ile Cys His Arg Asp Leu Lys Leu Glu Asn
        115                 120                 125

Thr Leu Leu Asp Gly Ser Ile Ala Pro Arg Leu Lys Ile Cys Asp Phe
    130                 135                 140
```

```
Gly Tyr Ser Lys Ser Ser Leu Leu His Ser Gln Pro Lys Ser Thr Val
145                 150                 155                 160

Gly Thr Pro Ala Tyr Ile Ala Pro Glu Val Leu Ala Arg Lys Glu Tyr
                165                 170                 175

Asp Gly Lys Val Ala Asp Val Trp Ser Cys Gly Val Thr Leu Tyr Val
            180                 185                 190

Met Leu Val Gly Ala Tyr Pro Phe Glu Asp Pro Asp Glu Pro Arg Asn
        195                 200                 205

Phe Arg Lys Thr Ile Thr Arg Ile Leu Ser Val Gln Tyr Met Val Pro
    210                 215                 220

Asp Tyr Val Arg Val Ser Met Glu Cys Arg His Leu Leu Ser Arg Ile
225                 230                 235                 240

Phe Val Ala Asn Pro Glu Gln Arg Ile Thr Ile Pro Glu Ile Lys Asn
                245                 250                 255

His Pro Trp Phe Leu Lys Asn Leu Pro Ile Glu Met Thr Asp Glu Tyr
            260                 265                 270

Gln Met Ser Val Gln Met Asn Asp Ile Asn Thr Pro Ser Gln Gly Leu
        275                 280                 285

Glu Glu Ile Met Ala Ile Ile Gln Glu Ala Arg Lys Pro Gly Asp Gly
    290                 295                 300

Ser Lys Phe Ser Gly Gln Ile Pro Gly Leu Gly Ser Met Glu Leu Asp
305                 310                 315                 320

Asp Val Asp Thr Asp Asp Ile Asp Val Glu Asp Ser Gly Asp Phe Val
                325                 330                 335

Cys Ala Leu
```

<210> 29
<211> 1005
<212> DNA
<213> Oryza sativa

<400> 29

```
atggaggaga ggtacgaggc gttgaaggag ctcggggccg gcaacttcgg ggtggccagg      60
ctggtcaggg acaagaggag caaggagctc gtcgccgtca agtacatcga gaggggcaag     120
aagattgatg aaaatgtgca gagggagatc atcaatcata ggtcgctccg gcatcccaat     180
atcattcggt ttaaggaggt ttgtttgaca cccacacacc tagccattgt catggagtat     240
gctgctggtg gagaactctt tgaacaaatc tgcaccgcag ggcgattcag cgaagacgag     300
gcaaggtact tcttccagca gctaatatca ggtgtcagct actgtcattc tctggaaatt     360
tgccaccgtg atcttaaact tgagaacacc ctcctggatg gaagcccaac acctcgtgtg     420
aagatttgtg actttggtta ctcaaagtct gctttgctgc attcgaagcc gaagtctaca     480
gttggtactc cagcatacat agcgccagaa gttctttcaa gagaagaata tgatggcaag     540
gtagcagacg tttggtcctg tggtgtgaca ctgtacgtga tgcttgtcgg ttcatacccg     600
tttgaagatc caggtgatcc gaggaatttc cgcaaaacga tcagcagaat tcttggcgtg     660
caatactcca tcccggacta cgtgagggtg tcttccgact gcaggcgcct tctatctcaa     720
atatttgttg ccgatccttc aaagaggatc acgatccctg agataaagaa gcacacgtgg     780
tttctgaaga atctgccaaa ggagatatcg gagagggaga aggccgacta caaggacacg     840

gacgccgccc ctccgacgca ggccgtcgag gagatcatgc ggatcatcca ggaggccaag     900
gtccccggcg acatggccgc cgccgacccg gcgctgctcg cggagctcgc cgagctgaag     960
agcgacgacg aagaggaggc cgccgatgag tatgacacct actga                    1005
```

<210> 30
<211> 334
<212> PRT
<213> Oryza sativa

<400> 30

```
Met Glu Glu Arg Tyr Glu Ala Leu Lys Glu Leu Gly Ala Gly Asn Phe
1               5                   10                  15

Gly Val Ala Arg Leu Val Arg Asp Lys Arg Ser Lys Glu Leu Val Ala
            20                  25                  30

Val Lys Tyr Ile Glu Arg Gly Lys Lys Ile Asp Glu Asn Val Gln Arg
        35                  40                  45

Glu Ile Ile Asn His Arg Ser Leu Arg His Pro Asn Ile Ile Arg Phe
    50                  55                  60

Lys Glu Val Cys Leu Thr Pro Thr His Leu Ala Ile Val Met Glu Tyr
65                  70                  75                  80

Ala Ala Gly Gly Glu Leu Phe Glu Gln Ile Cys Thr Ala Gly Arg Phe
                85                  90                  95

Ser Glu Asp Glu Ala Arg Tyr Phe Phe Gln Gln Leu Ile Ser Gly Val
            100                 105                 110

Ser Tyr Cys His Ser Leu Glu Ile Cys His Arg Asp Leu Lys Leu Glu
        115                 120                 125

Asn Thr Leu Leu Asp Gly Ser Pro Thr Pro Arg Val Lys Ile Cys Asp
    130                 135                 140

Phe Gly Tyr Ser Lys Ser Ala Leu Leu His Ser Lys Pro Lys Ser Thr
145                 150                 155                 160

Val Gly Thr Pro Ala Tyr Ile Ala Pro Glu Val Leu Ser Arg Glu Glu
                165                 170                 175

Tyr Asp Gly Lys Val Ala Asp Val Trp Ser Cys Gly Val Thr Leu Tyr
            180                 185                 190

Val Met Leu Val Gly Ser Tyr Pro Phe Glu Asp Pro Gly Asp Pro Arg
        195                 200                 205

Asn Phe Arg Lys Thr Ile Ser Arg Ile Leu Gly Val Gln Tyr Ser Ile
    210                 215                 220

Pro Asp Tyr Val Arg Val Ser Ser Asp Cys Arg Arg Leu Leu Ser Gln
225                 230                 235                 240

Ile Phe Val Ala Asp Pro Ser Lys Arg Ile Thr Ile Pro Glu Ile Lys
                245                 250                 255

Lys His Thr Trp Phe Leu Lys Asn Leu Pro Lys Glu Ile Ser Glu Arg
```

260 265 270

Glu Lys Ala Asp Tyr Lys Asp Thr Asp Ala Ala Pro Pro Thr Gln Ala
275 280 285

Val Glu Glu Ile Met Arg Ile Ile Gln Glu Ala Lys Val Pro Gly Asp
290 295 300

Met Ala Ala Ala Asp Pro Ala Leu Leu Ala Glu Leu Ala Glu Leu Lys
305 310 315 320

Ser Asp Asp Glu Glu Glu Ala Ala Asp Glu Tyr Asp Thr Tyr
325 330

<210> 31
<211> 1083
<212> DNA
<213> Oryza sativa

<400> 31

```
atggagaagt acgaggcggt gagggacatc gggtcgggga acttcggggt ggcgcggctg      60
atgcgcaacc gcgagacccg cgagctcgtc gccgtcaagt gcatcgagcg cggccaccgg     120
atagatgaga atgtgtacag ggagatcatc aaccaccgct cgctgcgcca ccccaacatc     180
attcgcttca aggaggtgat actgacgcca acgcatctta tgattgtcat ggagttcgca     240
gcaggcgggg agctgttcga tcgaatctgt gatcgtggac ggttcagtga ggatgaggcc     300
aggtatttct ttcagcagct gatctgtgga gtgagctact gccatcacat gcaaatatgc     360
catagagatt tgaagttgga gaatgttctc ttggatggca gcccagctcc acggcttaag     420
atatgtgatt ttggctactc caagtcatca gtattgcatt caagacccaa atcagcagtg     480
gggacgccag catatatcgc accagaggtg ctatcccgcc gtgagtatga tggaaagctt     540
gcagatgtat ggtcctgtgg tgtgactctt tacgtcatgc ttgtgggagc ctacccattt     600
gaagaccagg acgaccccaa gaacattcgc aaaaccattc agagaataat gtcagtgcaa     660
tataagatac cagattacgt ccacatatct gcagaatgca aacagcttat tgcccgcatt     720
tttgtcaaca atccattgag gagaatcacg atgaaggaaa taaagagcca cccgtggttc     780
ttgaagaacc tccccaggga gctcacggag actgcgcaag ccatgtacta caggagggac     840
aactccgtgc cttccttctc agaccagacc tcagaagaga tcatgaagat tgttcaagaa     900
gcaagaacca tgccgaaatc atccaggaca ggctactgga gcgacgcggg ttcagacgag     960
gaggagaagg aagaggaaga gaggccagaa gagaacgagg aagaggagga agatgagtac    1020
gataagaggg tcaaagaggt ccatgcgagc ggggagctcc gtatgagctc actgcgcata    1080
tga                                                                  1083
```

<210> 32
<211> 360
<212> PRT
<213> Oryza sativa

<400> 32

```
Met Glu Lys Tyr Glu Ala Val Arg Asp Ile Gly Ser Gly Asn Phe Gly
1               5                   10                  15

Val Ala Arg Leu Met Arg Asn Arg Glu Thr Arg Glu Leu Val Ala Val
            20                  25                  30

Lys Cys Ile Glu Arg Gly His Arg Ile Asp Glu Asn Val Tyr Arg Glu
        35                  40                  45

Ile Ile Asn His Arg Ser Leu Arg His Pro Asn Ile Ile Arg Phe Lys
    50                  55                  60

Glu Val Ile Leu Thr Pro Thr His Leu Met Ile Val Met Glu Phe Ala
```

```
65                  70                  75                  80

Ala Gly Gly Glu Leu Phe Asp Arg Ile Cys Asp Arg Gly Arg Phe Ser
                85                  90                  95

Glu Asp Glu Ala Arg Tyr Phe Phe Gln Gln Leu Ile Cys Gly Val Ser
            100                 105                 110

Tyr Cys His His Met Gln Ile Cys His Arg Asp Leu Lys Leu Glu Asn
        115                 120                 125

Val Leu Leu Asp Gly Ser Pro Ala Pro Arg Leu Lys Ile Cys Asp Phe
    130                 135                 140

Gly Tyr Ser Lys Ser Ser Val Leu His Ser Arg Pro Lys Ser Ala Val
145                 150                 155                 160

Gly Thr Pro Ala Tyr Ile Ala Pro Glu Val Leu Ser Arg Arg Glu Tyr
                165                 170                 175

Asp Gly Lys Leu Ala Asp Val Trp Ser Cys Gly Val Thr Leu Tyr Val
            180                 185                 190

Met Leu Val Gly Ala Tyr Pro Phe Glu Asp Gln Asp Asp Pro Lys Asn
        195                 200                 205

Ile Arg Lys Thr Ile Gln Arg Ile Met Ser Val Gln Tyr Lys Ile Pro
    210                 215                 220

Asp Tyr Val His Ile Ser Ala Glu Cys Lys Gln Leu Ile Ala Arg Ile
225                 230                 235                 240

Phe Val Asn Asn Pro Leu Arg Arg Ile Thr Met Lys Glu Ile Lys Ser
                245                 250                 255

His Pro Trp Phe Leu Lys Asn Leu Pro Arg Glu Leu Thr Glu Thr Ala
            260                 265                 270

Gln Ala Met Tyr Tyr Arg Arg Asp Asn Ser Val Pro Ser Phe Ser Asp
        275                 280                 285

Gln Thr Ser Glu Glu Ile Met Lys Ile Val Gln Glu Ala Arg Thr Met
    290                 295                 300

Pro Lys Ser Ser Arg Thr Gly Tyr Trp Ser Asp Ala Gly Ser Asp Glu
305                 310                 315                 320

Glu Glu Lys Glu Glu Glu Glu Arg Pro Glu Glu Asn Glu Glu Glu Glu
                325                 330                 335

Glu Asp Glu Tyr Asp Lys Arg Val Lys Glu Val His Ala Ser Gly Glu
            340                 345                 350

Leu Arg Met Ser Ser Leu Arg Ile
        355                 360
```

<210> 33
<211> 1113
<212> DNA

<213> Oryza sativa

<400> 33

```
atggagaaat acgagccagt tcgggagatc ggggcgggca acttcggggt agcgaagctg     60
atgcggaaca aggagacgcg ggagctggtg gcgatgaagt tcatcgagag agggaacagg    120
atcgacgaga acgtgttccg ggagatcgtg aatcatcgtt cgctgcgtca cccgaacata    180
ataaggttca aggaggtggt ggtgacgggg aggcatctgg cgatcgtgat ggagtacgcg    240
gcgggagggg agctgttcga gaggatatgc gaggcgggga ggttccacga ggacgaggcg    300
cgctacttct tccagcagct ggtgtgcggg gtgagctact gccacgccat gcagatctgc    360
caccgcgacc tcaagctgga gaatacgctg ctggacggca gcccggcccc gcgcctcaag    420
atctgcgact tcggctactc caagtcctcc ctcctccact cccgccccaa atccaccgtc    480
ggcacccccg cctacatcgc ccccgaggtc ctctcccgcc gcgagtacga cggcaagctc    540
gccgacgtct ggtcctgcgg cgtcaccctc tacgtcatgc tcgtcggcgc ttaccctttc    600
gaggatccca aggaccccaa gaacttcaga aagaccatct cgcgcatcat gtccgtccag    660
tacaagatcc ccgagtacgt ccacgtctcc cagccctgcc gccacctcct ctcccgcatc    720
ttcgtcgcca acccctacaa gcgcatcagc atgggcgaga tcaagagcca cccctggttc    780
ctcaagaacc tgccgcgcga gctcaaggag gaggcgcagg ccgtctacta caaccgccgg    840
ggagccgatc acgcggcttc cagcgcaagt agtgcggctg ctgcagctgc cttctcgccg    900
cagagcgtgg aggacatcat gaggatcgtg caggaggcgc agaccgtccc caagcccgac    960
aagcccgtct ctggctacgg ctggggcacc gacgacgacg acgacgacca acaaccagct   1020
gaggaggagg acgaagaaga cgactacgac aggacggtgc gcgaggttca cgccagcgtc   1080
gacctcgaca tgtcaaacct ccaaatctcc tga                                 1113
```

<210> 34
<211> 370
<212> PRT
<213> Oryza sativa

<400> 34

```
Met Glu Lys Tyr Glu Pro Val Arg Glu Ile Gly Ala Gly Asn Phe Gly
1               5                   10                  15

Val Ala Lys Leu Met Arg Asn Lys Glu Thr Arg Glu Leu Val Ala Met
            20                  25                  30

Lys Phe Ile Glu Arg Gly Asn Arg Ile Asp Glu Asn Val Phe Arg Glu
        35                  40                  45

Ile Val Asn His Arg Ser Leu Arg His Pro Asn Ile Ile Arg Phe Lys
    50                  55                  60

Glu Val Val Val Thr Gly Arg His Leu Ala Ile Val Met Glu Tyr Ala
65                  70                  75                  80

Ala Gly Gly Glu Leu Phe Glu Arg Ile Cys Glu Ala Gly Arg Phe His
                85                  90                  95

Glu Asp Glu Ala Arg Tyr Phe Phe Gln Gln Leu Val Cys Gly Val Ser
            100                 105                 110

Tyr Cys His Ala Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
145                 150                 155                 160
```

```
Gly Thr Pro Ala Tyr Ile Ala Pro Glu Val Leu Ser Arg Arg Glu Tyr
                165                 170                 175

Asp Gly Lys Leu Ala Asp Val Trp Ser Cys Gly Val Thr Leu Tyr Val
            180                 185                 190

Met Leu Val Gly Ala Tyr Pro Phe Glu Asp Pro Lys Asp Pro Lys Asn
        195                 200                 205

Phe Arg Lys Thr Ile Ser Arg Ile Met Ser Val Gln Tyr Lys Ile Pro
    210                 215                 220

Glu Tyr Val His Val Ser Gln Pro Cys Arg His Leu Leu Ser Arg Ile
225                 230                 235                 240

Phe Val Ala Asn Pro Tyr Lys Arg Ile Ser Met Gly Glu Ile Lys Ser
                245                 250                 255

His Pro Trp Phe Leu Lys Asn Leu Pro Arg Glu Leu Lys Glu Glu Ala
            260                 265                 270

Gln Ala Val Tyr Tyr Asn Arg Arg Gly Ala Asp His Ala Ala Ser Ser
        275                 280                 285

Ala Ser Ser Ala Ala Ala Ala Ala Ala Phe Ser Pro Gln Ser Val Glu
    290                 295                 300

Asp Ile Met Arg Ile Val Gln Glu Ala Gln Thr Val Pro Lys Pro Asp
305                 310                 315                 320

Lys Pro Val Ser Gly Tyr Gly Trp Gly Thr Asp Asp Asp Asp Asp Asp
                325                 330                 335

Gln Gln Pro Ala Glu Glu Glu Asp Glu Glu Asp Asp Tyr Asp Arg Thr
            340                 345                 350

Val Arg Glu Val His Ala Ser Val Asp Leu Asp Met Ser Asn Leu Gln
        355                 360                 365

Ile Ser
    370
```

<210> 35
<211> 1098
<212> DNA
<213> Oryza sativa

<400> 35

```
atggagaagt acgagctgct caaggacatc gggtcgggca acttcggtgt ggcgcggctg     60
atgcggaaca gggagaccaa ggagctcgtc gccatgaagt acataccgcg tggcctcaag    120
attgacgaga atgtggcgag ggagatcata aaccaccgct cgctgcggca cccaaacatc    180
atccggttca aggaggtcgt gctcacgcct acccacctcg cgatcgtcat ggagtacgcc    240
gccggcggcg agctgttcga ccggatctgc agcgccggga gattcagcga ggacgagtcg    300
aggtatttct tccagcaact aatttgcggc gtcagctact gccacttcat gcaaatttgc    360
caccgggatt tgaagctgga gaacacgctg ctggatggca gccctgcgcc gcgcctcaag    420
atctgcgact ttggctactc caagtcatca ctgctgcact caaagccgaa gtcgacggtc    480
gggactcccg cgtacatcgc tccggaggtg ctctctcgcc gggagtatga cggcaagatg    540
gcagatgtat ggtcttgtgg ggtgaccctt tatgtgatgc tcgtcggtgc ttaccctttt    600
gaggacccag atgatcccaa gaatttcaga aaaacaatcg ggagaatcgt atcaattcag    660

tacaaaatac cagagtacgt ccatatatcc caagattgta gacaactcct ctctcgaatc    720
tttgtcgcga atcctgcaaa gagaataaca ataagagaga tcagaaacca cccttggttt    780
atgaagaact tgccgcggga gcttacagaa gcggcgcaag cgaagtacta caagaaggac    840
aacagtgccc gtacattctc ggatcagacc gtcgacgaga tcatgaagat tgtacaagag    900
gcaaagacac cacctccatc gtcgactcca gtggccggtt tcggttggac cgaggaagaa    960
gagcaggagg acggtaagaa tcccgacgac gacgagggag acagggatga ggaggagggc   1020
gaggaaggcg atagcgagga cgagtacacc aagcaggtga agcaagccca tgccagctgt   1080
gacttgcaga agagctga                                                 1098
```

<210> 36
<211> 365
<212> PRT
<213> Oryza sativa

<400> 36

Met Glu Lys Tyr Glu Leu Leu Lys Asp Ile Gly Ser Gly Asn Phe Gly
1               5                   10                  15

Val Ala Arg Leu Met Arg Asn Arg Glu Thr Lys Glu Leu Val Ala Met
            20                  25                  30

Lys Tyr Ile Pro Arg Gly Leu Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Val Leu Thr Pro Thr His Leu Ala Ile Val Met Glu Tyr Ala
65                  70                  75                  80

Ala Gly Gly Glu Leu Phe Asp Arg Ile Cys Ser Ala Gly Arg Phe Ser
                85                  90                  95

Glu Asp Glu Ser Arg Tyr Phe Phe Gln Gln Leu Ile Cys Gly Val Ser
            100                 105                 110

Tyr Cys His Phe Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Lys Pro Lys Ser Thr Val
145                 150                 155                 160

Gly Thr Pro Ala Tyr Ile Ala Pro Glu Val Leu Ser Arg Arg Glu Tyr
                165                 170                 175

Asp Gly Lys Met Ala Asp Val Trp Ser Cys Gly Val Thr Leu Tyr Val
            180                 185                 190

Met Leu Val Gly Ala Tyr Pro Phe Glu Asp Pro Asp Asp Pro Lys Asn
        195                 200                 205

Phe Arg Lys Thr Ile Gly Arg Ile Val Ser Ile Gln Tyr Lys Ile Pro
    210                 215                 220

Glu Tyr Val His Ile Ser Gln Asp Cys Arg Gln Leu Leu Ser Arg Ile
225                 230                 235                 240

```
Phe Val Ala Asn Pro Ala Lys Arg Ile Thr Ile Arg Glu Ile Arg Asn
                245                 250                 255

His Pro Trp Phe Met Lys Asn Leu Pro Arg Glu Leu Thr Glu Ala Ala
            260                 265                 270

Gln Ala Lys Tyr Tyr Lys Lys Asp Asn Ser Ala Arg Thr Phe Ser Asp
        275                 280                 285

Gln Thr Val Asp Glu Ile Met Lys Ile Val Gln Glu Ala Lys Thr Pro
    290                 295                 300

Pro Pro Ser Ser Thr Pro Val Ala Gly Phe Gly Trp Thr Glu Glu Glu
305                 310                 315                 320

Glu Gln Glu Asp Gly Lys Asn Pro Asp Asp Asp Glu Gly Asp Arg Asp
                325                 330                 335

Glu Glu Glu Gly Glu Glu Gly Asp Ser Glu Asp Glu Tyr Thr Lys Gln
            340                 345                 350

Val Lys Gln Ala His Ala Ser Cys Asp Leu Gln Lys Ser
        355                 360                 365
```

<210> 37
<211> 1080
<212> DNA
<213> Oryza sativa

<400> 37

```
atggagaggt acgagctgct caaggacatc ggcgccggga acttcggggt ggcgcggctg     60
atgcggaata aggagaccaa ggagctggtc gccatgaagt acatccctcg ggcctcaag     120
attgacgaga atgtggcgag ggagatcatc aaccaccggt cgctgcggca ccccaacatc    180
atccgcttca aggaggtggt ggtcacgccg acgcacctgg cgatcgtgat ggagtacgct    240
gccggcggcg agttgttcga ccggatctgc aacgccggga ggttcagcga ggacgaggcc    300
aggtatttct tccagcagct catctgcggc gtgagctact gccacttcat gcaaatttgc    360
caccgggatt tgaagctgga gaacacgctg ctggacggca gcccggcgcc ccgcctcaag    420
atctgcgact tcggttactc caagtcgtcg ctgctgcact cgaagcccaa gtcgacggtc    480
gggacgccgg cgtacatcgc gccggaggtg ctatcccgcc gggagtacga cggcaagaca    540
gccgatgtgt ggtcttgtgg agtgactctt tatgtgatgc ttgttggtgc ttacccctt     600
gaggaccctg atgaccccaa gaatttcaga aagaccattg ggagaataat gtcaattcag    660
tacaaaatac ccgagtacgt ccatgtatcc caggactgca ggcaactcct ttctagaatt    720
tttgttgcaa accctgcaaa gagaataaca ataagggaga tcaggaacca cccatggttc    780
ctgaagaacc tgccaagaga gctcacagaa gctgcacagg caatgtacta caagaaggat    840
aacagtgccc cgacctactc cgtccagtcg gtcgaggaga tcatgaagat tgtcgaggaa    900
gcgcggacgc cgcctcggtc ctccaccccc gtggccggct ttggctggca agaggaggat    960
gagcaggagg acaacagcaa gaagccagag gaagaacagg aggaagagga agatgctgag   1020
gatgagtacg acaagcaggt gaaacaagtc catgccagtg gtgagtttca gctcagctga   1080
```

<210> 38
<211> 359
<212> PRT
<213> Oryza sativa

<400> 38

```
Met Glu Arg Tyr Glu Leu Leu Lys Asp Ile Gly Ala Gly Asn Phe Gly
1               5                   10                  15
```

Val Ala Arg Leu Met Arg Asn Lys Glu Thr Lys Glu Leu Val Ala Met
20 25 30

Lys Tyr Ile Pro Arg Gly Leu Lys Ile Asp Glu Asn Val Ala Arg Glu
35 40 45

Ile Ile Asn His Arg Ser Leu Arg His Pro Asn Ile Ile Arg Phe Lys
50 55 60

Glu Val Val Val Thr Pro Thr His Leu Ala Ile Val Met Glu Tyr Ala
65 70 75 80

Ala Gly Gly Glu Leu Phe Asp Arg Ile Cys Asn Ala Gly Arg Phe Ser
85 90 95

Glu Asp Glu Ala Arg Tyr Phe Phe Gln Gln Leu Ile Cys Gly Val Ser
100 105 110

Tyr Cys His Phe Met Gln Ile Cys His Arg Asp Leu Lys Leu Glu Asn
115 120 125

Thr Leu Leu Asp Gly Ser Pro Ala Pro Arg Leu Lys Ile Cys Asp Phe
130 135 140

Gly Tyr Ser Lys Ser Ser Leu Leu His Ser Lys Pro Lys Ser Thr Val
145 150 155 160

Gly Thr Pro Ala Tyr Ile Ala Pro Glu Val Leu Ser Arg Arg Glu Tyr
165 170 175

Asp Gly Lys Thr Ala Asp Val Trp Ser Cys Gly Val Thr Leu Tyr Val
180 185 190

Met Leu Val Gly Ala Tyr Pro Phe Glu Asp Pro Asp Asp Pro Lys Asn
195 200 205

Phe Arg Lys Thr Ile Gly Arg Ile Met Ser Ile Gln Tyr Lys Ile Pro
210 215 220

Glu Tyr Val His Val Ser Gln Asp Cys Arg Gln Leu Leu Ser Arg Ile
225 230 235 240

Phe Val Ala Asn Pro Ala Lys Arg Ile Thr Ile Arg Glu Ile Arg Asn
245 250 255

His Pro Trp Phe Leu Lys Asn Leu Pro Arg Glu Leu Thr Glu Ala Ala
260 265 270

Gln Ala Met Tyr Tyr Lys Lys Asp Asn Ser Ala Pro Thr Tyr Ser Val
275 280 285

Gln Ser Val Glu Glu Ile Met Lys Ile Val Glu Glu Ala Arg Thr Pro
290 295 300

Pro Arg Ser Ser Thr Pro Val Ala Gly Phe Gly Trp Gln Glu Glu Asp
305 310 315 320

Glu Gln Glu Asp Asn Ser Lys Lys Pro Glu Glu Glu Gln Glu Glu Glu
325 330 335

Glu Asp Ala Glu Asp Glu Tyr Asp Lys Gln Val Lys Gln Val His Ala
340 345 350

Ser Gly Glu Phe Gln Leu Ser
355

<210> 39
<211> 1116
<212> DNA
<213> Oryza sativa

<400> 39

```
atggcagcgg cgggggccgg ggcgggggcg ccggatcggg cggcgctgac ggtgggcccg     60
gggatggaca tgccgatcat gcacgacagc gaccggtacg agctcgtgcg cgacatcggc    120
tccggcaact tcggcgtcgc ccgcctcatg cgcgaccgcc gcaccatgga gctcgtcgcc    180
gtcaagtaca tcgagcgcgg cgagaagata gatgataatg tccagcgtga gattataaat    240
caccgatcgt tgaaacatcc taacattatt aggtttaagg aggttatttt aaccccaact    300
catcttgcta ttgtcatgga atatgcctct ggtggtgagc ttttcgagag aatttgtaag    360
aatgtacggt tcagtgaaga tgaggctcgc tacttcttcc agcagcttat ctcgggagtc    420
agctactgcc attcaatgca agtatgccac cgtgatttga agttggagaa tacactgctg    480
gatggaagcc ctgctccacg cttgaaaata tgtgactttg gctattctaa gtcttcagtt    540
ctccattcac aaccaaaatc cactgtagga acccctgctt atattgcacc tgaagttctg    600
ttgaagaaag aatacgatgg caagactgct gatgtatggt cctgtggtgt gactctatat    660
gttatggtag ttggtgcata tcctttcgag gatccagaag agcctaagaa cttccgtaaa    720
acaattcagc gtatcttgaa tgttcagtac tcaattccag aaaacgtgga catatctcca    780
gaatgtaggc atctaatttc gaggattttt gtcggggatc cgtctttgag gataacaatc    840
ccagaaatac ggagccatgg ctggttcttg aagaaccttc ctgcagattt gatggacgat    900
gatagtatga gcagccagta tgaggaacct gatcagccaa tgcaaaccat ggatcagatc    960
atgcaaattt taactgaggc caccatacca cctgcttgct ctcgaataaa ccacatccta   1020
actgatggac tcgacctaga cgatgacatg gatgacctcg attccgactc agatattgat   1080
gttgatagca gcggcgagat cgtctatgcg atgtaa                             1116
```

<210> 40
<211> 371
<212> PRT
<213> Oryza sativa

<400> 40

```
Met Ala Ala Ala Gly Ala Gly Ala Gly Ala Pro Asp Arg Ala Ala Leu
1               5                   10                  15

Thr Val Gly Pro Gly Met Asp Met Pro Ile Met His Asp Ser Asp Arg
            20                  25                  30

Tyr Glu Leu Val Arg Asp Ile Gly Ser Gly Asn Phe Gly Val Ala Arg
        35                  40                  45

Leu Met Arg Asp Arg Arg Thr Met Glu Leu Val Ala Val Lys Tyr Ile
    50                  55                  60

Glu Arg Gly Glu Lys Ile Asp Asp Asn Val Gln Arg Glu Ile Ile Asn
65                  70                  75                  80

His Arg Ser Leu Lys His Pro Asn Ile Ile Arg Phe Lys Glu Val Ile
                85                  90                  95

Leu Thr Pro Thr His Leu Ala Ile Val Met Glu Tyr Ala Ser Gly Gly
            100                 105                 110
```

```
Glu Leu Phe Glu Arg Ile Cys Lys Asn Val Arg Phe Ser Glu Asp Glu
        115                 120                 125

Ala Arg Tyr Phe Phe Gln Gln Leu Ile Ser Gly Val Ser Tyr Cys His
    130                 135                 140

Ser Met Gln Val Cys His Arg Asp Leu Lys Leu Glu Asn Thr Leu Leu
145                 150                 155                 160

Asp Gly Ser Pro Ala Pro Arg Leu Lys Ile Cys Asp Phe Gly Tyr Ser
                165                 170                 175

Lys Ser Ser Val Leu His Ser Gln Pro Lys Ser Thr Val Gly Thr Pro
            180                 185                 190

Ala Tyr Ile Ala Pro Glu Val Leu Leu Lys Lys Glu Tyr Asp Gly Lys
        195                 200                 205

Thr Ala Asp Val Trp Ser Cys Gly Val Thr Leu Tyr Val Met Val Val
    210                 215                 220

Gly Ala Tyr Pro Phe Glu Asp Pro Glu Glu Pro Lys Asn Phe Arg Lys
225                 230                 235                 240

Thr Ile Gln Arg Ile Leu Asn Val Gln Tyr Ser Ile Pro Glu Asn Val
                245                 250                 255

Asp Ile Ser Pro Glu Cys Arg His Leu Ile Ser Arg Ile Phe Val Gly
            260                 265                 270

Asp Pro Ser Leu Arg Ile Thr Ile Pro Glu Ile Arg Ser His Gly Trp
        275                 280                 285

Phe Leu Lys Asn Leu Pro Ala Asp Leu Met Asp Asp Asp Ser Met Ser
    290                 295                 300

Ser Gln Tyr Glu Glu Pro Asp Gln Pro Met Gln Thr Met Asp Gln Ile
305                 310                 315                 320

Met Gln Ile Leu Thr Glu Ala Thr Ile Pro Pro Ala Cys Ser Arg Ile
                325                 330                 335

Asn His Ile Leu Thr Asp Gly Leu Asp Leu Asp Asp Asp Met Asp Asp
            340                 345                 350

Leu Asp Ser Asp Ser Asp Ile Asp Val Asp Ser Ser Gly Glu Ile Val
        355                 360                 365

Tyr Ala Met
    370
```

<210> 41
<211> 1086
<212> DNA
<213> Oryza sativa

<400> 41

```
atggagaggg cggcggcggg gccgctgggg atggagatgc cgataatgca cgacggtgac      60
aggtacgagc tggtgaagga gatcgggtcg gggaacttcg gcgtcgcccg cctcatgcgc     120
aaccgcgcct ccggcgacct cgtcgccgtc aagtacatcg accgcggcga gaagattgac     180
gagaacgtgc agagggagat catcaaccac aggtcgctgc gccaccccaa catcatccga     240
ttcaaggagg ttattctgac gccgacgcat ctcgcgatcg tcatggagta cgcctccggc     300
ggcgagctct tcgagcgcat ctgcagcgcc ggccgcttca gcgaggacga ggctcgtttc     360
ttcttccagc agctgatatc tggagttagc tactgccatt ccatgcaagt atgccatcgt     420
gacttaaagc tggagaacac tctgctagat ggaagtactg ctcctcgctt gaagatatgt     480
gactttggtt actcgaagtc atcggttctt cattcacaac caaaatcaac agttggaact     540
ccagcttata ttgctccaga agttttgctc aagaaagaat acgatggaaa gattgccgat     600
gtttggtcat gcggtgtgac gctctacgtg atgttggttg gcgcataccc tttcgaggat     660
cctgaagatc ccaagaactt cagaaagaca attcagaaaa tattgggtgt tcagtactca     720
attccagact atgtccacat atctccggag tgccgcgatc tcattacgag gatttttgtt     780
ggcaacccag ctagtaggat caccatgcct gagataaaga accacccatg gttcatgaag     840
aacatcccgg ctgacctcat ggatgatggc atggttagca atcagtacga ggagcctgac     900
cagccgatgc agaatatgaa cgagatcatg cagatactgg cagaagcaac aattccagca     960
gcaggcacca gtggaatcaa tcagttcttg actgacagcc ttgacctcga cgacgacatg    1020
gaggatatgg actcggacct tgaccttgac attgagagca gcggagagat cgtatatgcc    1080
atgtaa                                                               1086
```

<210> 42
<211> 361
<212> PRT
<213> Oryza sativa

<400> 42

```
Met Glu Arg Ala Ala Ala Gly Pro Leu Gly Met Glu Met Pro Ile Met
1               5                   10                  15

His Asp Gly Asp Arg Tyr Glu Leu Val Lys Glu Ile Gly Ser Gly Asn
            20                  25                  30

Phe Gly Val Ala Arg Leu Met Arg Asn Arg Ala Ser Gly Asp Leu Val
        35                  40                  45

Ala Val Lys Tyr Ile Asp Arg Gly Glu Lys Ile Asp Glu Asn Val Gln
    50                  55                  60

Arg Glu Ile Ile Asn His Arg Ser Leu Arg His Pro Asn Ile Ile Arg
65                  70                  75                  80

Phe Lys Glu Val Ile Leu Thr Pro Thr His Leu Ala Ile Val Met Glu
                85                  90                  95

Tyr Ala Ser Gly Gly Glu Leu Phe Glu Arg Ile Cys Ser Ala Gly Arg
            100                 105                 110

Phe Ser Glu Asp Glu Ala Arg Phe Phe Phe Gln Gln Leu Ile Ser Gly
        115                 120                 125

Val Ser Tyr Cys His Ser Met Gln Val Cys His Arg Asp Leu Lys Leu
    130                 135                 140

Glu Asn Thr Leu Leu Asp Gly Ser Thr Ala Pro Arg Leu Lys Ile Cys
145                 150                 155                 160

Asp Phe Gly Tyr Ser Lys Ser Ser Val Leu His Ser Gln Pro Lys Ser
                165                 170                 175

Thr Val Gly Thr Pro Ala Tyr Ile Ala Pro Glu Val Leu Leu Lys Lys
```

```
            180                 185                 190

Glu Tyr Asp Gly Lys Ile Ala Asp Val Trp Ser Cys Gly Val Thr Leu
        195                 200                 205

Tyr Val Met Leu Val Gly Ala Tyr Pro Phe Glu Asp Pro Glu Asp Pro
    210                 215                 220

Lys Asn Phe Arg Lys Thr Ile Gln Lys Ile Leu Gly Val Gln Tyr Ser
225                 230                 235                 240

Ile Pro Asp Tyr Val His Ile Ser Pro Glu Cys Arg Asp Leu Ile Thr
                245                 250                 255

Arg Ile Phe Val Gly Asn Pro Ala Ser Arg Ile Thr Met Pro Glu Ile
            260                 265                 270

Lys Asn His Pro Trp Phe Met Lys Asn Ile Pro Ala Asp Leu Met Asp
        275                 280                 285

Asp Gly Met Val Ser Asn Gln Tyr Glu Glu Pro Asp Gln Pro Met Gln
    290                 295                 300

Asn Met Asn Glu Ile Met Gln Ile Leu Ala Glu Ala Thr Ile Pro Ala
305                 310                 315                 320

Ala Gly Thr Ser Gly Ile Asn Gln Phe Leu Thr Asp Ser Leu Asp Leu
                325                 330                 335

Asp Asp Asp Met Glu Asp Met Asp Ser Asp Leu Asp Leu Asp Ile Glu
            340                 345                 350

Ser Ser Gly Glu Ile Val Tyr Ala Met
        355                 360
```

<210> 43
<211> 1089
<212> DNA
<213> Oryza sativa

<400> 43

```
atggaccggg cggcgctgac ggtggggccg gggatggaca tgccgataat gcacgacggc     60
gaccggtacg agctggtgcg ggacatcggc tccggcaact tcggcgtcgc gcgcctcatg    120
cgcagccgcg ccgacggcca gctcgtcgcc gtcaagtaca tcgagcgcgg cgacaagatc    180
gacgagaacg tgcagcggga gatcatcaac caccgctcgc tgcgccaccc caacatcatc    240
cgcttcaagg aggtcatcct cacccccacc cacctcgcca tcgtcatgga gtacgcctcc    300
ggcggcgagc tcttcgagcg tatctgcaac gccggcaggt tcagcgagga cgaggcacgg    360
ttctttttcc agcaactgat ttcaggagtc agctattgcc attccatgca agtatgccat    420
cgtgacctga agctggagaa caccctgctc gacggcagca cggcgcctcg cctcaagata    480
tgcgactttg gctattcaaa gtcgtctgtt cttcattcgc aaccaaaatc tactgttgga    540
actccggcat acatcgctcc tgaggttctg ctgaagaagg aatatgatgg aaagattgct    600
gatgtgtggt cgtgtggagt aaccctctac gtaatgctgg ttggtgcata tccttttgag    660
gatccagatg agcctaagaa tttcaggaag acaattcaga gaatattggg tgtgcagtac    720
tctattccag attatgtcca catatctcca gagtgccgag atcttattgc gaggattttt    780
gtggccaacc cagccactag aatctctatc cccgagatca gaaatcatcc atggttcttg    840
aagaatctcc cagctgacct tatggatgat agcaagatga gcagccagta cgaggagccc    900
gaacagccaa tgcagagcat ggatgagatc atgcagatac tggcagaggc gaccatacca    960
gcagctgggt ctggtggaat caaccagttc ttgaatgatg gccttgacct cgatgatgac   1020
atggaggacc ttgattcaga ccccgatctt gacgtggaaa gcagtgggga gatagtatac   1080

gctatgtga                                                           1089
```

<210> 44
<211> 362
<212> PRT
<213> Oryza sativa

<400> 44

```
Met Asp Arg Ala Ala Leu Thr Val Gly Pro Gly Met Asp Met Pro Ile
1               5                   10                  15

Met His Asp Gly Asp Arg Tyr Glu Leu Val Arg Asp Ile Gly Ser Gly
            20                  25                  30

Asn Phe Gly Val Ala Arg Leu Met Arg Ser Arg Ala Asp Gly Gln Leu
        35                  40                  45

Val Ala Val Lys Tyr Ile Glu Arg Gly Asp Lys Ile Asp Glu Asn Val
    50                  55                  60

Gln Arg Glu Ile Ile Asn His Arg Ser Leu Arg His Pro Asn Ile Ile
65                  70                  75                  80

Arg Phe Lys Glu Val Ile Leu Thr Pro Thr His Leu Ala Ile Val Met
                85                  90                  95

Glu Tyr Ala Ser Gly Gly Glu Leu Phe Glu Arg Ile Cys Asn Ala Gly
            100                 105                 110

Arg Phe Ser Glu Asp Glu Ala Arg Phe Phe Phe Gln Gln Leu Ile Ser
        115                 120                 125

Gly Val Ser Tyr Cys His Ser Met Gln Val Cys His Arg Asp Leu Lys
    130                 135                 140

Leu Glu Asn Thr Leu Leu Asp Gly Ser Thr Ala Pro Arg Leu Lys Ile
145                 150                 155                 160

Cys Asp Phe Gly Tyr Ser Lys Ser Ser Val Leu His Ser Gln Pro Lys
                165                 170                 175

Ser Thr Val Gly Thr Pro Ala Tyr Ile Ala Pro Glu Val Leu Leu Lys
            180                 185                 190

Lys Glu Tyr Asp Gly Lys Ile Ala Asp Val Trp Ser Cys Gly Val Thr
        195                 200                 205

Leu Tyr Val Met Leu Val Gly Ala Tyr Pro Phe Glu Asp Pro Asp Glu
    210                 215                 220

Pro Lys Asn Phe Arg Lys Thr Ile Gln Arg Ile Leu Gly Val Gln Tyr
225                 230                 235                 240

Ser Ile Pro Asp Tyr Val His Ile Ser Pro Glu Cys Arg Asp Leu Ile
                245                 250                 255

Ala Arg Ile Phe Val Ala Asn Pro Ala Thr Arg Ile Ser Ile Pro Glu
            260                 265                 270
```

```
Ile Arg Asn His Pro Trp Phe Leu Lys Asn Leu Pro Ala Asp Leu Met
        275                 280                 285

Asp Asp Ser Lys Met Ser Ser Gln Tyr Glu Glu Pro Glu Gln Pro Met
    290                 295                 300

Gln Ser Met Asp Glu Ile Met Gln Ile Leu Ala Glu Ala Thr Ile Pro
305                 310                 315                 320

Ala Ala Gly Ser Gly Gly Ile Asn Gln Phe Leu Asn Asp Gly Leu Asp
                325                 330                 335

Leu Asp Asp Asp Met Glu Asp Leu Asp Ser Asp Pro Asp Leu Asp Val
            340                 345                 350

Glu Ser Ser Gly Glu Ile Val Tyr Ala Met
        355                 360
```

<210> 45
<211> 1080
<212> DNA
<213> Brassica napus

<400> 45

```
atggagaagt acgagctggt gaaagacata ggagctggga atttcggagt ggcgaggctc      60
atgaaggtca aagactctaa ggagctcgtt gccatgaagt acatcgagcg tggtcccaag     120
attgatgaga acgtggcaag agagatttat aatcacagat cgcttcgcca tcctaatatt     180
atccgcttta aggaggtggt gttgactccg actcatcttg ctattgccat ggagtatgct     240
gctggtggtg aacttttcga gcgtatatgc ggtgctggaa gattcagtga ggatgaggcg     300
agatacttct tccagcagct tatatcaggt gttagctatt gccatgctat gcaaatatgc     360
catagagatc tgaagctcga gaatacactc cttgatggaa gtcctgctcc acgtctcaaa     420
atctgtgatt ttggttattc caagtcctct ctactgcact cgaggcctaa atcaactgtt     480
ggaactccag catatattgc acctgaggtc ctctctcgga gagaatatga tggcaagatg     540
gctgatgtat ggtcctgtgg tgttactctt tatgtcatgc ttgttggagc ataccctttt     600
gaagaccagg aagaccccaa aaacttcagg aaaacaatac aaaaaatcat ggctgttcag     660
tacaagatcc cggactacgt ccacatctca caagattgca aacatctcct ttcccgtata     720
tttgtggcca actcactcaa gaggataacc attgcggaaa tcaagaaaca cccatggttc     780
acgaagaact tgccaaggga gctcacagag acagctcaag ctgcatattt caagaaagag     840
aatccaacct tctccgccca gaccgctgaa gagatcatga agatagtgga tgacgccaaa     900
acgcctccgc ctgtttcccg ttccattgga ggttttggct ggggaggaga gggagattta     960
gaggggaaag aggaagagga ggtggatgaa gaggaggttg aggaagagga agacgaagaa    1020
gatgaatatg ataagactgt aaaggaagta cacgcaagcg gagaagtgag aatcagttga    1080
```

<210> 46
<211> 359
<212> PRT
<213> Brassica napus

<400> 46

Met Glu Lys Tyr Glu Leu Val Lys Asp Ile Gly Ala Gly Asn Phe Gly
1               5                   10                  15

Val Ala Arg Leu Met Lys Val Lys Asp Ser Lys Glu Leu Val Ala Met
            20                  25                  30

Lys Tyr Ile Glu Arg Gly Pro Lys Ile Asp Glu Asn Val Ala Arg Glu
        35                  40                  45

Ile Tyr Asn His Arg Ser Leu Arg His Pro Asn Ile Ile Arg Phe Lys

```
            50                  55                  60

        Glu Val Val Leu Thr Pro Thr His Leu Ala Ile Ala Met Glu Tyr Ala
        65                  70                  75                  80

        Ala Gly Gly Glu Leu Phe Glu Arg Ile Cys Gly Ala Gly Arg Phe Ser
                        85                  90                  95

        Glu Asp Glu Ala Arg Tyr Phe Phe Gln Gln Leu Ile Ser Gly Val Ser
                    100                 105                 110

        Tyr Cys His Ala Met Gln Ile Cys His Arg Asp Leu Lys Leu Glu Asn
                115                 120                 125

        Thr Leu Leu Asp Gly Ser Pro Ala Pro Arg Leu Lys Ile Cys Asp Phe
            130                 135                 140

        Gly Tyr Ser Lys Ser Ser Leu Leu His Ser Arg Pro Lys Ser Thr Val
        145                 150                 155                 160

        Gly Thr Pro Ala Tyr Ile Ala Pro Glu Val Leu Ser Arg Arg Glu Tyr
                        165                 170                 175

        Asp Gly Lys Met Ala Asp Val Trp Ser Cys Gly Val Thr Leu Tyr Val
                    180                 185                 190

        Met Leu Val Gly Ala Tyr Pro Phe Glu Asp Gln Glu Asp Pro Lys Asn
                195                 200                 205

        Phe Arg Lys Thr Ile Gln Lys Ile Met Ala Val Gln Tyr Lys Ile Pro
            210                 215                 220

        Asp Tyr Val His Ile Ser Gln Asp Cys Lys His Leu Leu Ser Arg Ile
        225                 230                 235                 240

        Phe Val Ala Asn Ser Leu Lys Arg Ile Thr Ile Ala Glu Ile Lys Lys
                        245                 250                 255

        His Pro Trp Phe Thr Lys Asn Leu Pro Arg Glu Leu Thr Glu Thr Ala
                    260                 265                 270

        Gln Ala Ala Tyr Phe Lys Lys Glu Asn Pro Thr Phe Ser Ala Gln Thr
                275                 280                 285

        Ala Glu Glu Ile Met Lys Ile Val Asp Asp Ala Lys Thr Pro Pro Pro
            290                 295                 300

        Val Ser Arg Ser Ile Gly Gly Phe Gly Trp Gly Gly Glu Gly Asp Leu
        305                 310                 315                 320

        Glu Gly Lys Glu Glu Glu Glu Val Asp Glu Glu Glu Val Glu Glu Glu
                        325                 330                 335

        Glu Asp Glu Glu Asp Glu Tyr Asp Lys Thr Val Lys Glu Val His Ala
                    340                 345                 350

        Ser Gly Glu Val Arg Ile Ser
                355
```

<210> 47
<211> 1065
<212> DNA
<213> Brassica napus

<400> 47

```
atggagaagt acgagctggt gaaagacata ggcgctggga atttcggagt ggcgaggctc      60
atgaaggtca aaaactctaa agagcttgtt gccatgaagt acatcgagcg tggtcccaag     120
attgatgaga atgtggcaag agagatcatt aatcacagat cgcttcgtca tcctaatatt     180
atccgtttta aggaggttgt gttgactcca actcatcttg ctattgccat ggaatatgct     240
gctggtggtg aattattcga gcgtatatgc agtgctggaa gattcagtga ggatgaggcg     300
agatacttct tccagcagct tatatcaggt gttagctatt gccatgctat gcaaatatgc     360
catagagatc tgaagctcga gaacacactc ctggatggaa gtcctgctcc acgtctcaaa     420
atctgtgatt ttggttattc caagtcctct ctactgcact cgaggcccaa atccacagtt     480
ggaactccag catatattgc acctgaggtc ctttctcgga gagagtatga tggcaagatg     540
gctgatgtat ggtcttgtgg tgtaactctt tatgtcatgc ttgttggagc ctacccattc     600
gaagaccagg aagacccaaa gaacttcagg aaaacaatac aaaaaatcat ggctgttcag     660
tacaagatcc cggactacgt ccacatctca caggattgca aacacctcct ttcccgtata     720
tttgttgcca attcactcaa gaggataacc attgcagaaa tcaagaaaca cccatggttc     780
ctgaagaacc tgccaaggga gctcacagag acagctcaag ctgcatattt caagaaagag     840
aatccaacct tctccccgca gaccgctgaa gagatcatga agatagtgga tgacgccaaa     900
acgcctccgc ctgtttccag atccattgga gggtttggct ggggaggaaa gggagacgaa     960
gaggaagaag aagtggatga agaggaggtg gtggaggaag aggaagacga agaagatgaa    1020
tatgataaga ctgtaaagga agcacacgca agtggagaag tgtga                   1065
```

<210> 48
<211> 354
<212> PRT
<213> Brassica napus

<400> 48

```
Met Glu Lys Tyr Glu Leu Val Lys Asp Ile Gly Ala Gly Asn Phe Gly
1               5                   10                  15

Val Ala Arg Leu Met Lys Val Lys Asn Ser Lys Glu Leu Val Ala Met
            20                  25                  30

Lys Tyr Ile Glu Arg Gly Pro Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Val Leu Thr Pro Thr His Leu Ala Ile Ala Met Glu Tyr Ala
65                  70                  75                  80

Ala Gly Gly Glu Leu Phe Glu Arg Ile Cys Ser Ala Gly Arg Phe Ser
                85                  90                  95

Glu Asp Glu Ala Arg Tyr Phe Phe Gln Gln Leu Ile Ser Gly Val Ser
            100                 105                 110

Tyr Cys His Ala Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
```

```
145                 150                 155                 160

Gly Thr Pro Ala Tyr Ile Ala Pro Glu Val Leu Ser Arg Arg Glu Tyr
                165                 170                 175

Asp Gly Lys Met Ala Asp Val Trp Ser Cys Gly Val Thr Leu Tyr Val
            180                 185                 190

Met Leu Val Gly Ala Tyr Pro Phe Glu Asp Gln Glu Asp Pro Lys Asn
        195                 200                 205

Phe Arg Lys Thr Ile Gln Lys Ile Met Ala Val Gln Tyr Lys Ile Pro
    210                 215                 220

Asp Tyr Val His Ile Ser Gln Asp Cys Lys His Leu Leu Ser Arg Ile
225                 230                 235                 240

Phe Val Ala Asn Ser Leu Lys Arg Ile Thr Ile Ala Glu Ile Lys Lys
                245                 250                 255

His Pro Trp Phe Leu Lys Asn Leu Pro Arg Glu Leu Thr Glu Thr Ala
            260                 265                 270

Gln Ala Ala Tyr Phe Lys Lys Glu Asn Pro Thr Phe Ser Pro Gln Thr
        275                 280                 285

Ala Glu Glu Ile Met Lys Ile Val Asp Asp Ala Lys Thr Pro Pro Pro
    290                 295                 300

Val Ser Arg Ser Ile Gly Gly Phe Gly Trp Gly Gly Lys Gly Asp Glu
305                 310                 315                 320

Glu Glu Glu Glu Val Asp Glu Glu Glu Val Val Glu Glu Glu Glu Asp
                325                 330                 335

Glu Glu Asp Glu Tyr Asp Lys Thr Val Lys Glu Ala His Ala Ser Gly
            340                 345                 350

Glu Val
```

<210> 49
<211> 1056
<212> DNA
<213> Glycine max

<400> 49

```
atggataagt atgaggctgt caaggatttg ggagctggga attttggggt ggctaggctc      60
atgaggaaca aggagaccaa ggagcttgtt gccatgaaat acatcgagcg tggccaaaag     120
attgatgaga atgtggcaag agagattatc aaccacagat cccttcggca ccccaatata     180
attcgcttca aggaggtggt tttgaccccc acccatttgg ccatagtgat ggagtatgcg     240
gctggaggag agctctttga gaggatatgc aatgctggca ggttcagtga agatgaggct     300
agatatttct ttcagcagct gatttctggt gtacattact gtcatgccat gcaaatatgt     360
cacagagatt tgaagctaga aaataccctt ttagatggaa gccctgcacc ccgcctgaaa     420
atttgtgact ttggttattc caagtcatca ttacttcatt cacggccaaa atcaactgtt     480
ggaactccag cttatatagc accagaggtt ctttccagga gggagtatga tggcaagttg     540
gctgatgtat ggtcatgtgg agtgactctt tatgtcatgc tggttggagc ttatcccttt     600
gaggatcagg atgaccctag gaattttagg aaaacaattc agcgtataat ggctgttcaa     660
tacaaaatcc ctgattatgt tcacatatct caagactgca gacacctcct ttctcgtata     720

tttgtagcaa atccattaag gaggatctct cttaaggaaa ttaagagcca cccatggttt     780
ttaaagaatc ttccaagaga gctgactgaa tcagctcaag ctgtctatta ccagagaggc     840
aatccaagct tttcaattca aagtgtggag gagatcatga agattgtggg agaggcaagg     900
gaccctcctc cagtatctag acctgtcaaa ggttttggct gggatggcga agaagatgaa     960
ggggaagaag acgtggagga agaggaggac gaagaagacg agtatgacaa gagggtcaaa    1020
gaggttcatg caagtggaga atttcaaatc agttaa                              1056
```

<210> 50
<211> 351
<212> PRT
<213> Glycine max

<400> 50

```
Met Asp Lys Tyr Glu Ala Val Lys Asp Leu Gly Ala Gly Asn Phe Gly
1               5                   10                  15

Val Ala Arg Leu Met Arg Asn Lys Glu Thr Lys Glu Leu Val Ala Met
            20                  25                  30

Lys Tyr Ile Glu Arg Gly Gln Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Val Leu Thr Pro Thr His Leu Ala Ile Val Met Glu Tyr Ala
65                  70                  75                  80

Ala Gly Gly Glu Leu Phe Glu Arg Ile Cys Asn Ala Gly Arg Phe Ser
                85                  90                  95

Glu Asp Glu Ala Arg Tyr Phe Phe Gln Gln Leu Ile Ser Gly Val His
            100                 105                 110

Tyr Cys His Ala Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
145                 150                 155                 160

Gly Thr Pro Ala Tyr Ile Ala Pro Glu Val Leu Ser Arg Arg Glu Tyr
                165                 170                 175

Asp Gly Lys Leu Ala Asp Val Trp Ser Cys Gly Val Thr Leu Tyr Val
            180                 185                 190

Met Leu Val Gly Ala Tyr Pro Phe Glu Asp Gln Asp Asp Pro Arg Asn
        195                 200                 205

Phe Arg Lys Thr Ile Gln Arg Ile Met Ala Val Gln Tyr Lys Ile Pro
    210                 215                 220

Asp Tyr Val His Ile Ser Gln Asp Cys Arg His Leu Leu Ser Arg Ile
225                 230                 235                 240

Phe Val Ala Asn Pro Leu Arg Arg Ile Ser Leu Lys Glu Ile Lys Ser
```

245 250 255

His Pro Trp Phe Leu Lys Asn Leu Pro Arg Glu Leu Thr Glu Ser Ala
260 265 270

Gln Ala Val Tyr Tyr Gln Arg Gly Asn Pro Ser Phe Ser Ile Gln Ser
275 280 285

Val Glu Glu Ile Met Lys Ile Val Gly Glu Ala Arg Asp Pro Pro Pro
290 295 300

Val Ser Arg Pro Val Lys Gly Phe Gly Trp Asp Gly Glu Glu Asp Glu
305 310 315 320

Gly Glu Glu Asp Val Glu Glu Glu Glu Asp Glu Glu Asp Glu Tyr Asp
325 330 335

Lys Arg Val Lys Glu Val His Ala Ser Gly Glu Phe Gln Ile Ser
340 345 350

<210> 51
<211> 1050
<212> DNA
<213> Glycine max

<400> 51

```
atggataagt acgaggctgt taaggatttg ggagctggca attttggggt ggctaggctc     60
atgaggaaca aggtcaccaa ggagcttgta gccatgaaat acatcgagcg tggccccaag    120
attgatgaga acgtggcaag ggagattatg aaccacaggt cccttcggca tcccaatata    180
attcgttaca aggaggtggt tttgactccc acacatttag caatagtgat ggagtatgca    240
gcaggaggag agctctttga gaggatatgc agtgctggca ggttcagtga agatgaggct    300
agatatttct ttcagcagct gatttccggt gttcatttct gtcataccat gcaaatatgc    360
cacagagatt tgaagctaga aaataccctt ctagatggaa gtcctgcacc tcggttgaaa    420
atttgtgact tcggttattc caagtcatct ttgctgcact cacgacccaa atcaacagtt    480
ggaacaccag cttacatagc accggaagtt ctttctaggc gagagtatga cggaaagttg    540
gctgatgtat ggtcatgtgc ggtgactctt tatgtcatgc tggttggagc atatcccttt    600
gaggaccagg atgaccctag gaattttagg aaaacaattc agcgtataat ggctgttcaa    660
tacaaaatcc ctgattatgt tcacatatct caagattgta ggcacctcct ctctcgtata    720
tttgttgcaa atccattgag gagaattact attaaggaaa ttaagaatca cccatggttt    780
ttgaggaatc ttccaaggga gctaactgaa tctgctcaag ctatctatta ccagagagac    840
agcccaaact ttcaccttca aagtgtggat gagataatga aaattgtagg agaggcaaga    900
aatccacctc cagtatctag ggctctcaaa ggttttggct gggaaggtga agaagatttg    960
gatgaagaag tggaggaaga agaggatgaa gatgagtatg ataagagggt caaagaggtt   1020
catgcaagtg gcgaatttca aattagttaa                                    1050
```

<210> 52
<211> 349
<212> PRT
<213> Glycine max

<400> 52

```
Met Asp Lys Tyr Glu Ala Val Lys Asp Leu Gly Ala Gly Asn Phe Gly
1               5                   10                  15

Val Ala Arg Leu Met Arg Asn Lys Val Thr Lys Glu Leu Val Ala Met
            20                  25                  30

Lys Tyr Ile Glu Arg Gly Pro Lys Ile Asp Glu Asn Val Ala Arg Glu
        35                  40                  45
```

```
Ile Met Asn His Arg Ser Leu Arg His Pro Asn Ile Ile Arg Tyr Lys
    50                  55                  60

Glu Val Val Leu Thr Pro Thr His Leu Ala Ile Val Met Glu Tyr Ala
65                  70                  75                  80

Ala Gly Gly Glu Leu Phe Glu Arg Ile Cys Ser Ala Gly Arg Phe Ser
                85                  90                  95

Glu Asp Glu Ala Arg Tyr Phe Phe Gln Gln Leu Ile Ser Gly Val His
            100                 105                 110

Phe Cys His Thr Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
145                 150                 155                 160

Gly Thr Pro Ala Tyr Ile Ala Pro Glu Val Leu Ser Arg Arg Glu Tyr
                165                 170                 175

Asp Gly Lys Leu Ala Asp Val Trp Ser Cys Ala Val Thr Leu Tyr Val
            180                 185                 190

Met Leu Val Gly Ala Tyr Pro Phe Glu Asp Gln Asp Asp Pro Arg Asn
        195                 200                 205

Phe Arg Lys Thr Ile Gln Arg Ile Met Ala Val Gln Tyr Lys Ile Pro
    210                 215                 220

Asp Tyr Val His Ile Ser Gln Asp Cys Arg His Leu Leu Ser Arg Ile
225                 230                 235                 240

Phe Val Ala Asn Pro Leu Arg Arg Ile Thr Ile Lys Glu Ile Lys Asn
                245                 250                 255

His Pro Trp Phe Leu Arg Asn Leu Pro Arg Glu Leu Thr Glu Ser Ala
            260                 265                 270

Gln Ala Ile Tyr Tyr Gln Arg Asp Ser Pro Asn Phe His Leu Gln Ser
        275                 280                 285

Val Asp Glu Ile Met Lys Ile Val Gly Glu Ala Arg Asn Pro Pro Pro
    290                 295                 300

Val Ser Arg Ala Leu Lys Gly Phe Gly Trp Glu Gly Glu Glu Asp Leu
305                 310                 315                 320

Asp Glu Glu Val Glu Glu Glu Glu Asp Glu Asp Glu Tyr Asp Lys Arg
                325                 330                 335

Val Lys Glu Val His Ala Ser Gly Glu Phe Gln Ile Ser
            340                 345
```

<210> 53
<211> 1071

<212> DNA
<213> Nicotiana tabacum

<400> 53

```
atggataaat acgagcttgt gaaagatata gggtcaggga attttggtgt ggcaaggctg     60
atgaggcaca aggaaaccaa agaacttgtg gcaatgaaat acattgagag aggacataag    120
attgatgaga atgtagcaag ggagatcatt aatcatagat cgcttcggca tccaaacata    180
attcgattca aggaggtgtt agtgactccc actcatcttg ccattgttat ggaatatgca    240
gctggtggag aactgtttga gcgcatttgc aatgcaggaa ggtttagtga agatgaggct    300
aggtactttt tccagcagct tatttcagga gttcactact gtcacaacat gcaaatatgc    360
catagagatt tgaagctgga gaatacgctt cttgatggaa gtccagctcc acgcttgaag    420
atatgtgatt ttggatactc aaagtcgtcc ctgttgcatt cgaggccaaa atcaactgtt    480
gggactccag cttatattgc tcctgaggtc ctatcaagaa gagaatatga tggcaagctg    540
gctgatgttt ggtcatgcgg ggtgacactt tatgtgatgc tggttggggc atatcctttt    600
gaagatcagg aggatccgaa gaattttagg aaaactattc aacgaataat ggcggtacag    660
tacaagattc ccgactatgt tcacatatca caagattgta ggcaccttct ctctcggata    720
tttgttgcta atccagcaag gaggatcaca atcaaagaaa tcaagtctca cccatggttt    780
ttgaagaatt tgccgaggga attaacagaa gcagcacagg cagcttatta cagaagagaa    840
aacccaacat tttcacttca gagtgttgag gagatcatga aaattgtgga agaggcaaag    900
actcccgctc cagcttcccg ttcagtctca ggctttggct ggggaggaga agaagaagaa    960
gaggagaagg aaggagatgt agaagaagag gaagaggatg aagaagaaga agacgaatat   1020
gaaaagcaag tgaagcaggc acatgaaagc ggagaagttc gtctcaccta a            1071
```

<210> 54
<211> 356
<212> PRT
<213> Nicotiana tabacum

<400> 54

```
Met Asp Lys Tyr Glu Leu Val Lys Asp Ile Gly Ser Gly Asn Phe Gly
1               5                   10                  15

Val Ala Arg Leu Met Arg His Lys Glu Thr Lys Glu Leu Val Ala Met
            20                  25                  30

Lys Tyr Ile Glu Arg Gly His Lys Ile Asp Glu Asn Val Ala Arg Glu
        35                  40                  45

Ile Ile Asn His Arg Ser Leu Arg His Pro Asn Ile Ile Arg Phe Lys
    50                  55                  60

Glu Val Leu Val Thr Pro Thr His Leu Ala Ile Val Met Glu Tyr Ala
65                  70                  75                  80

Ala Gly Gly Glu Leu Phe Glu Arg Ile Cys Asn Ala Gly Arg Phe Ser
                85                  90                  95

Glu Asp Glu Ala Arg Tyr Phe Phe Gln Gln Leu Ile Ser Gly Val His
            100                 105                 110

Tyr Cys His Asn Met Gln Ile Cys His Arg Asp Leu Lys Leu Glu Asn
        115                 120                 125

Thr Leu Leu Asp Gly Ser Pro Ala Pro Arg Leu Lys Ile Cys Asp Phe
    130                 135                 140

Gly Tyr Ser Lys Ser Ser Leu Leu His Ser Arg Pro Lys Ser Thr Val
145                 150                 155                 160
```

Gly Thr Pro Ala Tyr Ile Ala Pro Glu Val Leu Ser Arg Arg Glu Tyr
165 170 175

Asp Gly Lys Leu Ala Asp Val Trp Ser Cys Gly Val Thr Leu Tyr Val
180 185 190

Met Leu Val Gly Ala Tyr Pro Phe Glu Asp Gln Glu Asp Pro Lys Asn
195 200 205

Phe Arg Lys Thr Ile Gln Arg Ile Met Ala Val Gln Tyr Lys Ile Pro
210 215 220

Asp Tyr Val His Ile Ser Gln Asp Cys Arg His Leu Leu Ser Arg Ile
225 230 235 240

Phe Val Ala Asn Pro Ala Arg Arg Ile Thr Ile Lys Glu Ile Lys Ser
245 250 255

His Pro Trp Phe Leu Lys Asn Leu Pro Arg Glu Leu Thr Glu Ala Ala
260 265 270

Gln Ala Ala Tyr Tyr Arg Arg Glu Asn Pro Thr Phe Ser Leu Gln Ser
275 280 285

Val Glu Glu Ile Met Lys Ile Val Glu Glu Ala Lys Thr Pro Ala Pro
290 295 300

Ala Ser Arg Ser Val Ser Gly Phe Gly Trp Gly Gly Glu Glu Glu Glu
305 310 315 320

Glu Glu Lys Glu Gly Asp Val Glu Glu Glu Glu Glu Asp Glu Glu Glu
325 330 335

Glu Asp Glu Tyr Glu Lys Gln Val Lys Gln Ala His Glu Ser Gly Glu
340 345 350

Val Arg Leu Thr
355

<210> 55
<211> 2193
<212> DNA
<213> Oryza sativa

<400> 55

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag attttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc tttttcgat tagatgcaag      540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
```

```
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtacctttttg tttgaggtaa aatcagagca ccggtgattt tgcttggtgt   1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga atttttttttc ccaaatatct taaaaagtca   1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccattttttaa ttatatgaaa tgaactgtag cataagcagt attcatttgg   1920
attatttttt ttattagctc tcacccccttc attattctga gctgaaagtc tggcatgaac   1980
tgtcctcaat tttgttttca aattcacatc gattatctat gcattatcct cttgtatcta    2040
cctgtagaag tttcttttttg gttattcctt gactgcttga ttacagaaag aaatttatga   2100
agctgtaatc gggatagtta tactgcttgt tcttatgatt catttccttt gtgcagttct    2160
tggtgtagct tgccactttc accagcaaag ttc                                 2193
```

## Description

The present invention relates generally to the field of molecular biology and concerns a method for increasing yield in plants grown under non-stress conditions by increasing the activity of an SNF1 related protein kinase (SnRK2) or a homologue thereof in a plant. The present invention also concerns plants having increased expression of a nucleic acid encoding an SnRK2 protein kinase or a homologue thereof, which plants have increased yield characteristics relative to corresponding wild type plants. Constructs useful in the methods of the invention are also provided.

Given the ever-increasing world population, and the dwindling area of land available for agriculture, it remains a major goal of agricultural research to improve the efficiency of agriculture and to increase the diversity of plants in horticulture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic complements that may not always result in the desirable trait being passed on from parent plants. Advances in molecular biology have allowed mankind to manipulate the germplasm of animals and plants. Genetic engineering of plants entails the isolation and manipulation of genetic material (typically in the form of DNA or RNA) and the subsequent introduction of that genetic material into a plant. Such technology has led to the development of plants having various improved economic, agronomic or horticultural traits. Traits of particular economic interest are growth characteristics such as high yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance may also be important factors in determining yield. Crop yield may therefore be increased by optimising one of the abovementioned factors.

The yeast protein kinase SNF1 is reportedly involved in the response to glucose starvation stress. It supposedly takes part in activating genes that are repressed by glucose by phosphorylating the repressor protein Mig1. SNF1 has orthologues in other organisms such as the AMP-activated protein kinase (AMPK) in mammals. AMPK becomes activated by increased 5'-AMP concentrations as a result of ATP depletion, which may be caused by stress conditions, including heat shock or glucose starvation. Plants also have SNF1-related kinases, named SnRKs. Plant SnRKs are divided in three subgroups, SnRK1 to SnRK3. The SnRK1 subgroup is most closely related to SNF1, both structurally and functionally; whereas the subgroups SnRK2 and SnRK3 may be unique to plants. The SnRK2 proteins lack the C-terminal regulatory domain found in SNF1, but instead have at their C-terminus an acidic stretch of glutamic and aspartic acids. SnRK2 proteins have a molecular weight of around 40kDa and are encoded by a small gene family: both *Arabidopsis* and rice have been reported to have 10 *SnRK2* genes. The first plant SNF1-related protein kinase 2 (SnRK2), designated PKABA1, was isolated by Anderberg and Walker-Simmons (Proc. Natl. Acad. Sci. USA 89, 10183-10187, 1992). It was found to be induced by abscisic acid (ABA) and dehydration. Later, related proteins were isolated, such as ASK1 and ASK2, (Park et al., Plant Molecular Biology 22, 615-624, 1993). These genes were reported to be expressed in several plant organs, but were most abundant in leaves. Another member of the SnRK2 subgroup is OST1 (Mustilli et al., Plant Cell 14, 3089-3099, 2002). OST1 was expressed in stomatal guard cells and vascular tissue, and was postulated to act between perception of abscisic acid (ABA) and production of reactive oxygen species that elicits stomatal closure. In rice, all the SnRK2 proteins were found to be activated by hyperosmotic stress and some of them were also activated by ABA (Kobyashi et al., Plant Cell 16, 1163-1177, 2004). REK (renamed SAPK3, Kobyashi *et al*., 2004) was reported to be expressed in leaves and maturing seeds, but not in stems or roots. Recombinant REK proteins showed increased autophosphorylation activity in the presence of $Ca^{2+}$.

WO 98/05760 discloses more than 20 nucleotide sequences encoding proteins involved in phosphorus uptake and metabolism (psr proteins). One of these psr proteins is the protein kinase psrPK, a protein related to SnRK2 which is expressed upon phosphate starvation. It was speculated that this protein and other psr proteins would be useful in manipulating phosphorus metabolism, however none of the proposed phenotypes, many of them relating to increased stress resistance, were enabled. Assmann and Li (WO 01/02541) described the protein kinase AAPK, another relative of SnRK2. Loss of function of AAPK was reported to reduce sensitivity to abscisic acid-induced stomatal closure. It was therefore suggested that the opposite, (increased expression or increased activity of AAPK) would result in plants with increased drought stress resistance. The authors however did not show that this was indeed the case. So far the available experimental data for SnRK2-related proteins mainly suggested a role in stress responses of plants.

None of the prior art documents has demonstrated or suggested that increased expression or increased activity and/or expression of an SnRK2 protein results in yield increase, relative to corresponding wild type and unstressed plants.

It has now surprisingly been found that increasing activity and/or expression of an SnRK2 protein in plants results in plants having improved growth characteristics, and in particular yield, relative to corresponding wild type plants. These results were obtained under standard plant growth conditions, and the yield increase is not the consequence of increased stress resistance.

Structurally, SnRK2 proteins are serine/threonine protein kinases, with a catalytic domain that is classified in the SMART database as an S_TKc type (SMART Accession number SM00220). The active site corresponds to the PROSITE signature, PS00108 (Prosite, Swiss Institute of Bioinformatics, http://us.expasy.org):

**[LIVMFYC]-x-[HY]-x-D-[LIVMFY]-K-x(2)-N-[LIVMFYCT](3)**

The C-terminal part comprises a stretch of poly (Glu and/or Asp) residues of unknown function.

According to one embodiment of the present invention there is provided a method for improving growth characteristics of a plant comprising increasing activity and/or expression in a plant of an SnRK2 polypeptide or a homologue thereof and optionally selecting for plants having improved growth characteristics.

Advantageously, performance of the method according to the present invention results in plants having a variety of improved growth characteristics, such as improved yield, improved biomass, each relative to corresponding wild type plants. Preferably, the improved growth characteristics comprise at least increased yield relative to corresponding wild type plants. Preferably, the increased yield is increased biomass and/or increased seed yield, which includes one or more of increased number of (filled) seeds, increased total weight of seeds, increased thousand kernel weight and increased harvest index. It should be noted that the yield increase is not the consequence of increased stress resistance.

The term "increased yield" as defined herein is taken to mean an increase in any one or more of the following, each relative to corresponding wild type plants: (i) increased biomass (weight) of one or more parts of a plant, particularly aboveground (harvestable) parts, increased root biomass or increased biomass of any other harvestable part; (ii) increased total seed yield, which includes an increase in seed biomass (seed weight) and which may be an increase in the seed weight per plant or on an individual seed basis; (iii) increased number of (filled) seeds; (iv) increased seed size; (v) increased seed volume; (vi) increased individual seed area; (vii) increased individual seed length; (viii) increased harvest index, which is expressed as a ratio of the yield of harvestable parts, such as seeds, over the total biomass; (ix) increased number of florets per panicle which is extrapolated from the total number of seeds counted and the number of primary panicles; and (x) increased thousand kernel weight (TKW), which is extrapolated from the number of filled seeds counted and their total weight. An increased TKW may result from an increased seed size (length, width or both) and/or seed weight. An increased TKW may result from an increase in embryo size and/or endosperm size.

Taking corn as an example, a yield increase may be manifested as one or more of the following: increase in the number of plants per hectare or acre, an increase in the number of ears per plant, an increase in the number of rows, number of kernels per row, kernel weight, TKW, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in TKW, among others.

Preferably, performance of the methods according to the present invention results in plants having increased yield and more particularly, increased biomass and/or increased seed yield. Preferably, the increased seed yield comprises an increase in one or more of number of (filled) seeds, total seed weight, seed size, thousand kernel weight and harvest index, each relative to control plants. Therefore, according to the present invention, there is provided a method for increasing plant yield, which method comprises increasing activity and/or expression in a plant of an SnRK2 polypeptide or a homologue thereof.

Since the improved plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts or cell types of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, flowering time and speed of seed maturation. An increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently increased, it may allow for the sowing of further seeds of the same plant species (for example sowing and harvesting of rice plants followed by sowing and harvesting of further rice plants all within one conventional growing period). Similarly, if the growth rate is sufficiently increased, it may allow for the sowing of further seeds of different plants species (for example the sowing and harvesting of rice plants followed by, for example, the sowing and optional harvesting of soy bean, potatoes or any other suitable plant). Harvesting additional times from the same rootstock in the case of some plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves plotting growth experiments, such parameters may be: T-Mid (the time taken for plants to reach 50% of their maximal size) and T-90 (time taken for plants to reach 90% of their maximal size), amongst others.

An increase in yield occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Typical abiotic or environmental stresses include temperature stresses caused by atypical hot or cold/ freezing temperatures; salt stress; water stress (drought or excess water). Abiotic stresses may also be caused by chemicals. Biotic stresses are typically those stresses caused by pathogens, such as bacteria, viruses, fungi and insects.

The abovementioned growth characteristics may advantageously be improved in any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest or the genetic modification in the gene/nucleic add of interest. The term "plant" also encompasses plant cells, suspension cultures, callus tissue, embryos, meristematic regions, gametophytes, sporophytes, pollen, and microspores, again wherein each of the aforementioned comprise the gene/nucleic acid of interest.

Plants that are particularly useful in the methods of the invention include algae, ferns, and all plants which belong to the superfamily Viridiplantae, in particular monocotyledonous and dicotyledonous plants, including fodder or forage legumes, ornamental plants, food crops, trees, or shrubs selected from the list comprising *Abelmoschus* spp., *Acer* spp., *Actinidia* spp., *Agropyron* spp., *Allium* spp., *Amaranthus* spp., *Ananas comosus, Annona* spp., *Apium graveolens, Arabidopsis thaliana, Arachis* spp, *Artocarpus* spp., *Asparagus officinalis, Avena sativa, Averrhoa carambola, Benincasa hispida, Bertholletia excelsea, Beta vulgaris, Brassica* spp., *Cadaba farinosa, Camellia sinensis, Canna indica, Capsicum* spp., *Carica papaya, Carissa macrocarpa, Carthamus tinctorius, Carya* spp., *Castanea* spp., *Cichorium endivia, Cinnamomum spp., Citrullus lanatus, Citrus* spp., Cocos spp., *Coffea* spp., Cola spp., *Colocasia esculenta, Corylus* spp., *Crataegus* spp., *Cucumis* spp., *Cucurbita* spp., *Cynara* spp., *Daucus carota, Desmodium* spp., *Dimocarpus longan, Dioscorea* spp., *Diospyros* spp., *Echinochloa* spp., *Eleusine coracana, Eriobotrya japonica, Eugenia uniflora, Fagopyrum* spp., *Fagus* spp., *Ficus carica, Fortunella spp., Fragaria* spp., *Ginkgo biloba, Glycine* spp., *Gossypium hirsutum, Helianthus* spp., *Hibiscus* spp., *Hordeum* spp., *Ipomoea batatas, Juglans* spp., *Lactuca sativa, Lathyrus* spp., *Lemna spp., Lens culinaris, Linum usitatissimum, Litchi chinensis, Lotus* spp., *Luffa acutangula, Lupinus* spp., *Macrotyloma* spp., *Malpighia emarginata, Malus* spp., *Mammea americana, Mangifera indica, Manihot* spp., *Manilkara zapota, Medicago sativa, Melilotus* spp., *Mentha* spp., *Momordica* spp., *Morus nigra, Musa* spp., *Nicotiana* spp., *Olea* spp., *Opuntia* spp., *Ornithopus* spp., *Oryza* spp., *Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Rubus* spp., *Saccharum* spp., *Sambucus* spp., *Secale cereale, Sesamum* spp., *Solanum* spp., *Sorghum bicolor, Spinacia* spp., *Syzygium* spp., *Tamarindus indica, Theobroma cacao, Trifolium* spp., *Triticosecale rimpaui, Triticum* spp., *Vaccinium* spp., *Vicia* spp., *Vigna spp., Vitis* spp., *Zea mays, Zizania palustris, Ziziphus* spp., amongst others.

According to a preferred feature of the present invention, the plant is a crop plant comprising soybean, sunflower, canola, alfalfa, rapeseed or cotton. Further preferably, the plant according to the present invention is a monocotyledonous plant such as sugarcane, most preferably a cereal, such as rice, maize, wheat, millet, barley, rye, oats or sorghum.

The activity of an SnRK2 protein may be increased by increasing levels of the SnRK2 polypeptide. Alternatively, activity may also be increased when there is no change in levels of an SnRK2, or even when there is a reduction in levels of an SnRK2. This may occur when the intrinsic properties of the polypeptide are altered, for example, by making a mutant or selecting a variant that is more active that the wild type.

The term "SnRK2 or homologue thereof" as defined herein refers to a polypeptide comprising (i) a functional serine/threonine kinase domain, (ii) the conserved signature sequence W(F/Y)(L/M/R/T)(K/R)(N/G/R)(L/P/I)(P/L)(A/G/V/R/K/I)(D/E/V) (SEQ ID NO: 6) and (iii) an acidic C-terminal domain that starts from the last residue of SEQ ID NO: 6. The "SnRK2 or homologue thereof" has in increasing order of preference at least 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 2. The overall sequence identity is determined using a global alignment algorithm, such as the Needleman Wunsch algorithm in the program GAP (GCG Wisconsin Package, Accelrys).

Furthermore, such "SnRK2 or homologue thereof", when expressed under control of a GOS2 promoter in the *Oryza sativa* cultivar Nipponbare, increases aboveground biomass and/or seed yield compared to corresponding wild type plants. This increase in seed yield may be measured in several ways, for example as an increase of thousand kernel weight.

The various structural domains in an SnRK2 protein may be identified using specialised databases e.g. SMART (Schultz et al. (1998) Proc. Natl. Acad. Sci. USA 95, 5857-5864; Letunic et al. (2002) Nucleic Acids Res 30, 242-244; http://smart.embl-heidelberg.de/), InterPro (Mulder et al., (2003) Nucl. Acids. Res. 31, 315-318; http://www.ebi.ac.uk/interpro/), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004), http://www.expasy.org/prosite/) or Pfam (Bateman et al., Nucleic Acids Research 30(1):276-280 (2002), http://www.sanger.ac.uk/Software/Pfam/).

The kinase domain of SnRK2 is of a S_TKc type (SMART accession number SM00221, InterPro accession number IPR002290), and is functional in the sense that it has Ser/Thr kinase activity. The predicted active site (ICHRDLKLENTLL, wherein D is the predicted catalytic residue) corresponds to the PROSITE signature PS00108. The ATP binding site (IGAGNFGVARLMKVKNSKELVAMK) corresponds to the PROSITE signature PS00107.

Preferably, the conserved signature sequence of SEQ ID NO: 6 has the sequence: W(F/Y)(LM/R)K(N/R)(L/I)P(A/G/V/R/K/I)(D/E), more preferably, the conserved signature sequence of SEQ ID NO: 6 has the sequence: W(F/Y)LKNLP(R/K)E; most preferably, the conserved signature sequence of SEQ ID NO: 6 has the sequence: WFLKNLPRE.

The acidic C-terminal domain as used herein is defined as the C-terminal part of the SnRK2 protein starting from the last residue in the conserved signature sequence defined above (D or E in SEQ ID NO: 6), and which C-terminal part has an isoelectric point (pI) ranging between 2.6 and 4.1, preferably between 3.6 and 3.9, most preferably the pI of the acidic C-terminal domain is 3.7. The pI values are calculated using the EMBOSS package (Rice *et al.*, Trends in Genetics 16,276-277,2000).

Methods for the search and identification of SnRK2 homologues would be well within the realm of persons skilled in the art. Such methods comprise comparison of the sequences represented by SEQ ID NO: 1 or 2, in a computer readable format, with sequences that are available in public databases such as MIPS (http://mips.gsf.de/), GenBank (http://www.ncbi.nlm.nih.gov/Genbank/index.html) or EMBL Nucleotide Sequence Database (http://www.ebi.ac.uk/embl/index.html), using algorithms well known in the art for the alignment or comparison of sequences, such as GAP (Needleman and Wunsch, J. Mol. Biol. 48; 443-453 (1970)), BESTFIT (using the local homology algorithm of Smith and Waterman (Advances in Applied Mathematics 2; 482-489 (1981))), BLAST (Altschul, S.F., Gish, W., Miller, W., Myers, E.W. & Lipman, D.J., J. Mol. Biol. 215:403-410 (1990)), FASTA and TFASTA (W. R. Pearson and D. J. Lipman Proc.Natl.Acad.Sci. USA 85:2444- 2448 (1988)). The software for performing BLAST analysis is publicly available through the National Centre for Biotechnology Information (NCBI). The homologues mentioned below were identified using BLAST default parameters (BLOSUM62 matrix, gap opening penalty 11 and gap extension penalty 1) and preferably full-length sequences are used for analysis.

Examples of proteins falling under the definition of "SnRK2 polypeptide or a homologue thereof" include *Arabidopsis* proteins and proteins from other species such as rice, soybean and tobacco.

Two special forms of homology, orthologous and paralogous, are evolutionary concepts used to describe ancestral relationships of genes. The term "paralogous" relates to homologous genes that result from one or more gene duplications within the genome of a species. The term "orthologous" relates to homologous genes in different organisms due to ancestral relationship of these genes.

Paralogues of SnRK2 polypeptides may easily be identified by performing a BLAST analysis against a set of sequences from the same species as the query sequence. Orthologues in, for example, monocot plant species may easily be found by performing a so-called reciprocal blast search. This may be done by a first blast involving blasting the sequence in question (for example, SEQ ID NO: 1 or SEQ ID NO: 2, being from *Arabidopsis thaliana*) against any sequence database, such as the publicly available NCBI database which may be found at: http://www.ncbi.nlm.nih.gov. If orthologues in rice were sought, the sequence in question would be blasted against, for example, the 28,469 full-length cDNA clones from *Oryza sativa* Nipponbare available at NCBI. BLASTn or tBLASTX may be used when starting from nucleotides or BLASTP or TBLASTN when starting from the protein, with standard default values. The blast results may be filtered. The full-length sequences of either the filtered results or the non-filtered results are then blasted back (second blast) against the sequences of the organism from which the sequence in question is derived, *in casu Arabidopsis thaliana.* The results of the first and second blasts are then compared. An orthologue is found when the results of the second blast give as hits with the highest similarity a query *SnRK2* nucleic acid or SnRK2 polypeptide. If for a specific query sequence the highest hit is a paralogue of SnRK2 then such query sequence is also considered a homologue of SnRK2, provided that this homologue comprises a functional serine/threonine kinase domain, the conserved signature sequence of SEQ ID NO: 6 and an acidic C-terminal region as defined above. In the case of large families, ClustalW may be used, followed by the construction of a neighbour joining tree, to help visualize the clustering.

The term "homologues" as used herein also encompasses paralogues and orthologues of the proteins useful in the methods according to the invention. Paralogues from *Arabidopsis* include the proteins as given in the GenBank accessions NP_172563, NP_849834 (SEQ ID NO: 8), NP_201170 (SEQ ID NO: 10), NP_196476 (SEQ ID NO: 12), NP_567945 (SEQ ID NO: 14), NP_179885 (SEQ ID NO: 16), NP_201489 (SEQ ID NO: 18), NP_974170 (SEQ ID NO: 20), NP_190619 (SEQ ID NO: 22), NP_195711 (SEQ ID NO: 24). Orthologues and paralogues from rice (including GenBank accessions BAD17997 (SEQ ID NO: 26), BAD17998 (SEQ ID NO: 28), BAD17999 (SEQ ID NO: 30), BAD18000 (SEQ ID NO: 32), BAD18001 (SEQ ID NO: 34), BAD18002 (SEQ ID NO: 36), BAD18003 (SEQ ID NO: 38), BAD18004 (SEQ ID NO: 40), BAD18005 (SEQ ID NO: 42), BAD18006 (SEQ ID NO: 44)), from *B. napus* (AAA33003 (SEQ ID NO: 46) and AAA33004 (SEQ ID NO: 48)), from soybean (AAB68961 (SEQ ID NO: 50) and AAB68962 (SEQ ID NO: 52)) and from tobacco (AAL89456 (SEQ ID NO: 54)) were identified using a reciprocal BLAST procedure. Preferably the orthologues and paralogues have the same structure and activity as SnRK2 and have the highest similarity to SnRK2 as represented by SEQ ID NO: 2 in a reciprocal BLAST search.

To determine the kinase activity of SnRK2, several assays are available and well known in the art (for example Current Protocols in Molecular Biology, Volumes 1 and 2, Ausubel et al. (1994), Current Protocols; or online such as http://www.protocol-online.org).

In brief, the kinase assay generally involves (1) bringing the kinase protein into contact with a substrate polypeptide containing the target site to be phosphorylated; (2) allowing phosphorylation of the target site in an appropriate kinase buffer under appropriate conditions; (3) separating phosphorylated products from non-phosphorylated substrate after a suitable reaction period. The presence or absence of kinase activity is determined by the presence or absence of a phosphorylated target. In addition, quantitative measurements may be performed.

Purified SnRK2 protein, or cell extracts containing or enriched in the SnRK2 protein could be used as source for the kinase protein. As a substrate, small peptides are particularly well suited. The peptide must comprise one or more serine, threonine, or tyrosine residues in a phosphorylation site motif. A compilation of phosphorylation sites may be found in Biochimica et Biophysica Acta 1314, 191-225, (1996). In addition, the peptide substrates may advantageously have a net positive charge to facilitate binding to phosphocellulose filters, (allowing to separate the phosphorylated from non-phosphorylated peptides and to detect the phosphorylated peptides). If a phosphorylation site motif is not known, a general tyrosine kinase substrate may be used. For example, "Src-related peptide" (RRLIEDAEYAARG) is a substrate for many receptor and non-receptor tyrosine kinases). To determine the kinetic parameters for phosphorylation of the synthetic peptide, a range of peptide concentrations is required. For initial reactions, a peptide concentration of 0.7-1.5 mM may be used.

For each kinase enzyme, it is important to determine the optimal buffer, ionic strength, and pH for activity. A standard 5x Kinase Buffer generally contains 5 mg/ml BSA (Bovine Serum Albumin preventing kinase adsorption to the assay tube), 150 mM Tris-Cl (pH 7.5), 100 mM $MgCl_2$. Divalent cations are required for most tyrosine kinases, although some tyrosine kinases (for example, insulin-, IGF-1-, and PDGF receptor kinases) require $MnCl_2$ instead of $MgCl_2$ (or in addition to $MgCl_2$). The optimal concentrations of divalent cations must be determined empirically for each protein kinase.

A commonly used donor of the phophoryl group is radio-labelled [gamma-$^{32}$P]ATP (normally at 0.2 mM final concentration). The amount of $^{32}$P incorporated in the peptides may be determined by measuring activity on the nitrocellulose dry pads in a scintillation counter.

Alternatively, the activity of an SnRK2 protein or homologue thereof may be assayed by expressing the SnRK2 protein or homologue thereof under control of a GOS2 promoter in the *Oryza sativa* cultivar Nipponbare, which results in plants with increased aboveground biomass and/or increased seed yield compared to corresponding wild type plants. This increase in seed yield may be measured in several ways, for example as an increase of thousand kernel weight.

The nucleic acid encoding an SnRK2 polypeptide or a homologue thereof may be any natural or synthetic nucleic acid. An SnRK2 polypeptide or a homologue thereof as defined hereinabove is encoded by an SnRK2 nucleic acid molecule. Therefore the term *"SnRK2* nucleic acid molecule" or *"SnRK2* gene" as defined herein is any nucleic acid molecule encoding an SnRK2 polypeptide or a homologue thereof as defined hereinabove. Examples of *SnRK2* nucleic acid molecules include those represented by SEQ ID NO: 1, and those encoding the above mentioned homologues. Functional variant *SnRK2* nucleic acids include portions of an SnRK2 nucleic acid molecule and/or nucleic acids capable of hybridising with an SnRK2 nucleic acid molecule. The term "functional" in the context of a functional variant refers to a variant *SnRK2* nucleic acid (i.e. a portion or a hybridising sequence), which encodes a polypeptide comprising a functional kinase domain, the conserved signature sequence of SEQ ID NO: 6 and an acidic C-terminal domain as defined above.

The SnRK2 type kinases in plants have a modular structure, consisting of a kinase domain and an acidic E and/or D rich domain. Preferred variants include those generated by domain deletion, stacking or shuffling (see for example He et al., Science 288, 2360-2363, 2000; or US patents 5,811,238 and 6,395,547).

The term portion as defined herein refers to a piece of DNA comprising at least 700 nucleotides and which portion comprises a functional kinase domain, the conserved signature sequence of SEQ ID NO: 6 and an acidic C-terminal domain as defined above. A portion may be prepared, for example, by making one or more deletions to an SnRK2 nucleic acid. The portions may be used in isolated form or they may be fused to other coding (or non coding) sequences in order to, for example, produce a protein that combines several activities, one of them being protein kinase activity. When fused to other coding sequences, the resulting polypeptide produced upon translation may be bigger than that predicted for the *SnRK2* fragment. Portions useful in the methods of the present invention comprise at least a functional kinase domain, the conserved signature sequence of SEQ ID NO: 6 and an acidic C-terminal domain as defined above. The functional portion may be a portion of a nucleic acids as represented by any one of SEQ ID NO: 7, 9, 11, 13, 15, 17, 19, 21, 23, 25, 27, 29, 31, 33, 35, 37, 39, 41, 43, 45, 47, 49, 51 and 53. Preferably, the functional portion is a portion of a nucleic acid as represented by SEQ ID NO: 1.

The term "hybridisation" as defined herein is a process wherein substantially homologous complementary nucleotide sequences anneal to each other. The hybridisation process may occur entirely in solution, i.e. both complementary nucleic acids are in solution. The hybridisation process may also occur with one of the complementary nucleic acids immobilised to a matrix such as magnetic beads, Sepharose beads or any other resin. The hybridisation process may furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. The skilled artisan is aware of various parameters which may be altered during hybridisation and washing and which will either maintain or change the stringency conditions.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

• DNA-DNA hybrids (Meinkoth and Wahl, Anal. Biochem., 138: 267-284, 1984):

$$T_m = 81.5°C + 16.6x\log[Na^+]^a + 0.41x\%[G/C^b] - 500x[L^c]^{-1} - 0.61x\% \text{ formamide}$$

• DNA-RNA or RNA-RNA hybrids:

$$T_m = 79.8 + 18.5 (\log_{10}[Na^+]^a) + 0.58 (\%G/C^b) + 11.8 (\%G/C^b)^2 - 820/L^c$$

• oligo-DNA or oligo-RNA[d] hybrids:

For <20 nucleotides: $T_m = 2 (l_n)$

For 20-35 nucleotides: $T_m = 22 + 1.46 (l_n)$

[a] or for other monovalent cation, but only accurate in the 0.01-0.4 M range.

[b] only accurate for %GC in the 30% to 75% range.

[c] L = length of duplex in base pairs.

[d] Oligo, oligonucleotide; $l_n$, effective length of primer = 2×(no. of G/C)+(no. of A/T). *Note:* for each 1% formamide, the $T_m$ is reduced by about 0.6 to 0.7°C, while the presence of 6M urea reduces the $T_m$ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. More or less stringent conditions may also be selected. Generally, low stringency conditions are selected to be about 50°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with Rnase.

Examples of hybridisation and wash conditions are listed in table 1:

**Table 1:**

| Stringency Condition | Polynucleotide Hybrid ± | Hybrid Length (bp) ‡ | Hybridization Temperature and Buffer † | Wash Temperature and Buffer † |
|---|---|---|---|---|
| A | DNA:DNA | > or equal to 50 | 65°C 1×SSC; or 42°C, 1×SSC and 50% formamide | 65°C; 0.3×SSC |
| B | DNA:DNA | <50 | Tb*; 1×SSC | Tb*; 1×SSC |
| C | DNA:RNA | > or equal to 50 | 67°C 1×SSC; or 45°C, 1×SSC and 50% formamide | 67°C; 0.3×SSC |
| D | DNA:RNA | <50 | Td*; 1×SSC | Td*; 1×SSC |
| E | RNA:RNA | > or equal to 50 | 70°C 1×SSC; or 50°C, 1×SSC and 50% formamide | 70°C; 0.3×SSC |
| F | RNA:RNA | <50 | Tf*; 1×SSC | Tf*; 1×SSC |
| G | DNA:DNA | > or equal to 50 | 65°C 4×SSC; or 45°C, 4×SSC and 50% formamide | 65°C; 1×SSC |
| H | DNA:DNA | <50 | Th*; 4×SSC | Th*; 4×SSC |
| I | DNA:RNA | > or equal to 50 | 67°C 4×SSC; or 45°C, 4×SSC and 50% formamide | 67°C; 1×SSC |
| J | DNA:RNA | <50 | Tj*; 4 ×SSC | Tj*; 4 ×SSC |
| K | RNA:RNA | > or equal to 50 | 70°C 4×SSC; or 40°C, 6×SSC and 50% formamide | 67°C; 1×SSC |
| L | RNA:RNA | <50 | Tl*; 2 ×SSC | Tl*; 2×SSC |
| M | DNA:DNA | > or equal to 50 | 50°C 4×SSC; or 40°C, 6×SSC and 50% formamide | 50°C; 2×SSC |
| N | DNA:DNA | <50 | Tn*; 6 ×SSC | Tn*; 6×SSC |
| O | DNA:RNA | > or equal to 50 | 55°C 4×SSC; or 42°C, 6×SSC and 50% formamide | 55°C; 2×SSC |

(continued)

| Stringency Condition | Polynucleotide Hybrid ± | Hybrid Length (bp) ‡ | Hybridization Temperature and Buffer † | Wash Temperature and Buffer † |
|---|---|---|---|---|
| P | DNA:RNA | <50 | Tp*; 6 ×SSC | Tp*; 6×SSC |
| Q | RNA:RNA | > or equal to 50 | 60°C 4×SSC; or 45°C, 6×SSC and 50% formamide | 60°C.; 2×SSC |
| R | RNA:RNA | <50 | Tr*; 4 ×SSC | Tr*; 4×SSC |

‡ The "hybrid length" is the anticipated length for the hybridising nucleic acid. When nucleic acids of known sequence are hybridised, the hybrid length may be determined by aligning the sequences and identifying the conserved regions described herein.

† SSPE (1×SSPE is 0.15M NaCl, 10mM $NaH_2PO_4$, and 1.25mM EDTA, pH7.4) may be substituted for SSC (1×SSC is 0.15M NaCl and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 μg/ml denatured, fragmented salmon sperm DNA, 0.5% sodium pyrophosphate, and up to 50% formamide.

* Tb-Tr: The hybridisation temperature for hybrids anticipated to be less than 50 base pairs in length should be 5-10°C less than the melting temperature $T_m$ of the hybrids; the $T_m$ is determined according to the above-mentioned equations.

± The present invention also encompasses the substitution of any one, or more DNA or RNA hybrid partners with either a PNA, or a modified nucleic acid.

For the purposes of defining the level of stringency, reference may conveniently be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

For example, a nucleic acid encoding SEQ ID NO: 2 or a homologue thereof may be used in a hybridisation experiment. Alternatively fragments thereof may be used as probes. Depending on the starting pool of sequences from which the SnRK2 protein is to be identified, different fragments for hybridization may be selected. For example, when a limited number of homologues with a high sequence identity to SnRK2 are desired, a less conserved fragment may be used for hybridisation. By aligning SEQ ID NO: 2 and homologues thereof, it is possible to design equivalent nucleic acid fragments useful as probes for hybridisation.

After hybridisation and washing, the duplexes may be detected by autoradiography (when radiolabeled probes were used) or by chemiluminescence, immunodetection, by fluorescent or chromogenic detection, depending on the type of probe labelling. Alternatively, a ribonuclease protection assay may be performed for detection of RNA: RNA hybrids.

The *SnRK2* nucleic acid molecule or functional variant thereof may be derived from any natural or artificial source. The nucleic acid/gene or functional variant thereof may be isolated from a microbial source, such as bacteria, yeast or fungi, or from a plant, alga or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a dicotyledonous species, preferably from the family Brassicaceae, further preferably from *Arabidopsis thaliana.* More preferably, the *SnRK2* isolated from *Arabidopsis thaliana* is represented by SEQ ID NO: 1 and the SnRK2 amino acid sequence is as represented by SEQ ID NO: 2.

The SnRK2 polypeptide or homologue thereof may be encoded by an alternative splice variant of an SnRK2 nucleic acid molecule or gene. The term "alternative splice variant" as used herein encompasses variants of a nucleic acid sequence in which selected introns and/or exons have been excised, replaced or added. Such variants will be ones in which the biological activity of the protein as outlined above is retained, which may be achieved by selectively retaining functional segments of the protein. Such splice variants may be found in nature or may be manmade. Methods for making such splice variants are well known in the art. Preferred splice variants are all splice variants derived from the nucleic acid represented by SEQ ID NO: 3, such as SEQ ID NO: 1. Further preferred are splice variants encoding a polypeptide having a functional kinase domain flanked by the conserved signature sequence of SEQ ID NO: 6 and the C-terminal acidic domain defined above.

The homologue may also be encoded by an allelic variant of a nucleic acid encoding an SnRK2 polypeptide or a homologue thereof, preferably an allelic variant of the nucleic acid represented by SEQ ID NO: 1. Further preferably, the polypeptide encoded by the allelic variant has a functional kinase domain flanked by the conserved signature sequence of SEQ ID NO: 6 and the C-terminal acidic domain defined above. Allelic variants exist in nature and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

The activity and/or expression of an SnRK2 polypeptide or a homologue thereof may be increased by introducing a genetic modification (preferably in the locus of an SnRK2 gene). The locus of a gene as defined herein is taken to mean a genomic region which includes the gene of interest and 10 kb up- or downstream of the coding region.

The genetic modification may be introduced, for example, by any one (or more) of the following methods: TDNA activation, TILLING, site-directed mutagenesis, homologous recombination, directed evolution or by introducing and expressing in a plant a nucleic acid encoding an SnRK2 polypeptide or a homologue thereof. Following introduction of the genetic modification there follows a step of selecting for increased activity and/or expression of an SnRK2 polypeptide, which increase in activity and/or expression gives plants having increased yield.

T-DNA activation tagging (Hayashi et al. Science 258, 1350-1353, 1992) involves insertion of T-DNA usually containing a promoter (may also be a translation enhancer or an intron), in the genomic region of the gene of interest or 10KB up- or down stream of the coding region of a gene in a configuration such that such promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to overexpression of genes near to the inserted T-DNA. The resulting transgenic plants show dominant phenotypes due to overexpression of genes close to the introduced promoter. The promoter to be introduced may be any promoter capable of directing expression of a gene in the desired organism, in this case a plant. For example, constitutive, tissue-preferred, cell type-preferred and inducible promoters are all suitable for use in T-DNA activation.

A genetic modification may also be introduced in the locus of an SnRK2 gene using the technique of TILLING (Targeted Induced Local Lesions IN Genomes). This is a mutagenesis technology useful to generate and/or identify, and to isolate mutagenised variants of an SnRK2 nucleic acid molecule capable of exhibiting SnRK2 activity. TILLING also allows selection of plants carrying such mutant variants. These mutant variants may even exhibit higher SnRK2 activity than that exhibited by the gene in its natural form. TILLING combines high-density mutagenesis with high-throughput screening methods. The steps typically followed in TILLING are: (a) EMS mutagenesis (Redei and Koncz (1992), In: C Koncz, N-H Chua, J Schell, eds, Methods in Arabidopsis Research. World Scientific, Singapore, pp 16-82; Feldmann et a/., (1994) In: EM Meyerowitz, CR Somerville, eds, Arabidopsis. Cold Spring Harbor Laboratory Press, Cold Spring Harbor, NY, pp 137-172; Lightner and Caspar (1998), In: J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum Nature Biotechnol. 18, 455-457, 2000, Stemple Nature Rev. Genet. 5, 145-150, 2004).

Site directed mutagenesis may be used to generate variants of *SnRK2* nucleic acids or portions thereof that retain activity, namely, protein kinase activity. Several methods are available to achieve site directed mutagenesis, the most common being PCR based methods (See for example Ausubel et a/., Current Protocols in Molecular Biology. Wiley Eds. http://www.4ulr.com/products/currentprotocols/index.html).

Directed evolution may be used to generate functional variants of *SnRK2* nucleic acid molecules encoding SnRK2 polypeptides or homologues, or portions thereof having an increased biological activity as outlined above. Directed evolution consists of iterations of DNA shuffling followed by appropriate screening and/or selection (Castle et al., (2004) Science 304(5674): 1151-4; US patents 5,811,238 and 6,395,547).

TDNA activation, TILLING, site-directed mutagenesis and directed evolution are examples of technologies that enable the generation novel alleles and functional variants of *SnRK2* that retain SnRK2 function as outlined above and which are therefore useful in the methods of the invention.

Homologous recombination allows introduction in a genome of a selected nucleic acid at a defined selected position. Homologous recombination is a standard technology used routinely in biological sciences for lower organism such as yeast or the moss *Physcomitrella.* Methods for performing homologous recombination in plants have been described not only for model plants (Offringa et al. (1990) EMBO J. 9, 3077-3084) but also for crop plants, for example rice (Terada et a/., (2002) Nature Biotechnol. 20, 1030-1034; or Iida and Terada (2004) Curr. Opin. Biotechnol. 15, 132-138). The nucleic acid to be targeted (which may be an *SnRK2* nucleic acid molecule or functional variant thereof as hereinbefore defined) need not be targeted to the locus of an *SnRK2* gene, but may be introduced in, for example, regions of high expression. The nucleic acid to be targeted may be an improved allele used to replace the endogenous gene or may be introduced in addition to the endogenous gene.

According to a preferred embodiment of the invention, plant growth characteristics may be improved by introducing and expressing in a plant a nucleic acid encoding an SnRK2 polypeptide or a homologue thereof.

A preferred method for introducing a genetic modification (which in this case need not be in the locus of an *SnRK2* gene) is to introduce and express in a plant a nucleic acid encoding an SnRK2 polypeptide or a homologue thereof. An SnRK2 polypeptide or a homologue thereof as mentioned above is one having kinase activity and, in increasing order of preference, having at least 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence represented by SEQ ID NO: 2, and furthermore comprising a kinase domain, the conserved signature sequence as represented by SEQ ID NO: 6 and a C-terminal acidic domain as defined above.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions (Table 2). To produce such homologues, amino acids of the protein may be replaced by other amino acids having similar properties (such as similar hydrophobicity, hydrophilicity, antigenicity, propensity to form or break $\alpha$-helical structures or $\beta$-sheet structures). Conservative substitution tables are well known in the art (see for example Creighton (1984) Proteins. W.H. Freeman and Company).

**Table 2**: Examples of conserved amino acid substitutions:

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Less conserved substitutions may be made in case the above-mentioned amino acid properties are not so critical.

A homologue may also be in the form of an "insertional variant" of a protein, i.e. where one or more amino acid residues are introduced into a predetermined site in a protein. Insertions may comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)6-tag, glutathione S-transferase-tag, protein A, maltose-binding protein, dihydrofolate reductase, Tag 100 epitope, c-myc epitope, FLAG®-epitope, lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

The SnRK2 polypeptide or homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as presented in SEQ ID NO: 2. "Derivatives" of a protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

According to a preferred aspect of the present invention, enhanced or increased expression of the *SnRK2* nucleic acid molecule is envisaged. Methods for obtaining enhanced or increased expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of an *SnRK2* nucleic acid or functional variant thereof. For example, endogenous promoters may be altered *in vivo* by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region may be derived from a natural gene, from a variety of other plant genes, or from T-DNA. The 3' end sequence to be added may be derived from, for example, the nopaline synthase or octopine synthase genes, or alternatively from other plant genes, or less preferably from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8, 4395-4405 (1988); Callis et al., Genes Dev. 1, 1183-1200 (1987)). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

Constructs and vectors are provided to facilitate introduction and/or expression of the nucleotide sequences useful in the methods according to the invention.

Therefore, there is provided a gene construct comprising:

(i) an *SnRK2* nucleic acid molecule or functional variant thereof;
(ii) one or more control sequence capable of driving expression of the nucleic acid sequence of (i); and optionally
(iii) a transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

Plants are transformed with a vector comprising the sequence of interest (i.e., an *SnRK2* nucleic acid or functional variant thereof). The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The promoter may be an inducible promoter, i.e. having induced or increased transcription initiation in response to a developmental, chemical, environmental or physical stimulus. An example of an inducible promoter being a stress-inducible promoter, i.e. a promoter activated when a plant is exposed to various stress conditions, is the water stress induced promoter WSI18 Additionally or alternatively, the promoter may be a tissue-specific promoter, i.e. one that is capable of preferentially initiating transcription in certain tissues, such as the leaves, roots, seed tissue etc. An example of a seed-specific promoter is the rice oleosin 18 kDa promoter (Wu et al. (1998) J Biochem 123(3): 386-91).

Preferably, the *SnRK2* nucleic acid or functional variant thereof is operably linked to a constitutive promoter. The term "constitutive" as defined herein refers to a promoter that is expressed predominantly in at least one tissue or organ and predominantly at any life stage of the plant. Preferably the promoter is expressed predominantly throughout the plant. Preferably, the constitutive promoter capable of preferentially expressing the nucleic acid throughout the plant has a comparable expression profile to a GOS2 promoter. More preferably, the constitutive promoter has the same expression profile as the rice GOS2 promoter, most preferably, the promoter capable of preferentially expressing the nucleic acid throughout the plant is the GOS2 promoter from rice represented in SEQ ID NO: 55. It should be clear that the applicability of the present invention is not restricted to the *SnRK2* nucleic acid represented by SEQ ID NO: 1, nor is the applicability of the invention restricted to expression of an *SnRK2* nucleic acid when driven by a GOS2 promoter. Examples of other constitutive promoters that may also be used to drive expression of a *SnRK2* nucleic acid are shown in Table 3 below.

**Table 3**: Examples of constitutive promoters

| Gene Source | Expression Motif | Reference |
|---|---|---|
| Actin | Constitutive | McElroy et al, Plant Cell, 2: 163-171, 1990 |
| CAMV 35S | Constitutive | Odell et al, Nature, 313: 810-812, 1985 |
| CaMV 19S | Constitutive | Nilsson et al., Physiol. Plant. 100:456-462, 1997 |
| GOS2 | Constitutive | de Pater et al, Plant J Nov;2(6):837-44, 1992 |
| Ubiquitin | Constitutive | Christensen et al, Plant Mol. Biol. 18: 675-689, 1992 |
| Rice cyclophilin | Constitutive | Buchholz et al, Plant Mol Biol. 25(5): 837-43, 1994 |
| Maize H3 histone | Constitutive | Lepetit et al, Mol. Gen. Genet. 231:276-285,1992 |
| Actin 2 | Constitutive | An et al, Plant J. 10(1); 107-121, 1996 |

Optionally, one or more terminator sequences may also be used in the construct introduced into a plant. The term "terminator" encompasses a control sequence which is a DNA sequence at the end of a transcriptional unit which signals 3’ processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs may further include an origin of replication sequence, which is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as *npt*II that phosphorylates neomycin and kanamycin, or *hpt*, phosphorylating hygromycin), to herbicides (for example *bar* which provides resistance to Basta; *aroA* or *gox* providing resistance against glyphosate), or genes that provide a metabolic trait (such as *manA* that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

The invention also provides a method for the production of transgenic plants having increased yield, comprising introduction and expression in a plant of an *SnRK2* nucleic acid or a functional variant thereof.

More specifically, the present invention provides a method for the production of transgenic increased yield plants having, which method comprises:

(i) introducing into a plant or plant cell an *SnRK2* nucleic acid; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens et al. (1982) Nature 296, 72-74; Negrutiu et al. (1987) Plant Mol. Biol. 8, 363-373); electroporation of protoplasts (Shillito et al. (1985) Bio/Technol 3, 1099-1102); microinjection into plant material (Crossway et al. (1986) Mol. Gen. Genet. 202, 179-185); DNA or RNA-coated particle bombardment (Klein et al. (1987) Nature 327, 70) infection with (non-integrative) viruses and the like. Transgenic rice plants expressing an *SnRK2* transgene are preferably produced via *Agrobacterium*-mediated transformation using any of the well known methods for rice transformation, such as described in any of the following: published European patent application EP 1198985 A1, Aldemita and Hodges (Planta 199, 612-617, 1996); Chan et al. (Plant Mol. Biol. 22, 491-506, 1993), Hiei et al. (Plant J. 6, 271-282, 1994), which disclosures are incorporated by reference herein as if fully set forth. In the case of com transformation, the preferred method is as described in either Ishida et al. (Nature Biotechnol. 14, 745-50, 1996) or Frame et al. (Plant Physiol. 129, 13-22, 2002), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art. The cultivation of transformed plant cells into mature plants may thus encompass steps of selection and/or regeneration and/or growing to maturity.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention also encompasses the use of *SnRK2* nucleic acids and to the use of SnRK2 polypeptides thereof.

One such use relates to increasing yield of plants, such as increased biomass and/or increased seed yield. The seed yield may include one or more of the following: increased number of (filled) seeds, increased seed weight, increased harvest index, increased thousand kernel weight, among others.

*SnRK2* nucleic acids or variants thereof or SnRK2 polypeptides or homologues thereof may find use in breeding programmes in which a DNA marker is identified which may be genetically linked to an SnRK2 gene or variant thereof. The *SnRK2* or variants thereof or SnRK2 proteins or homologues thereof may be used to define a molecular marker. This DNA or protein marker may then be used in breeding programs to select plants having improved growth characteristics. The *SnRK2* gene or variant thereof may, for example, be a nucleic acid as represented by SEQ ID NO: 1, or a nucleic acid encoding any of the above mentioned homologues.

Allelic variants of an *SnRK2* gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place by, for example, PCR. This is followed by a selection step for selection of superior allelic variants of the sequence in question and which give improved growth characteristics in a plant. Selection is typically carried out by monitoring growth performance of plants containing different allelic variants of the sequence in question, for example, different allelic variants of SEQ ID NO: 1, or of nucleic acids encoding any of the above mentioned homologues. Growth performance may be monitored in a greenhouse or in the field. Further optional steps include crossing plants, in which the superior allelic variant was identified, with another plant. This could be used, for example, to make a combination of interesting phenotypic features.

An *SnRK2* nucleic acid or variant thereof may also be used as a probe for genetically and physically mapping the genes that they are a part of, and as markers for traits linked to those genes. Such information may be useful in plant breeding in order to develop lines with desired phenotypes. Such use of *SnRK2* nucleic acids or variants thereof requires only a nucleic acid sequence of at least 10 nucleotides in length. The *SnRK2* nucleic adds or variants thereof may be used as restriction fragment length polymorphism (RFLP) markers. Southern blots of restriction-digested plant genomic DNA may be probed with the *SnRK2* nucleic acids or variants thereof. The resulting banding patterns may then be subjected to genetic analyses using computer programs such as MapMaker (Lander et al. (1987) Genomics 1, 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the *SnRK2* nucleic acid or variant thereof in the genetic map previously obtained using this population (Botstein et al. (1980) Am. J. Hum. Genet. 32, 314-331).

The production and use of plant gene-derived probes for use in genetic mapping is described in Bematzky and Tanksley (Plant Mol. Biol. Reporter 4, 37-41, 1986). Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Nonmammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7, 149-154). Although current methods of FISH mapping favour use of large clones (several to several hundred kb; see Laan et al. (1995) Genome Res. 5, 13-20), improvements in sensitivity may allow performance of FISH mapping using shorter probes.

A variety of nucleic acid amplification-based methods of genetic and physical mapping may be carried out using the nucleic acids. Examples include allele-specific amplification (Kazazian (1989) J. Lab. Clin. Med. 11, 95-96), polymorphism of PCR-amplified fragments (CAPS; Sheffield et al. (1993) Genomics 16, 325-332), allele-specific ligation (Landegren et al. (1988) Science 241, 1077-1080), nucleotide extension reactions (Sokolov (1990) Nucleic Acid Res. 18, 3671), Radiation Hybrid Mapping (Walter et al. (1997) Nat. Genet. 7, 22-28) and Happy Mapping (Dear and Cook (1989) Nucleic Acid Res. 17, 6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

In this way, generation, identification and/or isolation of improved plants with altered SnRK2 activity and/or expression, displaying improved growth characteristics may be performed.

*SnRK2* nucleic acids or functional variants thereof or SnRK2 polypeptides or homologues thereof may also find use as growth regulators. Since these molecules have been shown to be useful in improving the growth characteristics of plants, they would also be useful growth regulators, such as herbicides or growth stimulators.

## Description of figures

The present invention will now be described with reference to the following figures in which:

**Fig. 1** gives a graphical overview of SnRK2. The pentagram represents the kinase domain whereas the C-terminal region in light grey represents the Asp and/or Glu rich acidic region.

**Fig. 2** shows a binary vector for transformation and expression in *Oryza sativa* of an *Arabidopsis thaliana SnRK2* (internal reference CDS0758) under the control of a rice GOS2 promoter (internal reference PRO0129).

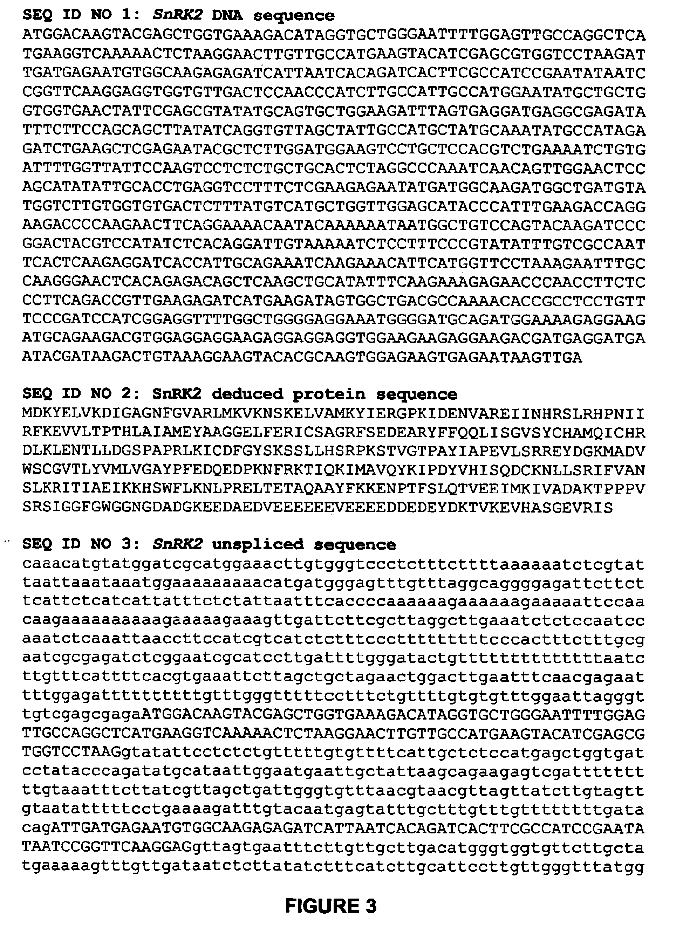

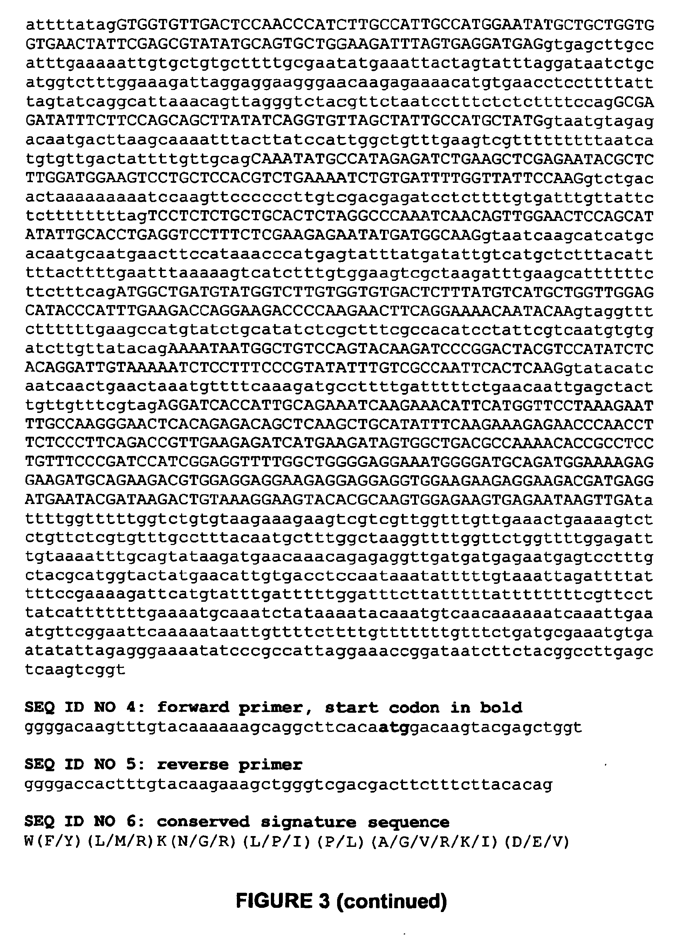

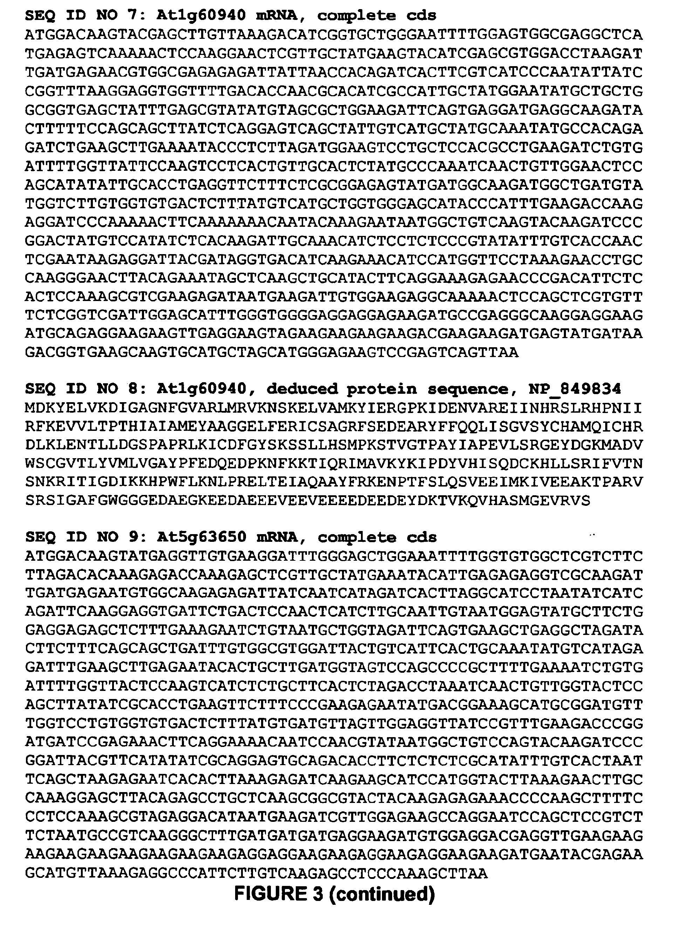

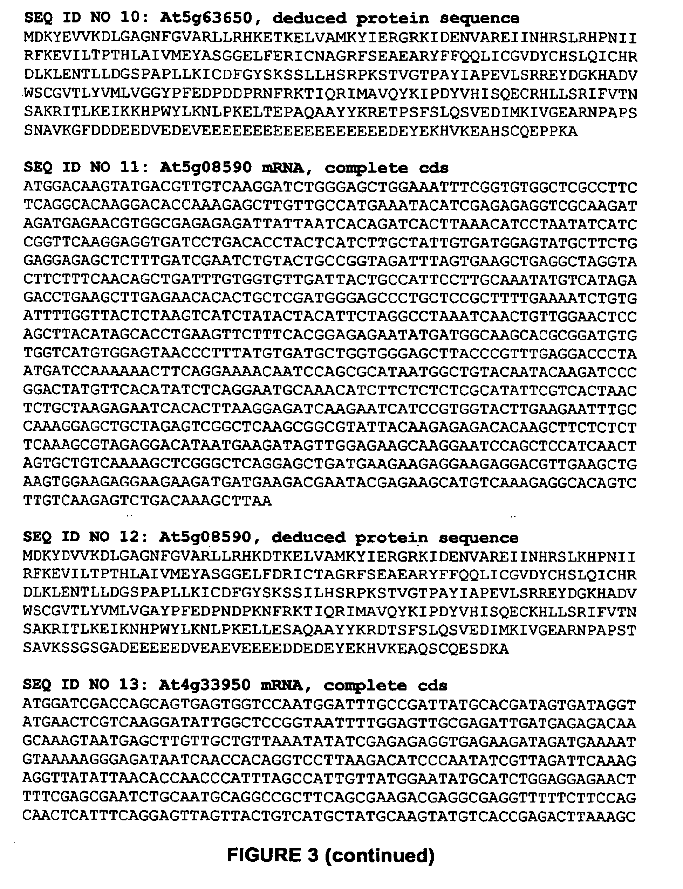

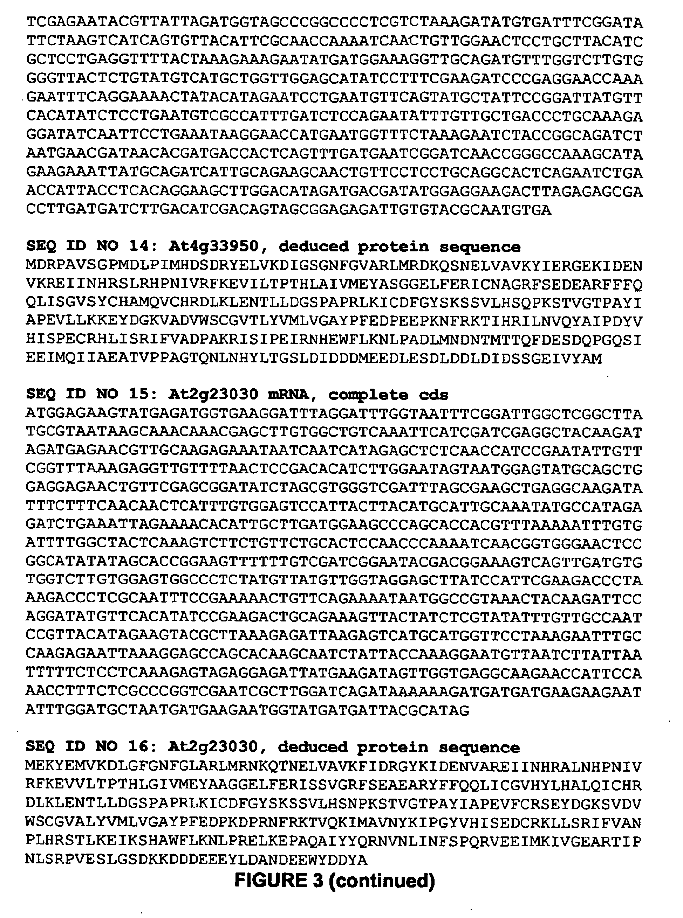

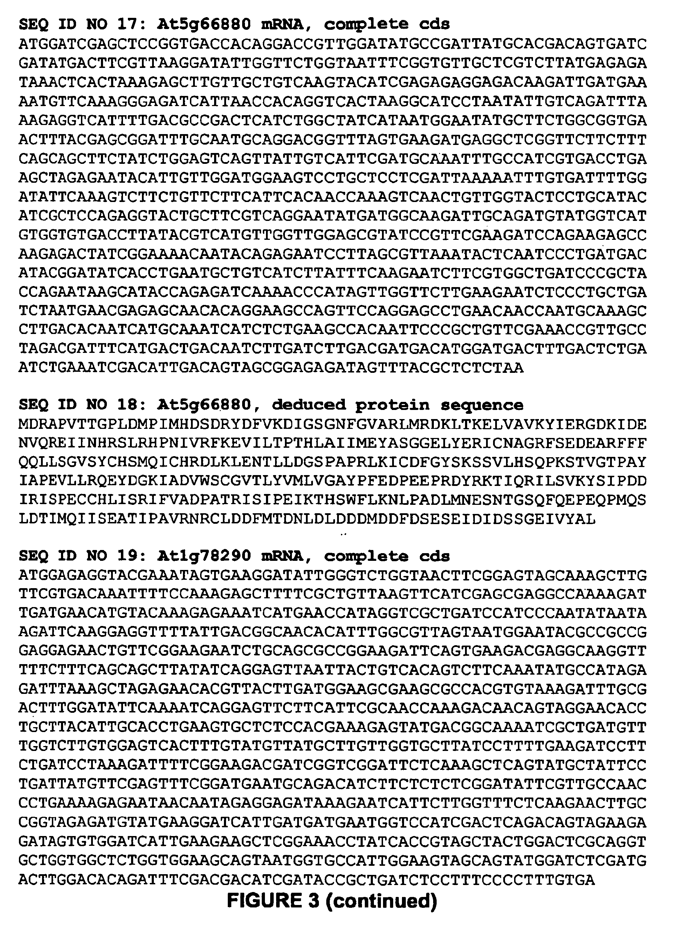

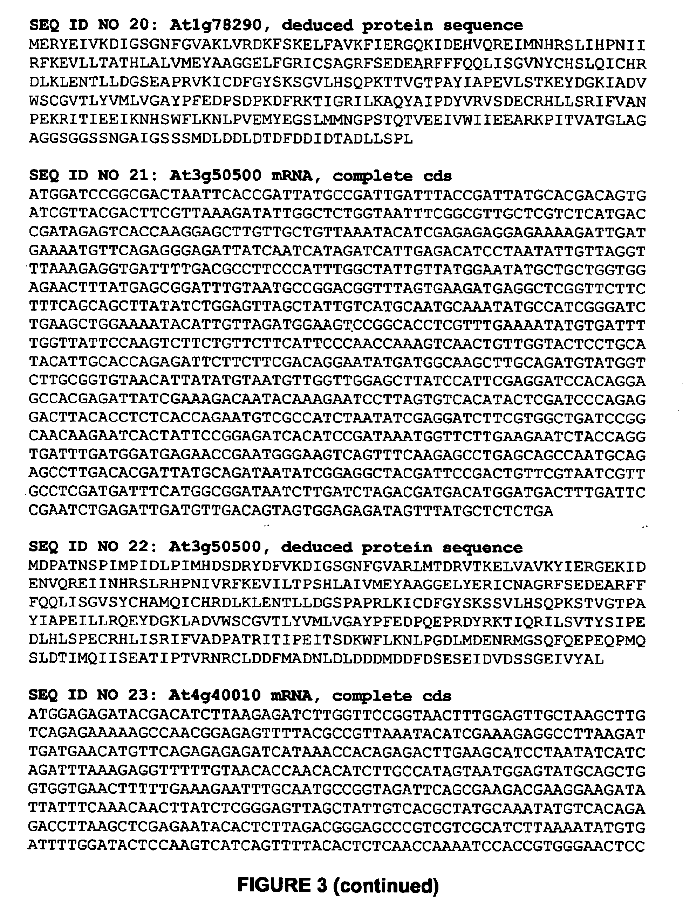

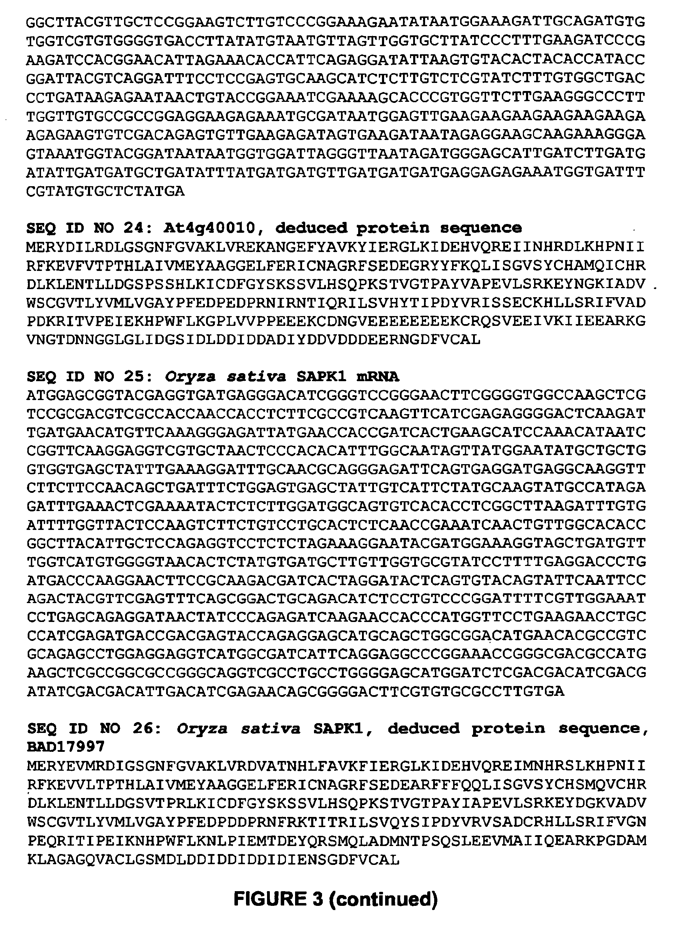

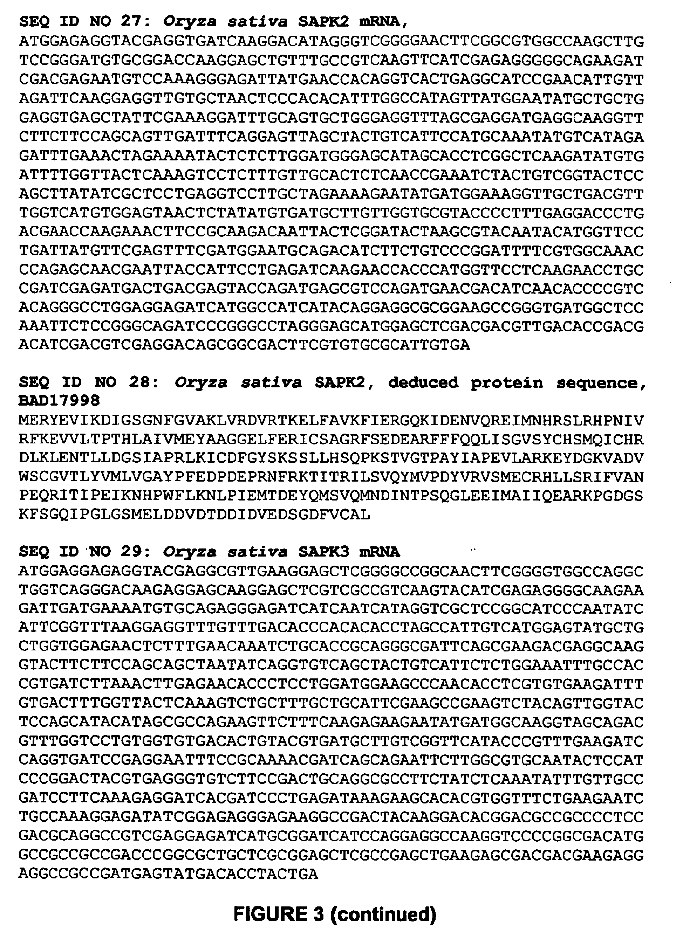

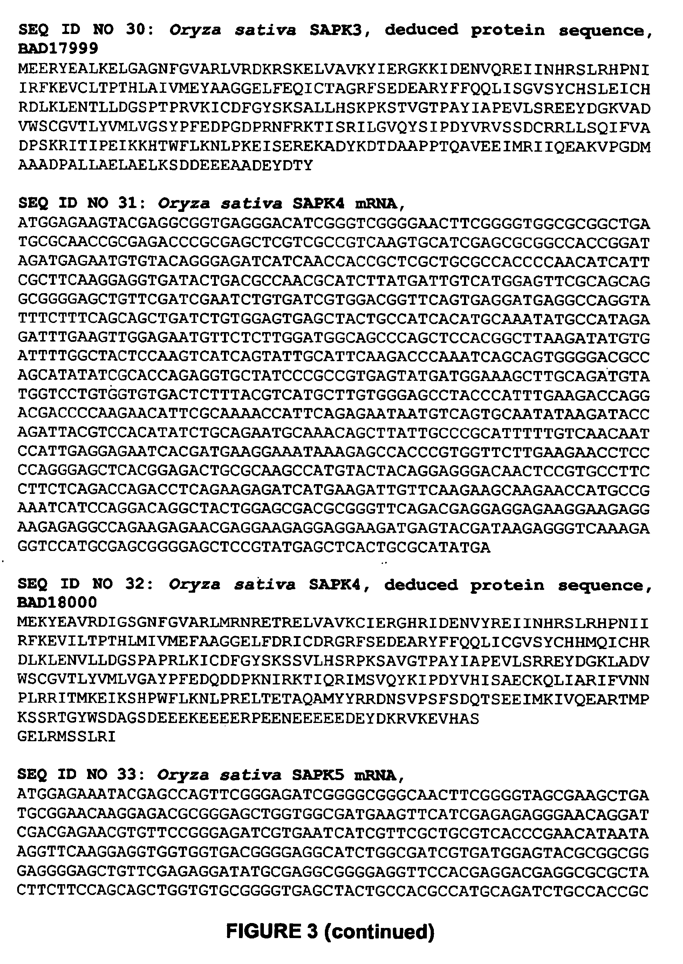

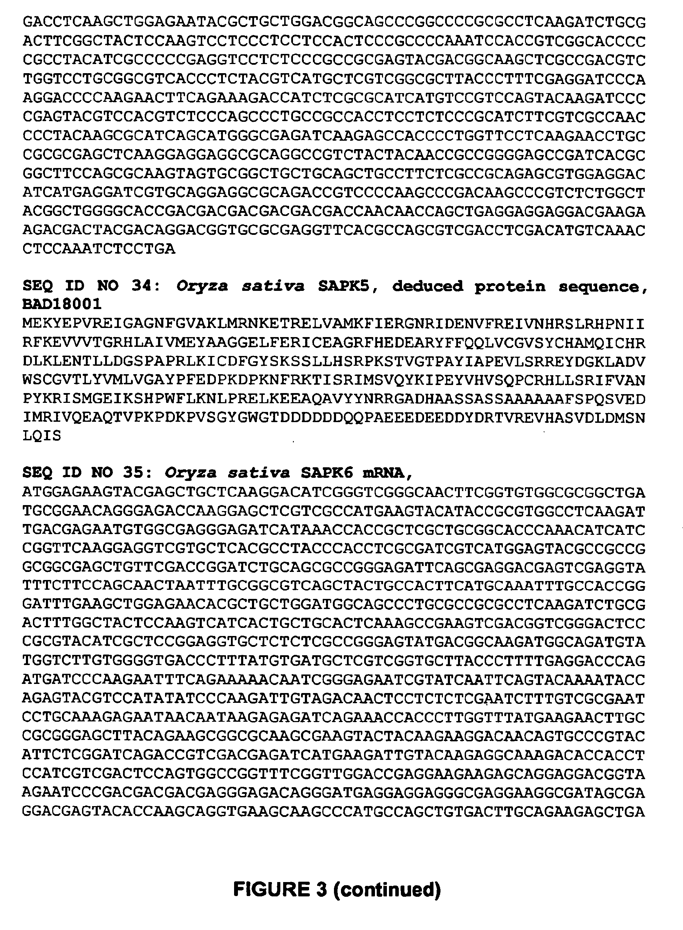

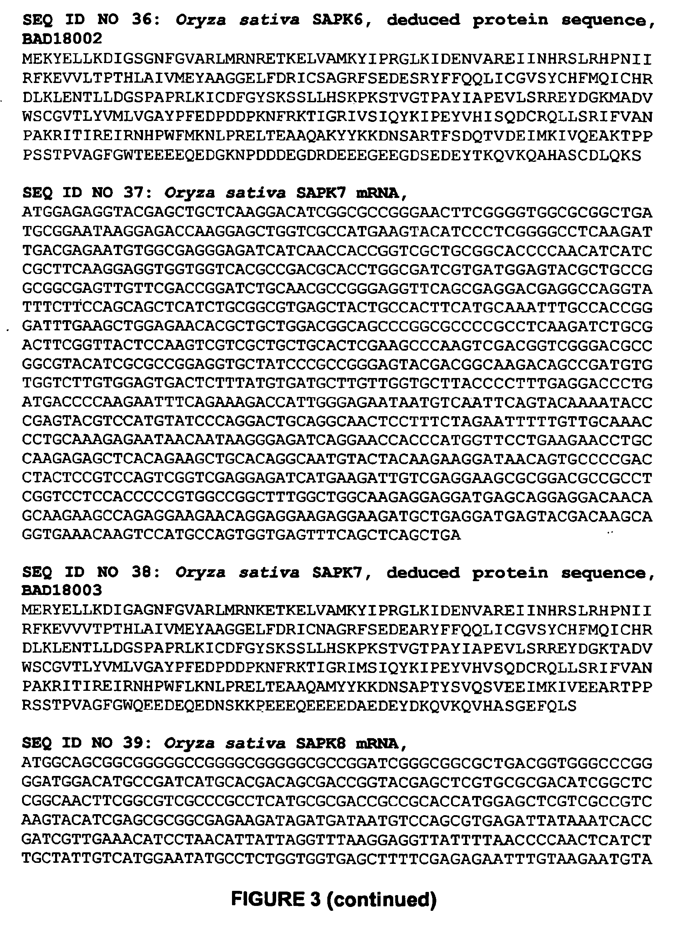

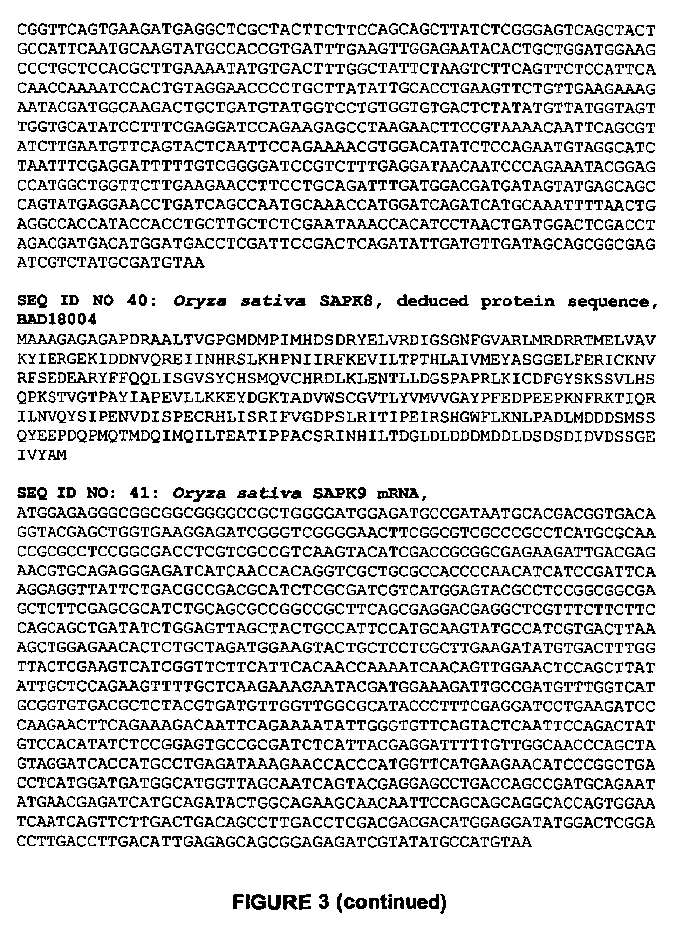

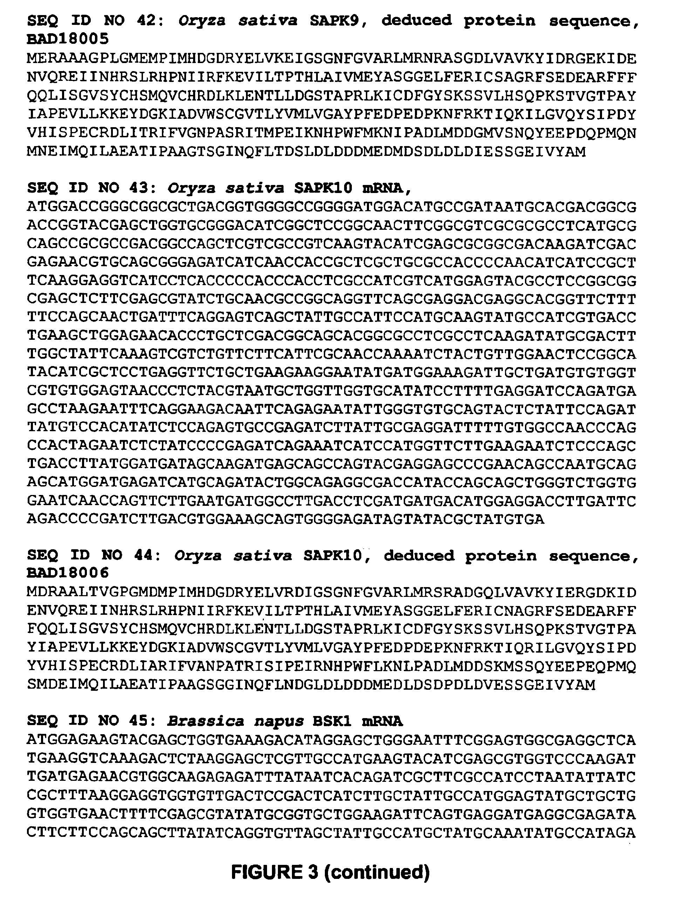

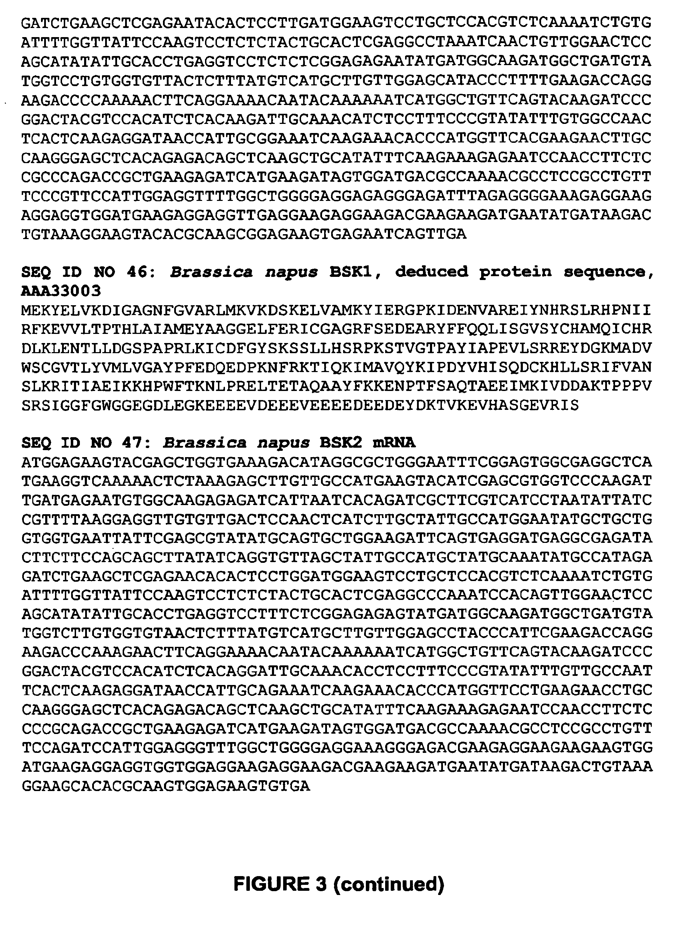

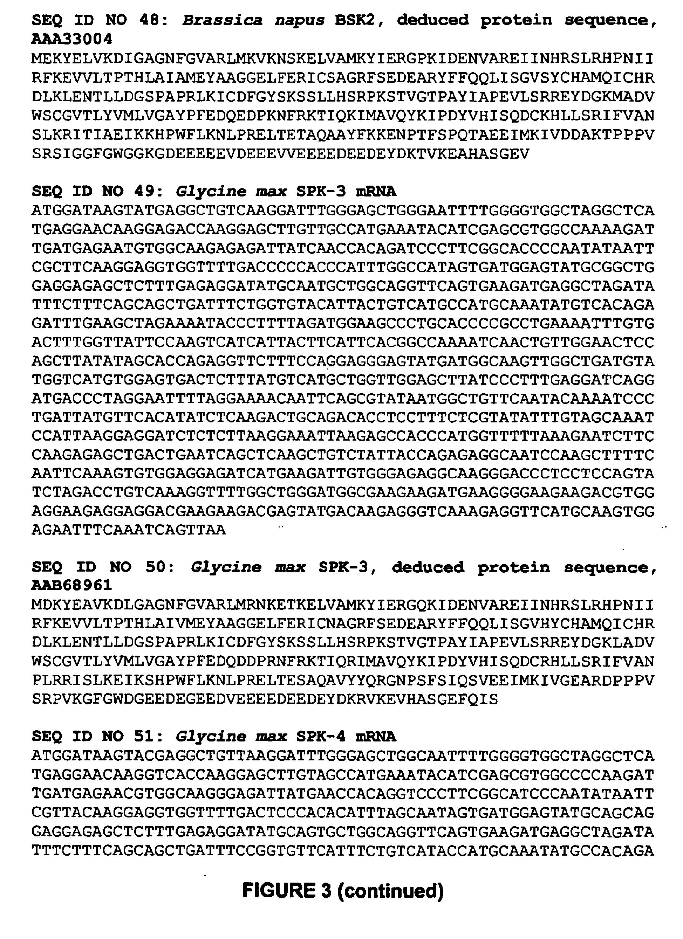

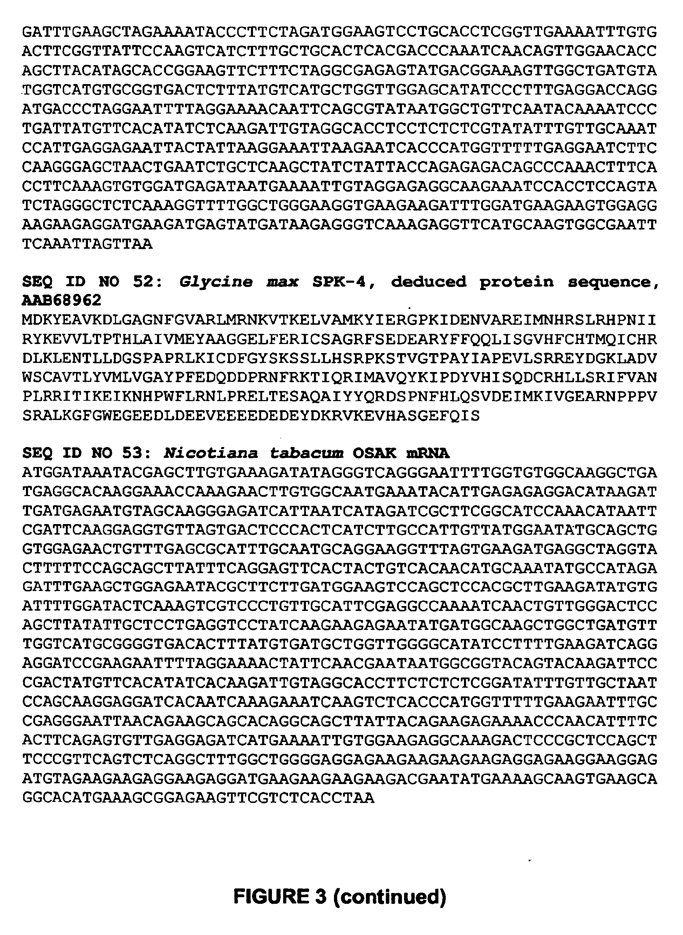

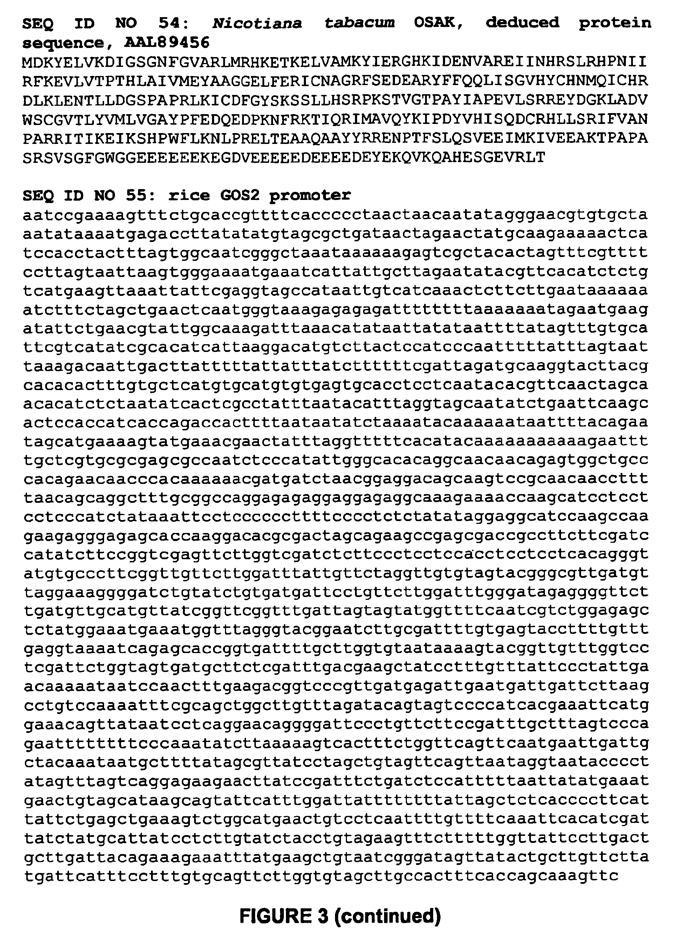

**Fig.3** details examples of sequences useful in performing the methods according to the present invention. SEQ ID NO: 1 and SEQ ID NO: 2 represent the nucleotide and protein sequence of SnRK2 used in the examples. SEQ ID NO: 3 represents the unspliced DNA sequence of *SnRK2.* SEQ ID NO: 4 and SEQ ID NO: 5 are primer sequences used for isolating the *SnRK2* nucleic acid. SEQ ID NO: 6 represents a consensus sequence of a conserved part in the SnRK2 proteins. SEQ ID NO: 7 to 53 are nucleotide and protein sequences of homologues of the SnRK2 coding sequence and protein sequence as given in SEQ ID NO: 1 and SEQ ID NO: 2.

**Examples**

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols (http://www.4ulr.com/products/currentprotocols/index.html). Standard materials and methods for plant molecular work are described in Plant Molecular Biology Labfax (1993) by R.D.D. Croy, published by BIOS Scientific Publications Ltd (UK) and Blackwell Scientific Publications (UK).

***Example 1: Gene Cloning***

The *Arabidopsis SnRK2* (internal code CDS0758) was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and the original number of clones was $1.59 \times 10^7$ cfu. Original titer was determined to be $9.6 \times 10^5$ cfu/ml, and after a first amplification of $6 \times 10^{11}$ cfu/ml. After plasmid extraction, 200 ng of template was used in a 50 µl PCR mix. Primers Prm02295 (SEQ ID NO: 4, sense) and Prm02296 (SEQ ID NO: 5, reverse complementary), which include the AttB sites for Gateway recombination, were used for PCR amplification. PCR was performed using Hifi Taq DNA polymerase in standard conditions. A PCR fragment of 1130 bp (without the attB sites) was amplified and purified also using standard methods. The first step of the Gateway procedure, the BP reaction, was then performed, during which the PCR fragment recombines *in vivo* with the pDONR201 plasmid to produce, according to the Gateway® terminology, an "entry clone", p028. Plasmid pDONR201 was purchased from Invitrogen, as part of the Gateway® technology.

***Example* 2: *Vector Construction and Rice Transformation***

The entry clone p028 was subsequently used in an LR reaction with p03069, a destination vector used for *Oryza sativa* transformation. This vector contained as functional elements within the T-DNA borders: a plant selectable marker; a visual marker expression cassette; and a Gateway cassette intended for LR *in vivo* recombination with the sequence of interest already cloned in the entry clone. A rice GOS2 promoter for constitutive expression was located upstream of this Gateway cassette.

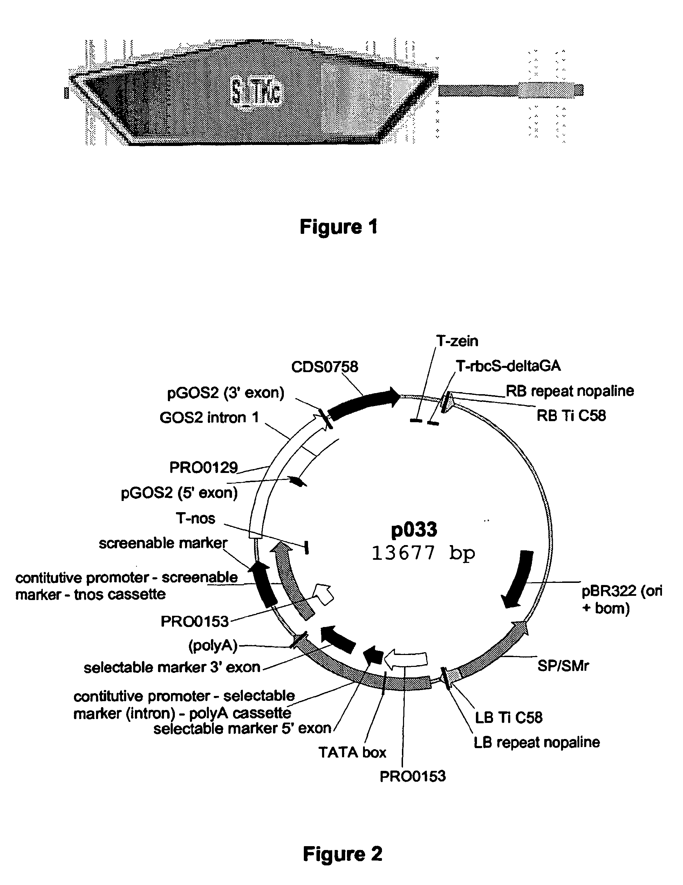

After the LR recombination step, the resulting expression vector p033 (Figure 2) was transformed into the *Agrobacterium strain* LBA4404 and subsequently to *Oryza sativa* plants. Transformed rice plants were allowed to grow and were then examined for the parameters described in Example 3.

***Example 3: Evaluation of Transformants: Growth Measurements***

Approximately 15 to 20 independent T0 transformants were generated. The primary transformants were transferred from tissue culture chambers to a greenhouse for growing and harvest of T1 seed. Five events of which the T1 progeny segregated 3:1 for presence/absence of the transgene were retained. For each of these events, 10 T1 seedlings containing the transgene (hetero- and homo-zygotes), and 10 T1 seedlings lacking the transgene (nullizygotes), were selected by visual marker screening. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.

The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of seed-related parameters described below.

These parameters were derived in an automated way from the digital images using image analysis software and were analysed statistically. A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants may also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

The data obtained in the first experiment were confirmed in a second experiment with T2 plants. Three lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.

A total number of 120 *SnRK2* transformed plants were evaluated in the T2 generation, that is 40 plants per event of which 20 positives for the transgene, and 20 negatives.

Because two experiments with overlapping events have been carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P-values are obtained by comparing likelihood ratio test to chi square distributions.

### *Example 4: Evaluation of Transformants: Measurement of Yield-Related Parameters*

Upon analysis of the seeds as described above, the inventors found that plants transformed with the *SnRK2* gene construct had a higher biomass (expressed as Total Areamax) and an increased Thousand Kernel Weight (TKW) compared to plants lacking the *SnRK2* transgene. Positive results obtained for plants in the T1 generation (increased Thousand Kernel Weight and a biomass increase of 9% (p-value 0.0309)) were again obtained in the T2 generation. In Table 4, data show the overall % increases for biomass and TKW, calculated from the data of the individual lines of the T2 generation, and the respective p-values from the F-test. These T2 data were re-evaluated in a combined analysis with the results for the T1 generation, and the obtained p-values show that the observed effects were significant.

**Table 4:**

| | T2 generation | | Combined analysis |
|---|---|---|---|
| | % difference | p-value | p-value |
| Total Areamax | +7 | 0.0158 | 0.0006 |
| TKW | +2 | 0.0107 | 0.0292 |

**Aboveground biomass:**

Plant aboveground area was determined by counting the total number of pixels from aboveground plant parts discriminated from the background. This value was averaged for the pictures taken on the same time point from the different angles and was converted to a physical surface value expressed in square mm by calibration (Total Areamax). Experiments show that the aboveground plant area measured this way correlates with the biomass of plant parts above ground. There was a significant increase in above ground biomass in the T1 generation, and this was confirmed in the T2 generation (with p-values of respectively 0.0309 in T1 and 0.0158 in T2). Also the combined analysis showed that the obtained increase in biomass was highly significant (p-value of 0.0006).

**Thousand Kernel Weight:**

The Thousand Kernel Weight (TKW) is extrapolated from the number of filled seeds counted and their total weight. There was a tendency for increased TKW in the T1 generation, and in the T2 generation, it was shown that the increase was a true overall effect and was significant. In particular, 2 of the four tested T2 lines showed a significantly increased TKW.

## Claims

1. Method for increasing yield in plants grown under non-stress conditions relative to control plants, comprising introducing and expressing in a plant an isolated *SnRK2* nucleic acid molecule encoding an SnRK2 polypeptide comprising the following:

   (i) a functional serine/threonine kinase domain;
   (ii) the conserved signature sequence W(F/Y)(L/M/R/T)(K/R)(N/G/R) (UP/I)(P/L)(AIGN/R/K/1)(D/EN) (SEQ ID NO: 6); and
   (iii) an acidic C-terminal domain that starts from the last residue of SEQ ID NO: 6.

2. Method of claim 1, wherein said *SnRK2* nucleic acid molecule is overexpressed in a plant.

3. Method according to claim 1 or 2, wherein said *SnRK2* nucleic acid molecule is of plant origin, preferably from a dicotyledonous plant, further preferably from the family *Brassicaceae*, more preferably the nucleic acid is from *Arabidopsis thaliana.*

4. Method according to any one of claims 1 to 3, wherein said *SnRK2* nucleic acid molecule is operably linked to a constitutive promoter.

5. Method according to claim 4, wherein said constitutive promoter is a GOS2 promoter.

6. Method according to any one of claims 1 to 5, wherein said increased yield is increased biomass and/or increased seed yield.

7. Method according to claim 6, wherein said increased seed yield is selected from any one or more of (i) increased seed biomass; (ii) increased number of (filled) seeds; (iii) increased seed size; (iv) increased seed volume; (v) increased harvest index (HI); and (vi) increased thousand kernel weight (TKW).

8. Use of an *SnRK2* nucleic acid molecule as defined in claim 1 or use of an SnRK2 polypeptide as defined in claim 1 in increasing yield in plants grown under non-stress conditions, in particular in improving yield, especially biomass and/or seed yield.

9. Use according to claim 8, wherein said improved seed yield comprises at least increased thousand kernel weight.

## Patentansprüche

1. Verfahren zum Erhöhen des Ertrags in Pflanzen, die im Vergleich zu Kontrollpflanzen unter Nichtstreßbedingungen herangezogen werden, umfassend das Einführen und Exprimieren eines isolierten *SnRK2*-Nukleinsäuremoleküls, das für ein SnRK2-Polypeptid, umfassend folgendes:

   (i) eine funktionelle Serin/Threoninkinasedomäne;
   (ii) die konservierte Signatursequenz W (F/Y) (L/M/R/T) (K/R) (N/G/R) (L/P/I) (P/L) (A/G/V/R/K/I) (D/E/V) (SEQ ID NO:6); und
   (iii) eine saure C-terminale Domäne, die beim letzten Rest der SEQ ID NO:6 beginnt,

   kodiert, in eine(r) Pflanze.

2. Verfahren nach Anspruch 1, wobei das *SnRK2*-Nukleinsäuremolekül in einer Pflanze überexprimiert wird.

3. Verfahren nach Anspruch 1 oder 2, wobei das *SnRK2*-Nukleinsäuremolekül von einer Pflanze, vorzugsweise von einer dikotylen Pflanze, stärker bevorzugt von der Familie *Brassicaceae*, stammt, noch stärker bevorzugt stammt die Nukleinsäure von *Arabidopsis thaliana.*

4. Verfahren nach einem der Ansprüche 1 bis 3, wobei das *SnRK2*-Nukleinsäuremolekül mit einem konstitutiven Promoter operativ verknüpft ist.

5. Verfahren nach Anspruch 4, wobei es sich bei dem konstitutiven Promoter um einen GOS2-Promoter handelt.

6. Verfahren nach einem der Ansprüche 1 bis 5, wobei es sich bei dem erhöhten Ertrag um erhöhten Biomasseertrag und/oder erhöhten Samenertrag handelt.

7. Verfahren nach Anspruch 6, wobei der erhöhte Samenertrag aus einem oder mehreren der folgenden ausgewählt ist: (i) erhöhte Samenbiomasse; (ii) erhöhte Anzahl an (gefüllten) Samen; (iii) erhöhte Samengröße; (iv) erhöhtes Samenvolumen; (v) erhöhter Harvest Index (HI); und (vi) erhöhtes Tausendkorngewicht (TKG).

8. Verwendung eines *SnRK2*-Nukleinsäuremoleküls wie in Anspruch 1 definiert oder Verwendung eines SnRK2-Polypeptids wie in Anspruch 1 definiert bei der Erhöhung des Ertrags in Pflanzen, die unter Nichtstreßbedingungen herangezogen werden, insbesondere bei der Erhöhung des Ertrags, speziell des Biomasse- und/oder Samenertrags.

9. Verwendung nach Anspruch 8, wobei der erhöhte Samenertrag mindestens ein erhöhtes Tausendkorngewicht umfaßt.

## Revendications

1. Méthode d'accroissement du rendement chez des plantes cultivées dans des conditions non stressantes par rapport à des plantes témoins, comprenant l'introduction et l'expression dans une plante d'une molécule d'acide nucléique *SnRK2* isolée codant pour un polypeptide SnRK2, comprenant ce qui suait :

   (i) un domaine sérine/thréonine kinase fonctionnel ;
   (ii) la séquence signature conservée W(F/Y) (L/M/R/T) (K/R) (N/G/R) (L/P/I) (P/L) (A/G/V/R/K/I) (D/E/V) (SEQ ID n°6) ; et
   (iii) un domaine C-terminal acide qui débute à partir du dernier résidu de la SEQ ID n°6.

2. Méthode selon la revendication 1, dans laquelle ladite molécule d'acide nucléique *SnRK2* est surexprimée chez une plante.

3. Méthode selon la revendication 1 ou 2, dans laquelle ladite molécule d'acide nucléique *SnRK2* est d'origine végétale, préférablement issue d'une plante dicotylédone, encore préférablement issue de la famille des Brassicacées, plus préférablement l'acide nucléique est issu d'*Arabidopsis thaliana*.

4. Méthode selon l'une quelconque des revendications 1 à 3, dans laquelle ladite molécule d'acide nucléique *SnRK2* est liée de manière opérante à un promoteur constitutif.

5. Méthode selon la revendication 4, dans laquelle ledit promoteur constitutif est un promoteur GOS2.

6. Méthode selon l'une quelconque des revendications 1 à 5, dans laquelle ledit rendement accru est une biomasse accrue et/ou un rendement accru en graines.

7. Méthode selon la revendication 6, dans laquelle ledit rendement accru en graines est choisi parmi un ou plusieurs quelconques parmi (i) une biomasse accrue en graines ; (ii) un nombre accru de graines (remplies) ; (iii) une taille accrue des graines ; (iv) un volume accru des graines ; (v) un indice de moisson (IM) accru ; et (vi) un poids de mille grains accru (PMG).

8. Utilisation d'une molécule d'acide nucléique *SnRK2* telle que définie selon la revendication 1 ou utilisation d'un polypeptide SnRK2 tel que défini selon la revendication 1 pour l'accroissement du rendement chez des plantes cultivées dans des conditions non stressantes, en particulier pour l'amélioration du rendement, notamment le rendement en biomasse et/ou en graines.

9. Utilisation selon la revendication 8, dans laquelle ledit rendement accru en graines comprend au moins le poids de mille grains accru.

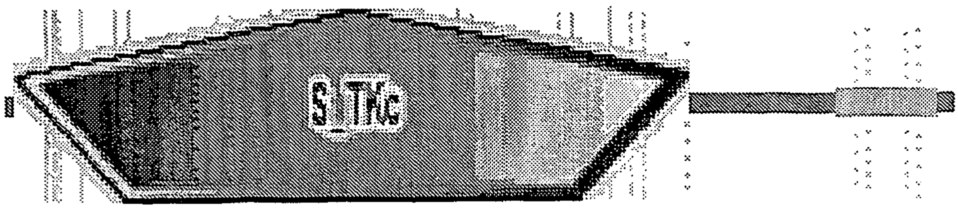

Figure 1

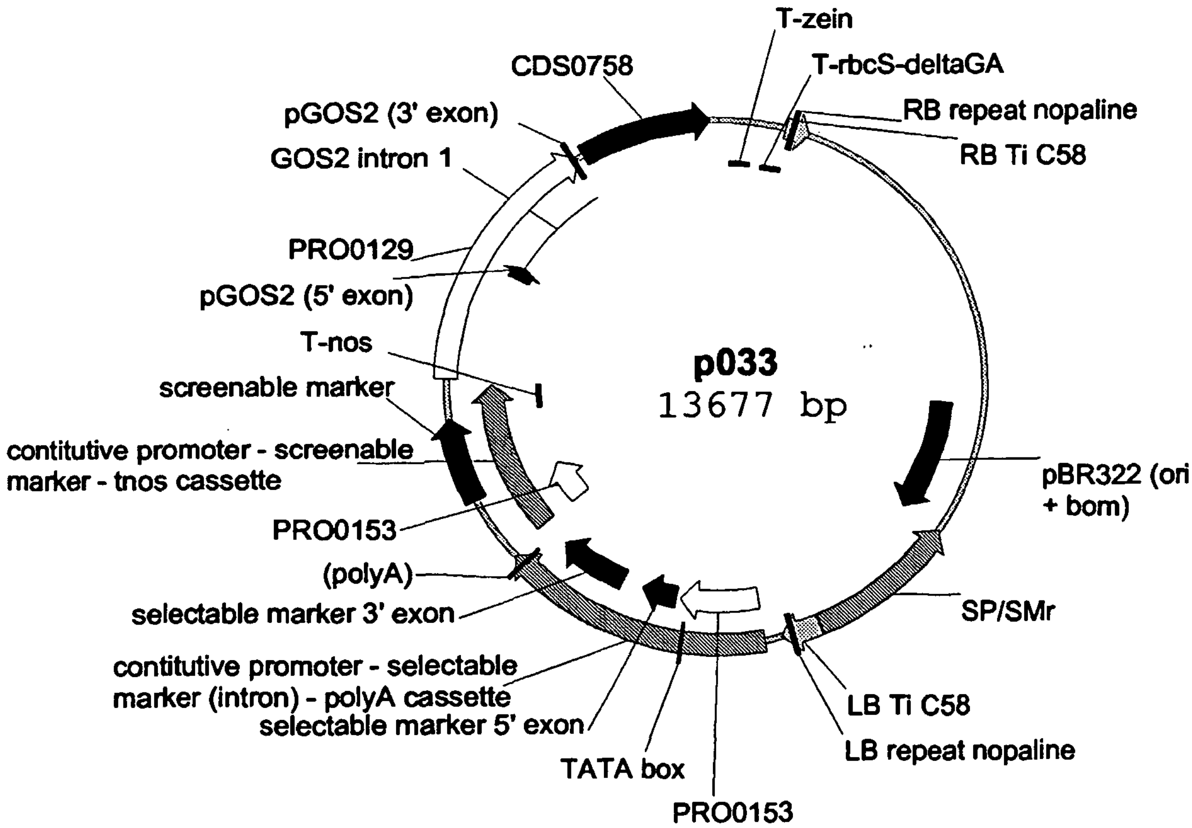


Figure 2

**SEQ ID NO 1: *SnRK2* DNA sequence**

ATGGACAAGTACGAGCTGGTGAAAGACATAGGTGCTGGGAATTTTGGAGTTGCCAGGCTCA
TGAAGGTCAAAAACTCTAAGGAACTTGTTGCCATGAAGTACATCGAGCGTGGTCCTAAGAT
TGATGAGAATGTGGCAAGAGAGATCATTAATCACAGATCACTTCGCCATCCGAATATAATC
CGGTTCAAGGAGGTGGTGTTGACTCCAACCCATCTTGCCATTGCCATGGAATATGCTGCTG
GTGGTGAACTATTCGAGCGTATATGCAGTGCTGGAAGATTTAGTGAGGATGAGGCGAGATA
TTTCTTCCAGCAGCTTATATCAGGTGTTAGCTATTGCCATGCTATGCAAATATGCCATAGA
GATCTGAAGCTCGAGAATACGCTCTTGGATGGAAGTCCTGCTCCACGTCTGAAAATCTGTG
ATTTTGGTTATTCCAAGTCCTCTCTGCTGCACTCTAGGCCCAAATCAACAGTTGGAACTCC
AGCATATATTGCACCTGAGGTCCTTTCTCGAAGAGAATATGATGGCAAGATGGCTGATGTA
TGGTCTTGTGGTGTGACTCTTTATGTCATGCTGGTTGGAGCATACCCATTTGAAGACCAGG
AAGACCCCAAGAACTTCAGGAAAACAATACAAAAAATAATGGCTGTCCAGTACAAGATCCC
GGACTACGTCCATATCTCACAGGATTGTAAAAATCTCCTTTCCCGTATATTTGTCGCCAAT
TCACTCAAGAGGATCACCATTGCAGAAATCAAGAAACATTCATGGTTCCTAAAGAATTTGC
CAAGGGAACTCACAGAGACAGCTCAAGCTGCATATTTCAAGAAAGAGAACCCAACCTTCTC
CCTTCAGACCGTTGAAGAGATCATGAAGATAGTGGCTGACGCCAAAACACCGCCTCCTGTT
TCCCGATCCATCGGAGGTTTTGGCTGGGGAGGAAATGGGGATGCAGATGGAAAAGAGGAAG
ATGCAGAAGACGTGGAGGAGGAAGAGGAGGAGGTGGAAGAAGAGGAAGACGATGAGGATGA
ATACGATAAGACTGTAAAGGAAGTACACGCAAGTGGAGAAGTGAGAATAAGTTGA

**SEQ ID NO 2: SnRK2 deduced protein sequence**

MDKYELVKDIGAGNFGVARLMKVKNSKELVAMKYIERGPKIDENVAREIINHRSLRHPNII
RFKEVVLTPTHLAIAMEYAAGGELFERICSAGRFSEDEARYFFQQLISGVSYCHAMQICHR
DLKLENTLLDGSPAPRLKICDFGYSKSSLLHSRPKSTVGTPAYIAPEVLSRREYDGKMADV
WSCGVTLYVMLVGAYPFEDQEDPKNFRKTIQKIMAVQYKIPDYVHISQDCKNLLSRIFVAN
SLKRITIAEIKKHSWFLKNLPRELTETAQAAYFKKENPTFSLQTVEEIMKIVADAKTPPPV
SRSIGGFGWGGNGDADGKEEDAEDVEEEEEEVEEEEDDEDEYDKTVKEVHASGEVRIS

**SEQ ID NO 3: *SnRK2* unspliced sequence**

caaacatgtatggatcgcatggaaacttgtgggtccctctttcttttaaaaaatctcgtat
taattaaataaatggaaaaaaaaacatgatgggagtttgtttaggcaggggagattcttct
tcattctcatcattatttctctattaatttcaccccaaaaaagaaaaaagaaaaattccaa
caagaaaaaaaaaagaaaaagaaagttgattcttcgcttaggcttgaaatctctccaatcc
aaatctcaaattaaccttccatcgtcatctctttccctttttttttcccactttctttgcg
aatcgcgagatctcggaatcgcatccttgatttgggatactgttttttttttttttaatc
ttgtttcattttcacgtgaaattcttagctgctagaactggacttgaatttcaacgagaat
tttggagatttttttttgtttgggtttttcctttctgttttgtgtgtttggaattagggt
tgtcgagcgagaATGGACAAGTACGAGCTGGTGAAAGACATAGGTGCTGGGAATTTTGGAG
TTGCCAGGCTCATGAAGGTCAAAAACTCTAAGGAACTTGTTGCCATGAAGTACATCGAGCG
TGGTCCTAAGgtatattcctctctgttttgtgttttcattgctctccatgagctggtgat
cctatacccagatatgcataattggaatgaattgctattaagcagaagagtcgatttttt
ttgtaaatttcttatcgttagctgattgggtgtttaacgtaacgttagttatcttgtagtt
gtaatattttcctgaaaagatttgtacaatgagtatttgctttgtttgtttttttgata
cagATTGATGAGAATGTGGCAAGAGAGATCATTAATCACAGATCACTTCGCCATCCGAATA
TAATCCGGTTCAAGGAGgttagtgaatttcttgttgcttgacatgggtggtgttcttgcta
tgaaaaagtttgttgataatctcttatatctttcatcttgcattccttgttgggtttatgg

## FIGURE 3

atttatagGTGGTGTTGACTCCAACCCATCTTGCCATTGCCATGGAATATGCTGCTGGTG
GTGAACTATTCGAGCGTATATGCAGTGCTGGAAGATTTAGTGAGGATGAGgtgagcttgcc
atttgaaaaattgtgctgtgcttttgcgaatatgaaattactagtatttaggataatctgc
atggtctttggaaagattaggaggaagggaacaagagaaaacatgtgaacctccttttatt
tagtatcaggcattaaacagttagggtctacgttctaatcctttctctcttttccagGCGA
GATATTTCTTCCAGCAGCTTATATCAGGTGTTAGCTATTGCCATGCTATGgtaatgtagag
acaatgacttaagcaaaatttacttatccattggctgtttgaagtcgtttttttttaatca
tgtgttgactattttgttgcagCAAATATGCCATAGAGATCTGAAGCTCGAGAATACGCTC
TTGGATGGAAGTCCTGCTCCACGTCTGAAAATCTGTGATTTTGGTTATTCCAAGgtctgac
actaaaaaaaaatccaagttccccccttgtcgacgagatcctcttttgtgatttgttattc
tctttttttagTCCTCTCTGCTGCACTCTAGGCCCAAATCAACAGTTGGAACTCCAGCAT
ATATTGCACCTGAGGTCCTTTCTCGAAGAGAATATGATGGCAAGgtaatcaagcatcatgc
acaatgcaatgaacttccataaacccatgagtatttatgatattgtcatgctctttacatt
tttacttttgaatttaaaaagtcatctttgtggaagtcgctaagatttgaagcatttttc
ttctttcagATGGCTGATGTATGGTCTTGTGGTGTGACTCTTTATGTCATGCTGGTTGGAG
CATACCCATTTGAAGACCAGGAAGACCCCAAGAACTTCAGGAAAACAATACAAgtaggttt
ctttttgaagccatgtatctgcatatctcgctttcgccacatcctattcgtcaatgtgtg
atcttgttatacagAAAATAATGGCTGTCCAGTACAAGATCCCGGACTACGTCCATATCTC
ACAGGATTGTAAAAATCTCCTTTCCCGTATATTTGTCGCCAATTCACTCAAGgtatacatc
aatcaactgaactaaatgttttcaaagatgccttttgatttttctgaacaattgagctact
tgttgtttcgtagAGGATCACCATTGCAGAAATCAAGAAACATTCATGGTTCCTAAAGAAT
TTGCCAAGGGAACTCACAGAGACAGCTCAAGCTGCATATTTCAAGAAAGAGAACCCAACCT
TCTCCCTTCAGACCGTTGAAGAGATCATGAAGATAGTGGCTGACGCCAAAACACCGCCTCC
TGTTTCCCGATCCATCGGAGGTTTTGGCTGGGGAGGAAATGGGGATGCAGATGGAAAAGAG
GAAGATGCAGAAGACGTGGAGGAGGAAGAGGAGGAGGTGGAAGAAGAGGAAGACGATGAGG
ATGAATACGATAAGACTGTAAAGGAAGTACACGCAAGTGGAGAAGTGAGAATAAGTTGAta
ttttggtttttggtctgtgtaagaaagaagtcgtcgttggtttgttgaaactgaaaagtct
ctgttctcgtgtttgcctttacaatgctttggctaaggttttggttctggttttggagatt
tgtaaaatttgcagtataagatgaacaaacagagaggttgatgatgagaatgagtcctttg
ctacgcatggtactatgaacattgtgacctccaataaatatttttgtaaattagattttat
tttccgaaaagattcatgtatttgatttttggatttcttattttattttttttcgttcct
tatcatttttttgaaaatgcaaatctataaaatacaaatgtcaacaaaaaatcaaattgaa
atgttcggaattcaaaaataattgttttctttttgtttttttgtttctgatgcgaaatgtga
atatattagagggaaaatatcccgccattaggaaaccggataatcttctacggccttgagc
tcaagtcggt

**SEQ ID NO 4: forward primer, start codon in bold**
ggggacaagtttgtacaaaaaagcaggcttcaca**atg**gacaagtacgagctggt

**SEQ ID NO 5: reverse primer**
ggggaccactttgtacaagaaagctgggtcgacgacttctttcttacacag

**SEQ ID NO 6: conserved signature sequence**
W(F/Y)(L/M/R)K(N/G/R)(L/P/I)(P/L)(A/G/V/R/K/I)(D/E/V)

## FIGURE 3 (continued)

**SEQ ID NO 7: At1g60940 mRNA, complete cds**

ATGGACAAGTACGAGCTTGTTAAAGACATCGGTGCTGGGAATTTTGGAGTGGCGAGGCTCA
TGAGAGTCAAAAACTCCAAGGAACTCGTTGCTATGAAGTACATCGAGCGTGGACCTAAGAT
TGATGAGAACGTGGCGAGAGAGATTATTAACCACAGATCACTTCGTCATCCCAATATTATC
CGGTTTAAGGAGGTGGTTTTGACACCAACGCACATCGCCATTGCTATGGAATATGCTGCTG
GCGGTGAGCTATTTGAGCGTATATGTAGCGCTGGAAGATTCAGTGAGGATGAGGCAAGATA
CTTTTTCCAGCAGCTTATCTCAGGAGTCAGCTATTGTCATGCTATGCAAATATGCCACAGA
GATCTGAAGCTTGAAAATACCCTCTTAGATGGAAGTCCTGCTCCACGCCTGAAGATCTGTG
ATTTTGGTTATTCCAAGTCCTCACTGTTGCACTCTATGCCCAAATCAACTGTTGGAACTCC
AGCATATATTGCACCTGAGGTTCTTTCTCGCGGAGAGTATGATGGCAAGATGGCTGATGTA
TGGTCTTGTGGTGTGACTCTTTATGTCATGCTGGTGGGAGCATACCCATTTGAAGACCAAG
AGGATCCCAAAAACTTCAAAAAAACAATACAAAGAATAATGGCTGTCAAGTACAAGATCCC
GGACTATGTCCATATCTCACAAGATTGCAAACATCTCCTCTCCCGTATATTTGTCACCAAC
TCGAATAAGAGGATTACGATAGGTGACATCAAGAAACATCCATGGTTCCTAAAGAACCTGC
CAAGGGAACTTACAGAAATAGCTCAAGCTGCATACTTCAGGAAAGAGAACCCGACATTCTC
ACTCCAAAGCGTCGAAGAGATAATGAAGATTGTGGAAGAGGCAAAAACTCCAGCTCGTGTT
TCTCGGTCGATTGGAGCATTTGGGTGGGGAGGAGGAGAAGATGCCGAGGGCAAGGAGGAAG
ATGCAGAGGAAGAAGTTGAGGAAGTAGAAGAAGAAGAAGACGAAGAAGATGAGTATGATAA
GACGGTGAAGCAAGTGCATGCTAGCATGGGAGAAGTCCGAGTCAGTTAA

**SEQ ID NO 8: At1g60940, deduced protein sequence, NP_849834**

MDKYELVKDIGAGNFGVARLMRVKNSKELVAMKYIERGPKIDENVAREIINHRSLRHPNII
RFKEVVLTPTHIAIAMEYAAGGELFERICSAGRFSEDEARYFFQQLISGVSYCHAMQICHR
DLKLENTLLDGSPAPRLKICDFGYSKSSLLHSMPKSTVGTPAYIAPEVLSRGEYDGKMADV
WSCGVTLYVMLVGAYPFEDQEDPKNFKKTIQRIMAVKYKIPDYVHISQDCKHLLSRIFVTN
SNKRITIGDIKKHPWFLKNLPRELTEIAQAAYFRKENPTFSLQSVEEIMKIVEEAKTPARV
SRSIGAFGWGGGEDAEGKEEDAEEEVEEVEEEEDEEDEYDKTVKQVHASMGEVRVS

**SEQ ID NO 9: At5g63650 mRNA, complete cds**

ATGGACAAGTATGAGGTTGTGAAGGATTTGGGAGCTGGAAATTTTGGTGTGGCTCGTCTTC
TTAGACACAAAGAGACCAAAGAGCTCGTTGCTATGAAATACATTGAGAGAGGTCGCAAGAT
TGATGAGAATGTGGCAAGAGAGATTATCAATCATAGATCACTTAGGCATCCTAATATCATC
AGATTCAAGGAGGTGATTCTGACTCCAACTCATCTTGCAATTGTAATGGAGTATGCTTCTG
GAGGAGAGCTCTTTGAAAGAATCTGTAATGCTGGTAGATTCAGTGAAGCTGAGGCTAGATA
CTTCTTTCAGCAGCTGATTTGTGGCGTGGATTACTGTCATTCACTGCAAATATGTCATAGA
GATTTGAAGCTTGAGAATACACTGCTTGATGGTAGTCCAGCCCCGCTTTTGAAAATCTGTG
ATTTTGGTTACTCCAAGTCATCTCTGCTTCACTCTAGACCTAAATCAACTGTTGGTACTCC
AGCTTATATCGCACCTGAAGTTCTTTCCCGAAGAGAATATGACGGAAAGCATGCGGATGTT
TGGTCCTGTGGTGTGACTCTTTATGTGATGTTAGTTGGAGGTTATCCGTTTGAAGACCCGG
ATGATCCGAGAAACTTCAGGAAAACAATCCAACGTATAATGGCTGTCCAGTACAAGATCCC
GGATTACGTTCATATATCGCAGGAGTGCAGACACCTTCTCTCTCGCATATTTGTCACTAAT
TCAGCTAAGAGAATCACACTTAAAGAGATCAAGAAGCATCCATGGTACTTAAAGAACTTGC
CAAAGGAGCTTACAGAGCCTGCTCAAGCGGCGTACTACAAGAGAGAAACCCCAAGCTTTTC
CCTCCAAAGCGTAGAGGACATAATGAAGATCGTTGGAGAAGCCAGGAATCCAGCTCCGTCT
TCTAATGCCGTCAAGGGCTTTGATGATGATGAGGAAGATGTGGAGGACGAGGTTGAAGAAG
AAGAAGAAGAAGAAGAAGAAGAGGAGGAAGAAGAGGAAGAGGAAGAAGATGAATACGAGAA
GCATGTTAAAGAGGCCCATTCTTGTCAAGAGCCTCCCAAAGCTTAA

**FIGURE 3 (continued)**

**SEQ ID NO 10: At5g63650, deduced protein sequence**
MDKYEVVKDLGAGNFGVARLLRHKETKELVAMKYIERGRKIDENVAREIINHRSLRHPNII
RFKEVILTPTHLAIVMEYASGGELFERICNAGRFSEAEARYFFQQLICGVDYCHSLQICHR
DLKLENTLLDGSPAPLLKICDFGYSKSSLLHSRPKSTVGTPAYIAPEVLSRREYDGKHADV
WSCGVTLYVMLVGGYPFEDPDDPRNFRKTIQRIMAVQYKIPDYVHISQECRHLLSRIFVTN
SAKRITLKEIKKHPWYLKNLPKELTEPAQAAYYKRETPSFSLQSVEDIMKIVGEARNPAPS
SNAVKGFDDDEEDVEDEVEEEEEEEEEEEEEEEEEEEDEYEKHVKEAHSCQEPPKA

**SEQ ID NO 11: At5g08590 mRNA, complete cds**
ATGGACAAGTATGACGTTGTCAAGGATCTGGGAGCTGGAAATTTCGGTGTGGCTCGCCTTC
TCAGGCACAAGGACACCAAAGAGCTTGTTGCCATGAAATACATCGAGAGAGGTCGCAAGAT
AGATGAGAACGTGGCGAGAGAGATTATTAATCACAGATCACTTAAACATCCTAATATCATC
CGGTTCAAGGAGGTGATCCTGACACCTACTCATCTTGCTATTGTGATGGAGTATGCTTCTG
GAGGAGAGCTCTTTGATCGAATCTGTACTGCCGGTAGATTTAGTGAAGCTGAGGCTAGGTA
CTTCTTTCAACAGCTGATTTGTGGTGTTGATTACTGCCATTCCTTGCAAATATGTCATAGA
GACCTGAAGCTTGAGAACACACTGCTCGATGGGAGCCCTGCTCCGCTTTTGAAAATCTGTG
ATTTTGGTTACTCTAAGTCATCTATACTACATTCTAGGCCTAAATCAACTGTTGGAACTCC
AGCTTACATAGCACCTGAAGTTCTTTCACGGAGAGAATATGATGGCAAGCACGCGGATGTG
TGGTCATGTGGAGTAACCCTTTATGTGATGCTGGTGGGAGCTTACCCGTTTGAGGACCCTA
ATGATCCAAAAAACTTCAGGAAAACAATCCAGCGCATAATGGCTGTACAATACAAGATCCC
GGACTATGTTCACATATCTCAGGAATGCAAACATCTTCTCTCTCGCATATTCGTCACTAAC
TCTGCTAAGAGAATCACACTTAAGGAGATCAAGAATCATCCGTGGTACTTGAAGAATTTGC
CAAAGGAGCTGCTAGAGTCGGCTCAAGCGGCGTATTACAAGAGAGACACAAGCTTCTCTCT
TCAAAGCGTAGAGGACATAATGAAGATAGTTGGAGAAGCAAGGAATCCAGCTCCATCAACT
AGTGCTGTCAAAAGCTCGGGCTCAGGAGCTGATGAAGAAGAGGAAGAGGACGTTGAAGCTG
AAGTGGAAGAGGAAGAAGATGATGAAGACGAATACGAGAAGCATGTCAAAGAGGCACAGTC
TTGTCAAGAGTCTGACAAAGCTTAA

**SEQ ID NO 12: At5g08590, deduced protein sequence**
MDKYDVVKDLGAGNFGVARLLRHKDTKELVAMKYIERGRKIDENVAREIINHRSLKHPNII
RFKEVILTPTHLAIVMEYASGGELFDRICTAGRFSEAEARYFFQQLICGVDYCHSLQICHR
DLKLENTLLDGSPAPLLKICDFGYSKSSILHSRPKSTVGTPAYIAPEVLSRREYDGKHADV
WSCGVTLYVMLVGAYPFEDPNDPKNFRKTIQRIMAVQYKIPDYVHISQECKHLLSRIFVTN
SAKRITLKEIKNHPWYLKNLPKELLESAQAAYYKRDTSFSLQSVEDIMKIVGEARNPAPST
SAVKSSGSGADEEEEEDVEAEVEEEEDDEDEYEKHVKEAQSCQESDKA

**SEQ ID NO 13: At4g33950 mRNA, complete cds**
ATGGATCGACCAGCAGTGAGTGGTCCAATGGATTTGCCGATTATGCACGATAGTGATAGGT
ATGAACTCGTCAAGGATATTGGCTCCGGTAATTTTGGAGTTGCGAGATTGATGAGAGACAA
GCAAAGTAATGAGCTTGTTGCTGTTAAATATATCGAGAGAGGTGAGAAGATAGATGAAAAT
GTAAAAAGGGAGATAATCAACCACAGGTCCTTAAGACATCCCAATATCGTTAGATTCAAAG
AGGTTATATTAACACCAACCCATTTAGCCATTGTTATGGAATATGCATCTGGAGGAGAACT
TTTCGAGCGAATCTGCAATGCAGGCCGCTTCAGCGAAGACGAGGCGAGGTTTTTCTTCCAG
CAACTCATTTCAGGAGTTAGTTACTGTCATGCTATGCAAGTATGTCACCGAGACTTAAAGC

**FIGURE 3 (continued)**

TCGAGAATACGTTATTAGATGGTAGCCCGGCCCCTCGTCTAAAGATATGTGATTTCGGATA
TTCTAAGTCATCAGTGTTACATTCGCAACCAAAATCAACTGTTGGAACTCCTGCTTACATC
GCTCCTGAGGTTTACTAAAGAAAGAATATGATGGAAAGGTTGCAGATGTTTGGTCTTGTG
GGGTTACTCTGTATGTCATGCTGGTTGGAGCATATCCTTTCGAAGATCCCGAGGAACCAAA
GAATTTCAGGAAAACTATACATAGAATCCTGAATGTTCAGTATGCTATTCCGGATTATGTT
CACATATCTCCTGAATGTCGCCATTTGATCTCCAGAATATTTGTTGCTGACCCTGCAAAGA
GGATATCAATTCCTGAAATAAGGAACCATGAATGGTTTCTAAAGAATCTACCGGCAGATCT
AATGAACGATAACACGATGACCACTCAGTTTGATGAATCGGATCAACCGGGCCAAAGCATA
GAAGAAATTATGCAGATCATTGCAGAAGCAACTGTTCCTCCTGCAGGCACTCAGAATCTGA
ACCATTACCTCACAGGAAGCTTGGACATAGATGACGATATGGAGGAAGACTTAGAGAGCGA
CCTTGATGATCTTGACATCGACAGTAGCGGAGAGATTGTGTACGCAATGTGA

**SEQ ID NO 14: At4g33950, deduced protein sequence**
MDRPAVSGPMDLPIMHDSDRYELVKDIGSGNFGVARLMRDKQSNELVAVKYIERGEKIDEN
VKREIINHRSLRHPNIVRFKEVILTPTHLAIVMEYASGGELFERICNAGRFSEDEARFFFQ
QLISGVSYCHAMQVCHRDLKLENTLLDGSPAPRLKICDFGYSKSSVLHSQPKSTVGTPAYI
APEVLLKKEYDGKVADVWSCGVTLYVMLVGAYPFEDPEEPKNFRKTIHRILNVQYAIPDYV
HISPECRHLISRIFVADPAKRISIPEIRNHEWFLKNLPADLMNDNTMTTQFDESDQPGQSI
EEIMQIIAEATVPPAGTQNLNHYLTGSLDIDDDMEEDLESDLDDLDIDSSGEIVYAM

**SEQ ID NO 15: At2g23030 mRNA, complete cds**
ATGGAGAAGTATGAGATGGTGAAGGATTTAGGATTTGGTAATTTCGGATTGGCTCGGCTTA
TGCGTAATAAGCAAACAAACGAGCTTGTGGCTGTCAAATTCATCGATCGAGGCTACAAGAT
AGATGAGAACGTTGCAAGAGAAATAATCAATCATAGAGCTCTCAACCATCCGAATATTGTT
CGGTTTAAAGAGGTTGTTTTAACTCCGACACATCTTGGAATAGTAATGGAGTATGCAGCTG
GAGGAGAACTGTTCGAGCGGATATCTAGCGTGGGTCGATTTAGCGAAGCTGAGGCAAGATA
TTTCTTTCAACAACTCATTTGTGGAGTCCATTACTTACATGCATTGCAAATATGCCATAGA
GATCTGAAATTAGAAAACACATTGCTTGATGGAAGCCCAGCACCACGTTTAAAAATTTGTG
ATTTTGGCTACTCAAAGTCTTCTGTTCTGCACTCCAACCCAAAATCAACGGTGGGAACTCC
GGCATATATAGCACCGGAAGTTTTTTGTCGATCGGAATACGACGGAAAGTCAGTTGATGTG
TGGTCTTGTGGAGTGGCCCTCTATGTTATGTTGGTAGGAGCTTATCCATTCGAAGACCCTA
AAGACCCTCGCAATTTCCGAAAAACTGTTCAGAAAATAATGGCCGTAAACTACAAGATTCC
AGGATATGTTCACATATCCGAAGACTGCAGAAAGTTACTATCTCGTATATTTGTTGCCAAT
CCGTTACATAGAAGTACGCTTAAAGAGATTAAGAGTCATGCATGGTTCCTAAAGAATTTGC
CAAGAGAATTAAAGGAGCCAGCACAAGCAATCTATTACCAAAGGAATGTTAATCTTATTAA
TTTTTCTCCTCAAAGAGTAGAGGAGATTATGAAGATAGTTGGTGAGGCAAGAACCATTCCA
AACCTTTCTCGCCCGGTCGAATCGCTTGGATCAGATAAAAAAGATGATGATGAAGAAGAAT
ATTTGGATGCTAATGATGAAGAATGGTATGATGATTACGCATAG

**SEQ ID NO 16: At2g23030, deduced protein sequence**
MEKYEMVKDLGFGNFGLARLMRNKQTNELVAVKFIDRGYKIDENVAREIINHRALNHPNIV
RFKEVVLTPTHLGIVMEYAAGGELFERISSVGRFSEAEARYFFQQLICGVHYLHALQICHR
DLKLENTLLDGSPAPRLKICDFGYSKSSVLHSNPKSTVGTPAYIAPEVFCRSEYDGKSVDV
WSCGVALYVMLVGAYPFEDPKDPRNFRKTVQKIMAVNYKIPGYVHISEDCRKLLSRIFVAN
PLHRSTLKEIKSHAWFLKNLPRELKEPAQAIYYQRNVNLINFSPQRVEEIMKIVGEARTIP
NLSRPVESLGSDKKDDDEEYLDANDEEWYDDYA

**FIGURE 3 (continued)**

**SEQ ID NO 17: At5g66880 mRNA, complete cds**
ATGGATCGAGCTCCGGTGACCACAGGACCGTTGGATATGCCGATTATGCACGACAGTGATC
GATATGACTTCGTTAAGGATATTGGTTCTGGTAATTTCGGTGTTGCTCGTCTTATGAGAGA
TAAACTCACTAAAGAGCTTGTTGCTGTCAAGTACATCGAGAGAGGAGACAAGATTGATGAA
AATGTTCAAAGGGAGATCATTAACCACAGGTCACTAAGGCATCCTAATATTGTCAGATTTA
AAGAGGTCATTTTGACGCCGACTCATCTGGCTATCATAATGGAATATGCTTCTGGCGGTGA
ACTTTACGAGCGGATTTGCAATGCAGGACGGTTTAGTGAAGATGAGGCTCGGTTCTTCTTT
CAGCAGCTTCTATCTGGAGTCAGTTATTGTCATTCGATGCAAATTTGCCATCGTGACCTGA
AGCTAGAGAATACATTGTTGGATGGAAGTCCTGCTCCTCGATTAAAAATTTGTGATTTTGG
ATATTCAAAGTCTTCTGTTCTTCATTCACAACCAAAGTCAACTGTTGGTACTCCTGCATAC
ATCGCTCCAGAGGTACTGCTTCGTCAGGAATATGATGGCAAGATTGCAGATGTATGGTCAT
GTGGTGTGACCTTATACGTCATGTTGGTTGGAGCGTATCCGTTCGAAGATCCAGAAGAGCC
AAGAGACTATCGGAAAACAATACAGAGAATCCTTAGCGTTAAATACTCAATCCCTGATGAC
ATACGGATATCACCTGAATGCTGTCATCTTATTTCAAGAATCTTCGTGGCTGATCCCGCTA
CCAGAATAAGCATACCAGAGATCAAAACCCATAGTTGGTTCTTGAAGAATCTCCCTGCTGA
TCTAATGAACGAGAGCAACACAGGAAGCCAGTTCCAGGAGCCTGAACAACCAATGCAAAGC
CTTGACACAATCATGCAAATCATCTCTGAAGCCACAATTCCCGCTGTTCGAAACCGTTGCC
TAGACGATTTCATGACTGACAATCTTGATCTTGACGATGACATGGATGACTTTGACTCTGA
ATCTGAAATCGACATTGACAGTAGCGGAGAGATAGTTTACGCTCTCTAA

**SEQ ID NO 18: At5g66880, deduced protein sequence**
MDRAPVTTGPLDMPIMHDSDRYDFVKDIGSGNFGVARLMRDKLTKELVAVKYIERGDKIDE
NVQREIINHRSLRHPNIVRFKEVILTPTHLAIIMEYASGGELYERICNAGRFSEDEARFFF
QQLLSGVSYCHSMQICHRDLKLENTLLDGSPAPRLKICDFGYSKSSVLHSQPKSTVGTPAY
IAPEVLLRQEYDGKIADVWSCGVTLYVMLVGAYPFEDPEEPRDYRKTIQRILSVKYSIPDD
IRISPECCHLISRIFVADPATRISIPEIKTHSWFLKNLPADLMNESNTGSQFQEPEQPMQS
LDTIMQIISEATIPAVRNRCLDDFMTDNLDLDDDMDDFDSESEIDIDSSGEIVYAL

**SEQ ID NO 19: At1g78290 mRNA, complete cds**
ATGGAGAGGTACGAAATAGTGAAGGATATTGGGTCTGGTAACTTCGGAGTAGCAAAGCTTG
TTCGTGACAAATTTTCCAAAGAGCTTTTCGCTGTTAAGTTCATCGAGCGAGGCCAAAAGAT
TGATGAACATGTACAAAGAGAAATCATGAACCATAGGTCGCTGATCCATCCCAATATAATA
AGATTCAAGGAGGTTTTATTGACGGCAACACATTTGGCGTTAGTAATGGAATACGCCGCCG
GAGGAGAACTGTTCGGAAGAATCTGCAGCGCCGGAAGATTCAGTGAAGACGAGGCAAGGTT
TTTCTTTCAGCAGCTTATATCAGGAGTTAATTACTGTCACAGTCTTCAAATATGCCATAGA
GATTTAAAGCTAGAGAACACGTTACTTGATGGAAGCGAAGCGCCACGTGTAAAGATTTGCG
ACTTTGGATATTCAAAATCAGGAGTTCTTCATTCGCAACCAAAGACAACAGTAGGAACACC
TGCTTACATTGCACCTGAAGTGCTCTCCACGAAAGAGTATGACGGCAAAATCGCTGATGTT
TGGTCTTGTGGAGTCACTTTGTATGTTATGCTTGTTGGTGCTTATCCTTTTGAAGATCCTT
CTGATCCTAAAGATTTTCGGAAGACGATCGGTCGGATTCTCAAAGCTCAGTATGCTATTCC
TGATTATGTTCGAGTTTCGGATGAATGCAGACATCTTCTCTCTCGGATATTCGTTGCCAAC
CCTGAAAAGAGAATAACAATAGAGGAGATAAAGAATCATTCTTGGTTTCTCAAGAACTTGC
CGGTAGAGATGTATGAAGGATCATTGATGATGAATGGTCCATCGACTCAGACAGTAGAAGA
GATAGTGTGGATCATTGAAGAAGCTCGGAAACCTATCACCGTAGCTACTGGACTCGCAGGT
GCTGGTGGCTCTGGTGGAAGCAGTAATGGTGCCATTGGAAGTAGCAGTATGGATCTCGATG
ACTTGGACACAGATTTCGACGACATCGATACCGCTGATCTCCTTTCCCCTTTGTGA

**FIGURE 3 (continued)**

**SEQ ID NO 20: At1g78290, deduced protein sequence**
MERYEIVKDIGSGNFGVAKLVRDKFSKELFAVKFIERGQKIDEHVQREIMNHRSLIHPNII
RFKEVLLTATHLALVMEYAAGGELFGRICSAGRFSEDEARFFFQQLISGVNYCHSLQICHR
DLKLENTLLDGSEAPRVKICDFGYSKSGVLHSQPKTTVGTPAYIAPEVLSTKEYDGKIADV
WSCGVTLYVMLVGAYPFEDPSDPKDFRKTIGRILKAQYAIPDYVRVSDECRHLLSRIFVAN
PEKRITIEEIKNHSWFLKNLPVEMYEGSLMMNGPSTQTVEEIVWIIEEARKPITVATGLAG
AGGSGGSSNGAIGSSSMDLDDLDTDFDDIDTADLLSPL

**SEQ ID NO 21: At3g50500 mRNA, complete cds**
ATGGATCCGGCGACTAATTCACCGATTATGCCGATTGATTTACCGATTATGCACGACAGTG
ATCGTTACGACTTCGTTAAAGATATTGGCTCTGGTAATTTCGGCGTTGCTCGTCTCATGAC
CGATAGAGTCACCAAGGAGCTTGTTGCTGTTAAATACATCGAGAGAGGAGAAAAGATTGAT
GAAAATGTTCAGAGGGAGATTATCAATCATAGATCATTGAGACATCCTAATATTGTTAGGT
TTAAAGAGGTGATTTTGACGCCTTCCCATTTGGCTATTGTTATGGAATATGCTGCTGGTGG
AGAACTTTATGAGCGGATTTGTAATGCCGGACGGTTTAGTGAAGATGAGGCTCGGTTCTTC
TTTCAGCAGCTTATATCTGGAGTTAGCTATTGTCATGCAATGCAAATATGCCATCGGGATC
TGAAGCTGGAAAATACATTGTTAGATGGAAGTCCGGCACCTCGTTTGAAAATATGTGATTT
TGGTTATTCCAAGTCTTCTGTTCTTCATTCCCAACCAAAGTCAACTGTTGGTACTCCTGCA
TACATTGCACCAGAGATTCTTCTTCGACAGGAATATGATGGCAAGCTTGCAGATGTATGGT
CTTGCGGTGTAACATTATATGTAATGTTGGTTGGAGCTTATCCATTCGAGGATCCACAGGA
GCCACGAGATTATCGAAAGACAATACAAAGAATCCTTAGTGTCACATACTCGATCCCAGAG
GACTTACACCTCTCACCAGAATGTCGCCATCTAATATCGAGGATCTTCGTGGCTGATCCGG
CAACAAGAATCACTATTCCGGAGATCACATCCGATAAATGGTTCTTGAAGAATCTACCAGG
TGATTTGATGGATGAGAACCGAATGGGAAGTCAGTTTCAAGAGCCTGAGCAGCCAATGCAG
AGCCTTGACACGATTATGCAGATAATATCGGAGGCTACGATTCCGACTGTTCGTAATCGTT
GCCTCGATGATTTCATGGCGGATAATCTTGATCTAGACGATGACATGGATGACTTTGATTC
CGAATCTGAGATTGATGTTGACAGTAGTGGAGAGATAGTTTATGCTCTCTGA

**SEQ ID NO 22: At3g50500, deduced protein sequence**
MDPATNSPIMPIDLPIMHDSDRYDFVKDIGSGNFGVARLMTDRVTKELVAVKYIERGEKID
ENVQREIINHRSLRHPNIVRFKEVILTPSHLAIVMEYAAGGELYERICNAGRFSEDEARFF
FQQLISGVSYCHAMQICHRDLKLENTLLDGSPAPRLKICDFGYSKSSVLHSQPKSTVGTPA
YIAPEILLRQEYDGKLADVWSCGVTLYVMLVGAYPFEDPQEPRDYRKTIQRILSVTYSIPE
DLHLSPECRHLISRIFVADPATRITIPEITSDKWFLKNLPGDLMDENRMGSQFQEPEQPMQ
SLDTIMQIISEATIPTVRNRCLDDFMADNLDLDDDMDDFDSESEIDVDSSGEIVYAL

**SEQ ID NO 23: At4g40010 mRNA, complete cds**
ATGGAGAGATACGACATCTTAAGAGATCTTGGTTCCGGTAACTTTGGAGTTGCTAAGCTTG
TCAGAGAAAAAGCCAACGGAGAGTTTTACGCCGTTAAATACATCGAAAGAGGCCTTAAGAT
TGATGAACATGTTCAGAGAGAGATCATAAACCACAGAGACTTGAAGCATCCTAATATCATC
AGATTTAAAGAGGTTTTTGTAACACCAACACATCTTGCCATAGTAATGGAGTATGCAGCTG
GTGGTGAACTTTTTGAAAGAATTTGCAATGCCGGTAGATTCAGCGAAGACGAAGGAAGATA
TTATTTCAAACAACTTATCTCGGGAGTTAGCTATTGTCACGCTATGCAAATATGTCACAGA
GACCTTAAGCTCGAGAATACACTCTTAGACGGGAGCCCGTCGTCGCATCTTAAAATATGTG
ATTTTGGATACTCCAAGTCATCAGTTTTACACTCTCAACCAAAATCCACCGTGGGAACTCC

**FIGURE 3 (continued)**

GGCTTACGTTGCTCCGGAAGTCTTGTCCCGGAAAGAATATAATGGAAAGATTGCAGATGTG
TGGTCGTGTGGGGTGACCTTATATGTAATGTTAGTTGGTGCTTATCCCTTTGAAGATCCCG
AAGATCCACGGAACATTAGAAACACCATTCAGAGGATATTAAGTGTACACTACACCATACC
GGATTACGTCAGGATTTCCTCCGAGTGCAAGCATCTCTTGTCTCGTATCTTTGTGGCTGAC
CCTGATAAGAGAATAACTGTACCGGAAATCGAAAAGCACCCGTGGTTCTTGAAGGGCCCTT
TGGTTGTGCCGCCGGAGGAAGAGAAATGCGATAATGGAGTTGAAGAAGAAGAAGAAGAAGA
AGAGAAGTGTCGACAGAGTGTTGAAGAGATAGTGAAGATAATAGAGGAAGCAAGAAAGGGA
GTAAATGGTACGGATAATAATGGTGGATTAGGGTTAATAGATGGGAGCATTGATCTTGATG
ATATTGATGATGCTGATATTTATGATGATGTTGATGATGATGAGGAGAGAAATGGTGATTT
CGTATGTGCTCTATGA

**SEQ ID NO 24: At4g40010, deduced protein sequence**
MERYDILRDLGSGNFGVAKLVREKANGEFYAVKYIERGLKIDEHVQREIINHRDLKHPNII
RFKEVFVTPTHLAIVMEYAAGGELFERICNAGRFSEDEGRYYFKQLISGVSYCHAMQICHR
DLKLENTLLDGSPSSHLKICDFGYSKSSVLHSQPKSTVGTPAYVAPEVLSRKEYNGKIADV
WSCGVTLYVMLVGAYPFEDPEDPRNIRNTIQRILSVHYTIPDYVRISSECKHLLSRIFVAD
PDKRITVPEIEKHPWFLKGPLVVPPEEEKCDNGVEEEEEEEEKCRQSVEEIVKIIEEARKG
VNGTDNNGGLGLIDGSIDLDDIDDADIYDDVDDDEERNGDFVCAL

**SEQ ID NO 25: *Oryza sativa* SAPK1 mRNA**
ATGGAGCGGTACGAGGTGATGAGGGACATCGGGTCCGGGAACTTCGGGGTGGCCAAGCTCG
TCCGCGACGTCGCCACCAACCACCTCTTCGCCGTCAAGTTCATCGAGAGGGGACTCAAGAT
TGATGAACATGTTCAAAGGGAGATTATGAACCACCGATCACTGAAGCATCCAAACATAATC
CGGTTCAAGGAGGTCGTGCTAACTCCCACACATTTGGCAATAGTTATGGAATATGCTGCTG
GTGGTGAGCTATTTGAAAGGATTTGCAACGCAGGGAGATTCAGTGAGGATGAGGCAAGGTT
CTTCTTCCAACAGCTGATTTCTGGAGTGAGCTATTGTCATTCTATGCAAGTATGCCATAGA
GATTTGAAACTCGAAAATACTCTCTTGGATGGCAGTGTCACACCTCGGCTTAAGATTTGTG
ATTTTGGTTACTCCAAGTCTTCTGTCCTGCACTCTCAACCGAAATCAACTGTTGGCACACC
GGCTTACATTGCTCCAGAGGTCCTCTCTAGAAAGGAATACGATGGAAAGGTAGCTGATGTT
TGGTCATGTGGGGTAACACTCTATGTGATGCTTGTTGGTGCGTATCCTTTTGAGGACCCTG
ATGACCCAAGGAACTTCCGCAAGACGATCACTAGGATACTCAGTGTACAGTATTCAATTCC
AGACTACGTTCGAGTTTCAGCGGACTGCAGACATCTCCTGTCCCGGATTTTCGTTGGAAAT
CCTGAGCAGAGGATAACTATCCCAGAGATCAAGAACCACCCATGGTTCCTGAAGAACCTGC
CCATCGAGATGACCGACGAGTACCAGAGGAGCATGCAGCTGGCGGACATGAACACGCCGTC
GCAGAGCCTGGAGGAGGTCATGGCGATCATTCAGGAGGCCCGGAAACCGGGCGACGCCATG
AAGCTCGCCGGCGCCGGGCAGGTCGCCTGCCTGGGGAGCATGGATCTCGACGACATCGACG
ATATCGACGACATTGACATCGAGAACAGCGGGGACTTCGTGTGCGCCTTGTGA

**SEQ ID NO 26: *Oryza sativa* SAPK1, deduced protein sequence, BAD17997**
MERYEVMRDIGSGNFGVAKLVRDVATNHLFAVKFIERGLKIDEHVQREIMNHRSLKHPNII
RFKEVVLTPTHLAIVMEYAAGGELFERICNAGRFSEDEARFFFQQLISGVSYCHSMQVCHR
DLKLENTLLDGSVTPRLKICDFGYSKSSVLHSQPKSTVGTPAYIAPEVLSRKEYDGKVADV
WSCGVTLYVMLVGAYPFEDPDDPRNFRKTITRILSVQYSIPDYVRVSADCRHLLSRIFVGN
PEQRITIPEIKNHPWFLKNLPIEMTDEYQRSMQLADMNTPSQSLEEVMAIIQEARKPGDAM
KLAGAGQVACLGSMDLDDIDDIDDIDIENSGDFVCAL

## FIGURE 3 (continued)

**SEQ ID NO 27: *Oryza sativa* SAPK2 mRNA,**
ATGGAGAGGTACGAGGTGATCAAGGACATAGGGTCGGGGAACTTCGGCGTGGCCAAGCTTG
TCCGGGATGTGCGGACCAAGGAGCTGTTTGCCGTCAAGTTCATCGAGAGGGGGCAGAAGAT
CGACGAGAATGTCCAAAGGGAGATTATGAACCACAGGTCACTGAGGCATCCGAACATTGTT
AGATTCAAGGAGGTTGTGCTAACTCCCACACATTTGGCCATAGTTATGGAATATGCTGCTG
GAGGTGAGCTATTCGAAAGGATTTGCAGTGCTGGGAGGTTTAGCGAGGATGAGGCAAGGTT
CTTCTTCCAGCAGTTGATTTCAGGAGTTAGCTACTGTCATTCCATGCAAATATGTCATAGA
GATTTGAAACTAGAAAATACTCTCTTGGATGGGAGCATAGCACCTCGGCTCAAGATATGTG
ATTTTGGTTACTCAAAGTCCTCTTTGTTGCACTCTCAACCGAAATCTACTGTCGGTACTCC
AGCTTATATCGCTCCTGAGGTCCTTGCTAGAAAAGAATATGATGGAAAGGTTGCTGACGTT
TGGTCATGTGGAGTAACTCTATATGTGATGCTTGTTGGTGCGTACCCCTTTGAGGACCCTG
ACGAACCAAGAAACTTCCGCAAGACAATTACTCGGATACTAAGCGTACAATACATGGTTCC
TGATTATGTTCGAGTTTCGATGGAATGCAGACATCTTCTGTCCCGGATTTTCGTGGCAAAC
CCAGAGCAACGAATTACCATTCCTGAGATCAAGAACCACCCATGGTTCCTCAAGAACCTGC
CGATCGAGATGACTGACGAGTACCAGATGAGCGTCCAGATGAACGACATCAACACCCCGTC
ACAGGGCCTGGAGGAGATCATGGCCATCATACAGGAGGCGCGGAAGCCGGGTGATGGCTCC
AAATTCTCCGGGCAGATCCCGGGCCTAGGGAGCATGGAGCTCGACGACGTTGACACCGACG
ACATCGACGTCGAGGACAGCGGCGACTTCGTGTGCGCATTGTGA

**SEQ ID NO 28: *Oryza sativa* SAPK2, deduced protein sequence, BAD17998**
MERYEVIKDIGSGNFGVAKLVRDVRTKELFAVKFIERGQKIDENVQREIMNHRSLRHPNIV
RFKEVVLTPTHLAIVMEYAAGGELFERICSAGRFSEDEARFFFQQLISGVSYCHSMQICHR
DLKLENTLLDGSIAPRLKICDFGYSKSSLLHSQPKSTVGTPAYIAPEVLARKEYDGKVADV
WSCGVTLYVMLVGAYPFEDPDEPRNFRKTITRILSVQYMVPDYVRVSMECRHLLSRIFVAN
PEQRITIPEIKNHPWFLKNLPIEMTDEYQMSVQMNDINTPSQGLEEIMAIIQEARKPGDGS
KFSGQIPGLGSMELDDVDTDDIDVEDSGDFVCAL

**SEQ ID NO 29: *Oryza sativa* SAPK3 mRNA**
ATGGAGGAGAGGTACGAGGCGTTGAAGGAGCTCGGGGCCGGCAACTTCGGGGTGGCCAGGC
TGGTCAGGGACAAGAGGAGCAAGGAGCTCGTCGCCGTCAAGTACATCGAGAGGGGCAAGAA
GATTGATGAAAATGTGCAGAGGGAGATCATCAATCATAGGTCGCTCCGGCATCCCAATATC
ATTCGGTTTAAGGAGGTTTGTTTGACACCCACACACCTAGCCATTGTCATGGAGTATGCTG
CTGGTGGAGAACTCTTTGAACAAATCTGCACCGCAGGGCGATTCAGCGAAGACGAGGCAAG
GTACTTCTTCCAGCAGCTAATATCAGGTGTCAGCTACTGTCATTCTCTGGAAATTTGCCAC
CGTGATCTTAAACTTGAGAACACCCTCCTGGATGGAAGCCCAACACCTCGTGTGAAGATTT
GTGACTTTGGTTACTCAAAGTCTGCTTTGCTGCATTCGAAGCCGAAGTCTACAGTTGGTAC
TCCAGCATACATAGCGCCAGAAGTTCTTTCAAGAGAAGAATATGATGGCAAGGTAGCAGAC
GTTTGGTCCTGTGGTGTGACACTGTACGTGATGCTTGTCGGTTCATACCCGTTTGAAGATC
CAGGTGATCCGAGGAATTTCCGCAAAACGATCAGCAGAATTCTTGGCGTGCAATACTCCAT
CCCGGACTACGTGAGGGTGTCTTCCGACTGCAGGCGCCTTCTATCTCAAATATTTGTTGCC
GATCCTTCAAAGAGGATCACGATCCCTGAGATAAAGAAGCACACGTGGTTTCTGAAGAATC
TGCCAAAGGAGATATCGGAGAGGGAGAAGGCCGACTACAAGGACACGGACGCCGCCCCTCC
GACGCAGGCCGTCGAGGAGATCATGCGGATCATCCAGGAGGCCAAGGTCCCCGGCGACATG
GCCGCCGCCGACCCGGCGCTGCTCGCGGAGCTCGCCGAGCTGAAGAGCGACGACGAAGAGG
AGGCCGCCGATGAGTATGACACCTACTGA

**FIGURE 3 (continued)**

**SEQ ID NO 30: *Oryza sativa* SAPK3, deduced protein sequence, BAD17999**
MEERYEALKELGAGNFGVARLVRDKRSKELVAVKYIERGKKIDENVQREIINHRSLRHPNI
IRFKEVCLTPTHLAIVMEYAAGGELFEQICTAGRFSEDEARYFFQQLISGVSYCHSLEICH
RDLKLENTLLDGSPTPRVKICDFGYSKSALLHSKPKSTVGTPAYIAPEVLSREEYDGKVAD
VWSCGVTLYVMLVGSYPFEDPGDPRNFRKTISRILGVQYSIPDYVRVSSDCRRLLSQIFVA
DPSKRITIPEIKKHTWFLKNLPKEISEREKADYKDTDAAPPTQAVEEIMRIIQEAKVPGDM
AAADPALLAELAELKSDDEEEAADEYDTY

**SEQ ID NO 31: *Oryza sativa* SAPK4 mRNA,**
ATGGAGAAGTACGAGGCGGTGAGGGACATCGGGTCGGGGAACTTCGGGGTGGCGCGGCTGA
TGCGCAACCGCGAGACCCGCGAGCTCGTCGCCGTCAAGTGCATCGAGCGCGGCCACCGGAT
AGATGAGAATGTGTACAGGGAGATCATCAACCACCGCTCGCTGCGCCACCCCAACATCATT
CGCTTCAAGGAGGTGATACTGACGCCAACGCATCTTATGATTGTCATGGAGTTCGCAGCAG
GCGGGGAGCTGTTCGATCGAATCTGTGATCGTGGACGGTTCAGTGAGGATGAGGCCAGGTA
TTTCTTTCAGCAGCTGATCTGTGGAGTGAGCTACTGCCATCACATGCAAATATGCCATAGA
GATTTGAAGTTGGAGAATGTTCTCTTGGATGGCAGCCCAGCTCCACGGCTTAAGATATGTG
ATTTTGGCTACTCCAAGTCATCAGTATTGCATTCAAGACCCAAATCAGCAGTGGGGACGCC
AGCATATATCGCACCAGAGGTGCTATCCCGCCGTGAGTATGATGGAAAGCTTGCAGATGTA
TGGTCCTGTGGTGTGACTCTTTACGTCATGCTTGTGGGAGCCTACCCATTTGAAGACCAGG
ACGACCCCAAGAACATTCGCAAAACCATTCAGAGAATAATGTCAGTGCAATATAAGATACC
AGATTACGTCCACATATCTGCAGAATGCAAACAGCTTATTGCCCGCATTTTTGTCAACAAT
CCATTGAGGAGAATCACGATGAAGGAAATAAAGAGCCACCCGTGGTTCTTGAAGAACCTCC
CCAGGGAGCTCACGGAGACTGCGCAAGCCATGTACTACAGGAGGGACAACTCCGTGCCTTC
CTTCTCAGACCAGACCTCAGAAGAGATCATGAAGATTGTTCAAGAAGCAAGAACCATGCCG
AAATCATCCAGGACAGGCTACTGGAGCGACGCGGGTTCAGACGAGGAGGAGAAGGAAGAGG
AAGAGAGGCCAGAAGAGAACGAGGAAGAGGAGGAAGATGAGTACGATAAGAGGGTCAAAGA
GGTCCATGCGAGCGGGGAGCTCCGTATGAGCTCACTGCGCATATGA

**SEQ ID NO 32: *Oryza sativa* SAPK4, deduced protein sequence, BAD18000**
MEKYEAVRDIGSGNFGVARLMRNRETRELVAVKCIERGHRIDENVYREIINHRSLRHPNII
RFKEVILTPTHLMIVMEFAAGGELFDRICDRGRFSEDEARYFFQQLICGVSYCHHMQICHR
DLKLENVLLDGSPAPRLKICDFGYSKSSVLHSRPKSAVGTPAYIAPEVLSRREYDGKLADV
WSCGVTLYVMLVGAYPFEDQDDPKNIRKTIQRIMSVQYKIPDYVHISAECKQLIARIFVNN
PLRRITMKEIKSHPWFLKNLPRELTETAQAMYYRRDNSVPSFSDQTSEEIMKIVQEARTMP
KSSRTGYWSDAGSDEEKEEEERPEENEEEEDEYDKRVKEVHAS
GELRMSSLRI

**SEQ ID NO 33: *Oryza sativa* SAPK5 mRNA,**
ATGGAGAAATACGAGCCAGTTCGGGAGATCGGGGCGGGCAACTTCGGGGTAGCGAAGCTGA
TGCGGAACAAGGAGACGCGGGAGCTGGTGGCGATGAAGTTCATCGAGAGAGGGAACAGGAT
CGACGAGAACGTGTTCCGGGAGATCGTGAATCATCGTTCGCTGCGTCACCCGAACATAATA
AGGTTCAAGGAGGTGGTGGTGACGGGGAGGCATCTGGCGATCGTGATGGAGTACGCGGCGG
GAGGGGAGCTGTTCGAGAGGATATGCGAGGCGGGGAGGTTCCACGAGGACGAGGCGCGCTA
CTTCTTCCAGCAGCTGGTGTGCGGGGTGAGCTACTGCCACGCCATGCAGATCTGCCACCGC

**FIGURE 3 (continued)**

GACCTCAAGCTGGAGAATACGCTGCTGGACGGCAGCCCGGCCCCGCGCCTCAAGATCTGCG
ACTTCGGCTACTCCAAGTCCTCCCTCCTCCACTCCCGCCCCAAATCCACCGTCGGCACCCC
CGCCTACATCGCCCCCGAGGTCCTCTCCCGCCGCGAGTACGACGGCAAGCTCGCCGACGTC
TGGTCCTGCGGCGTCACCCTCTACGTCATGCTCGTCGGCGCTTACCCTTTCGAGGATCCCA
AGGACCCCAAGAACTTCAGAAAGACCATCTCGCGCATCATGTCCGTCCAGTACAAGATCCC
CGAGTACGTCCACGTCTCCCAGCCCTGCCGCCACCTCCTCTCCCGCATCTTCGTCGCCAAC
CCCTACAAGCGCATCAGCATGGGCGAGATCAAGAGCCACCCCTGGTTCCTCAAGAACCTGC
CGCGCGAGCTCAAGGAGGAGGCGCAGGCCGTCTACTACAACCGCCGGGGAGCCGATCACGC
GGCTTCCAGCGCAAGTAGTGCGGCTGCTGCAGCTGCCTTCTCGCCGCAGAGCGTGGAGGAC
ATCATGAGGATCGTGCAGGAGGCGCAGACCGTCCCCAAGCCCGACAAGCCCGTCTCTGGCT
ACGGCTGGGGCACCGACGACGACGACGACGACCAACAACCAGCTGAGGAGGAGGACGAAGA
AGACGACTACGACAGGACGGTGCGCGAGGTTCACGCCAGCGTCGACCTCGACATGTCAAAC
CTCCAAATCTCCTGA

**SEQ ID NO 34: *Oryza sativa* SAPK5, deduced protein sequence, BAD18001**
MEKYEPVREIGAGNFGVAKLMRNKETRELVAMKFIERGNRIDENVFREIVNHRSLRHPNII
RFKEVVVTGRHLAIVMEYAAGGELFERICEAGRFHEDEARYFFQQLVCGVSYCHAMQICHR
DLKLENTLLDGSPAPRLKICDFGYSKSSLLHSRPKSTVGTPAYIAPEVLSRREYDGKLADV
WSCGVTLYVMLVGAYPFEDPKDPKNFRKTISRIMSVQYKIPEYVHVSQPCRHLLSRIFVAN
PYKRISMGEIKSHPWFLKNLPRELKEEAQAVYYNRRGADHAASSASSAAAAAAFSPQSVED
IMRIVQEAQTVPKPDKPVSGYGWGTDDDDDDQQPAEEEDEEDDYDRTVREVHASVDLDMSN
LQIS

**SEQ ID NO 35: *Oryza sativa* SAPK6 mRNA,**
ATGGAGAAGTACGAGCTGCTCAAGGACATCGGGTCGGGCAACTTCGGTGTGGCGCGGCTGA
TGCGGAACAGGGAGACCAAGGAGCTCGTCGCCATGAAGTACATACCGCGTGGCCTCAAGAT
TGACGAGAATGTGGCGAGGGAGATCATAAACCACCGCTCGCTGCGGCACCCAAACATCATC
CGGTTCAAGGAGGTCGTGCTCACGCCTACCCACCTCGCGATCGTCATGGAGTACGCCGCCG
GCGGCGAGCTGTTCGACCGGATCTGCAGCGCCGGGAGATTCAGCGAGGACGAGTCGAGGTA
TTTCTTCCAGCAACTAATTTGCGGCGTCAGCTACTGCCACTTCATGCAAATTTGCCACCGG
GATTTGAAGCTGGAGAACACGCTGCTGGATGGCAGCCCTGCGCCGCGCCTCAAGATCTGCG
ACTTTGGCTACTCCAAGTCATCACTGCTGCACTCAAAGCCGAAGTCGACGGTCGGGACTCC
CGCGTACATCGCTCCGGAGGTGCTCTCTCGCCGGGAGTATGACGGCAAGATGGCAGATGTA
TGGTCTTGTGGGGTGACCCTTTATGTGATGCTCGTCGGTGCTTACCCTTTTGAGGACCCAG
ATGATCCCAAGAATTTCAGAAAAACAATCGGGAGAATCGTATCAATTCAGTACAAAATACC
AGAGTACGTCCATATATCCCAAGATTGTAGACAACTCCTCTCTCGAATCTTTGTCGCGAAT
CCTGCAAAGAGAATAACAATAAGAGAGATCAGAAACCACCCTTGGTTTATGAAGAACTTGC
CGCGGGAGCTTACAGAAGCGGCGCAAGCGAAGTACTACAAGAAGGACAACAGTGCCCGTAC
ATTCTCGGATCAGACCGTCGACGAGATCATGAAGATTGTACAAGAGGCAAAGACACCACCT
CCATCGTCGACTCCAGTGGCCGGTTTCGGTTGGACCGAGGAAGAAGAGCAGGAGGACGGTA
AGAATCCCGACGACGACGAGGGAGACAGGGATGAGGAGGAGGGCGAGGAAGGCGATAGCGA
GGACGAGTACACCAAGCAGGTGAAGCAAGCCCATGCCAGCTGTGACTTGCAGAAGAGCTGA

**FIGURE 3 (continued)**

**SEQ ID NO 36: *Oryza sativa* SAPK6, deduced protein sequence, BAD18002**

MEKYELLKDIGSGNFGVARLMRNRETKELVAMKYIPRGLKIDENVAREIINHRSLRHPNII
RFKEVVLTPTHLAIVMEYAAGGELFDRICSAGRFSEDESRYFFQQLICGVSYCHFMQICHR
DLKLENTLLDGSPAPRLKICDFGYSKSSLLHSKPKSTVGTPAYIAPEVLSRREYDGKMADV
WSCGVTLYVMLVGAYPFEDPDDPKNFRKTIGRIVSIQYKIPEYVHISQDCRQLLSRIFVAN
PAKRITIREIRNHPWFMKNLPRELTEAAQAKYYKKDNSARTFSDQTVDEIMKIVQEAKTPP
PSSTPVAGFGWTEEEEQEDGKNPDDDEGDRDEEEGEEGDSEDEYTKQVKQAHASCDLQKS

**SEQ ID NO 37: *Oryza sativa* SAPK7 mRNA,**

ATGGAGAGGTACGAGCTGCTCAAGGACATCGGCGCCGGGAACTTCGGGGTGGCGCGGCTGA
TGCGGAATAAGGAGACCAAGGAGCTGGTCGCCATGAAGTACATCCCTCGGGGCCTCAAGAT
TGACGAGAATGTGGCGAGGGAGATCATCAACCACCGGTCGCTGCGGCACCCCAACATCATC
CGCTTCAAGGAGGTGGTGGTCACGCCGACGCACCTGGCGATCGTGATGGAGTACGCTGCCG
GCGGCGAGTTGTTCGACCGGATCTGCAACGCCGGGAGGTTCAGCGAGGACGAGGCCAGGTA
TTTCTTCCAGCAGCTCATCTGCGGCGTGAGCTACTGCCACTTCATGCAAATTTGCCACCGG
GATTTGAAGCTGGAGAACACGCTGCTGGACGGCAGCCCGGCGCCCCGCCTCAAGATCTGCG
ACTTCGGTTACTCCAAGTCGTCGCTGCTGCACTCGAAGCCCAAGTCGACGGTCGGGACGCC
GGCGTACATCGCGCCGGAGGTGCTATCCCGCCGGGAGTACGACGGCAAGACAGCCGATGTG
TGGTCTTGTGGAGTGACTCTTTATGTGATGCTTGTTGGTGCTTACCCCTTTGAGGACCCTG
ATGACCCCAAGAATTTCAGAAAGACCATTGGGAGAATAATGTCAATTCAGTACAAAATACC
CGAGTACGTCCATGTATCCCAGGACTGCAGGCAACTCCTTTCTAGAATTTTTGTTGCAAAC
CCTGCAAAGAGAATAACAATAAGGGAGATCAGGAACCACCCATGGTTCCTGAAGAACCTGC
CAAGAGAGCTCACAGAAGCTGCACAGGCAATGTACTACAAGAAGGATAACAGTGCCCCGAC
CTACTCCGTCCAGTCGGTCGAGGAGATCATGAAGATTGTCGAGGAAGCGCGGACGCCGCCT
CGGTCCTCCACCCCCGTGGCCGGCTTTGGCTGGCAAGAGGAGGATGAGCAGGAGGACAACA
GCAAGAAGCCAGAGGAAGAACAGGAGGAAGAGGAAGATGCTGAGGATGAGTACGACAAGCA
GGTGAAACAAGTCCATGCCAGTGGTGAGTTTCAGCTCAGCTGA

**SEQ ID NO 38: *Oryza sativa* SAPK7, deduced protein sequence, BAD18003**

MERYELLKDIGAGNFGVARLMRNKETKELVAMKYIPRGLKIDENVAREIINHRSLRHPNII
RFKEVVVTPTHLAIVMEYAAGGELFDRICNAGRFSEDEARYFFQQLICGVSYCHFMQICHR
DLKLENTLLDGSPAPRLKICDFGYSKSSLLHSKPKSTVGTPAYIAPEVLSRREYDGKTADV
WSCGVTLYVMLVGAYPFEDPDDPKNFRKTIGRIMSIQYKIPEYVHVSQDCRQLLSRIFVAN
PAKRITIREIRNHPWFLKNLPRELTEAAQAMYYKKDNSAPTYSVQSVEEIMKIVEEARTPP
RSSTPVAGFGWQEEDEQEDNSKKPEEEQEEEDAEDEYDKQVKQVHASGEFQLS

**SEQ ID NO 39: *Oryza sativa* SAPK8 mRNA,**

ATGGCAGCGGCGGGGGCCGGGGCGGGGGCGCCGGATCGGGCGGCGCTGACGGTGGGCCCGG
GGATGGACATGCCGATCATGCACGACAGCGACCGGTACGAGCTCGTGCGCGACATCGGCTC
CGGCAACTTCGGCGTCGCCCGCCTCATGCGCGACCGCCGCACCATGGAGCTCGTCGCCGTC
AAGTACATCGAGCGCGGCGAGAAGATAGATGATAATGTCCAGCGTGAGATTATAAATCACC
GATCGTTGAAACATCCTAACATTATTAGGTTTAAGGAGGTTATTTTAACCCCAACTCATCT
TGCTATTGTCATGGAATATGCCTCTGGTGGTGAGCTTTTCGAGAGAATTTGTAAGAATGTA

## FIGURE 3 (continued)

CGGTTCAGTGAAGATGAGGCTCGCTACTTCTTCCAGCAGCTTATCTCGGGAGTCAGCTACT
GCCATTCAATGCAAGTATGCCACCGTGATTTGAAGTTGGAGAATACACTGCTGGATGGAAG
CCCTGCTCCACGCTTGAAAATATGTGACTTTGGCTATTCTAAGTCTTCAGTTCTCCATTCA
CAACCAAAATCCACTGTAGGAACCCCTGCTTATATTGCACCTGAAGTTCTGTTGAAGAAAG
AATACGATGGCAAGACTGCTGATGTATGGTCCTGTGGTGTGACTCTATATGTTATGGTAGT
TGGTGCATATCCTTTCGAGGATCCAGAAGAGCCTAAGAACTTCCGTAAAACAATTCAGCGT
ATCTTGAATGTTCAGTACTCAATTCCAGAAAACGTGGACATATCTCCAGAATGTAGGCATC
TAATTTCGAGGATTTTTGTCGGGGATCCGTCTTTGAGGATAACAATCCCAGAAATACGGAG
CCATGGCTGGTTCTTGAAGAACCTTCCTGCAGATTTGATGGACGATGATAGTATGAGCAGC
CAGTATGAGGAACCTGATCAGCCAATGCAAACCATGGATCAGATCATGCAAATTTTAACTG
AGGCCACCATACCACCTGCTTGCTCTCGAATAAACCACATCCTAACTGATGGACTCGACCT
AGACGATGACATGGATGACCTCGATTCCGACTCAGATATTGATGTTGATAGCAGCGGCGAG
ATCGTCTATGCGATGTAA

**SEQ ID NO 40: *Oryza sativa* SAPK8, deduced protein sequence, BAD18004**
MAAAGAGAGAPDRAALTVGPGMDMPIMHDSDRYELVRDIGSGNFGVARLMRDRRTMELVAV
KYIERGEKIDDNVQREIINHRSLKHPNIIRFKEVILTPTHLAIVMEYASGGELFERICKNV
RFSEDEARYFFQQLISGVSYCHSMQVCHRDLKLENTLLDGSPAPRLKICDFGYSKSSVLHS
QPKSTVGTPAYIAPEVLLKKEYDGKTADVWSCGVTLYVMVVGAYPFEDPEEPKNFRKTIQR
ILNVQYSIPENVDISPECRHLISRIFVGDPSLRITIPEIRSHGWFLKNLPADLMDDDSMSS
QYEEPDQPMQTMDQIMQILTEATIPPACSRINHILTDGLDLDDDMDDLDSDSDIDVDSSGE
IVYAM

**SEQ ID NO: 41: *Oryza sativa* SAPK9 mRNA,**
ATGGAGAGGGCGGCGGCGGGGCCGCTGGGGATGGAGATGCCGATAATGCACGACGGTGACA
GGTACGAGCTGGTGAAGGAGATCGGGTCGGGGAACTTCGGCGTCGCCCGCCTCATGCGCAA
CCGCGCCTCCGGCGACCTCGTCGCCGTCAAGTACATCGACCGCGGCGAGAAGATTGACGAG
AACGTGCAGAGGGAGATCATCAACCACAGGTCGCTGCGCCACCCCAACATCATCCGATTCA
AGGAGGTTATTCTGACGCCGACGCATCTCGCGATCGTCATGGAGTACGCCTCCGGCGGCGA
GCTCTTCGAGCGCATCTGCAGCGCCGGCCGCTTCAGCGAGGACGAGGCTCGTTTCTTCTTC
CAGCAGCTGATATCTGGAGTTAGCTACTGCCATTCCATGCAAGTATGCCATCGTGACTTAA
AGCTGGAGAACACTCTGCTAGATGGAAGTACTGCTCCTCGCTTGAAGATATGTGACTTTGG
TTACTCGAAGTCATCGGTTCTTCATTCACAACCAAAATCAACAGTTGGAACTCCAGCTTAT
ATTGCTCCAGAAGTTTTGCTCAAGAAAGAATACGATGGAAAGATTGCCGATGTTTGGTCAT
GCGGTGTGACGCTCTACGTGATGTTGGTTGGCGCATACCCTTTCGAGGATCCTGAAGATCC
CAAGAACTTCAGAAAGACAATTCAGAAAATATTGGGTGTTCAGTACTCAATTCCAGACTAT
GTCCACATATCTCCGGAGTGCCGCGATCTCATTACGAGGATTTTTGTTGGCAACCCAGCTA
GTAGGATCACCATGCCTGAGATAAAGAACCACCCATGGTTCATGAAGAACATCCCGGCTGA
CCTCATGGATGATGGCATGGTTAGCAATCAGTACGAGGAGCCTGACCAGCCGATGCAGAAT
ATGAACGAGATCATGCAGATACTGGCAGAAGCAACAATTCCAGCAGCAGGCACCAGTGGAA
TCAATCAGTTCTTGACTGACAGCCTTGACCTCGACGACGACATGGAGGATATGGACTCGGA
CCTTGACCTTGACATTGAGAGCAGCGGAGAGATCGTATATGCCATGTAA

**FIGURE 3 (continued)**

**SEQ ID NO 42: *Oryza sativa* SAPK9, deduced protein sequence, BAD18005**

MERAAAGPLGMEMPIMHDGDRYELVKEIGSGNFGVARLMRNRASGDLVAVKYIDRGEKIDE
NVQREIINHRSLRHPNIIRFKEVILTPTHLAIVMEYASGGELFERICSAGRFSEDEARFFF
QQLISGVSYCHSMQVCHRDLKLENTLLDGSTAPRLKICDFGYSKSSVLHSQPKSTVGTPAY
IAPEVLLKKEYDGKIADVWSCGVTLYVMLVGAYPFEDPEDPKNFRKTIQKILGVQYSIPDY
VHISPECRDLITRIFVGNPASRITMPEIKNHPWFMKNIPADLMDDGMVSNQYEEPDQPMQN
MNEIMQILAEATIPAAGTSGINQFLTDSLDLDDDMEDMDSDLDLDIESSGEIVYAM

**SEQ ID NO 43: *Oryza sativa* SAPK10 mRNA,**

ATGGACCGGGCGGCGCTGACGGTGGGGCCGGGGATGGACATGCCGATAATGCACGACGGCG
ACCGGTACGAGCTGGTGCGGGACATCGGCTCCGGCAACTTCGGCGTCGCGCGCCTCATGCG
CAGCCGCGCCGACGGCCAGCTCGTCGCCGTCAAGTACATCGAGCGCGGCGACAAGATCGAC
GAGAACGTGCAGCGGGAGATCATCAACCACCGCTCGCTGCGCCACCCCAACATCATCCGCT
TCAAGGAGGTCATCCTCACCCCCACCCACCTCGCCATCGTCATGGAGTACGCCTCCGGCGG
CGAGCTCTTCGAGCGTATCTGCAACGCCGGCAGGTTCAGCGAGGACGAGGCACGGTTCTTT
TTCCAGCAACTGATTTCAGGAGTCAGCTATTGCCATTCCATGCAAGTATGCCATCGTGACC
TGAAGCTGGAGAACACCCTGCTCGACGGCAGCACGGCGCCTCGCCTCAAGATATGCGACTT
TGGCTATTCAAAGTCGTCTGTTCTTCATTCGCAACCAAAATCTACTGTTGGAACTCCGGCA
TACATCGCTCCTGAGGTTCTGCTGAAGAAGGAATATGATGGAAAGATTGCTGATGTGTGGT
CGTGTGGAGTAACCCTCTACGTAATGCTGGTTGGTGCATATCCTTTTGAGGATCCAGATGA
GCCTAAGAATTTCAGGAAGACAATTCAGAGAATATTGGGTGTGCAGTACTCTATTCCAGAT
TATGTCCACATATCTCCAGAGTGCCGAGATCTTATTGCGAGGATTTTTGTGGCCAACCCAG
CCACTAGAATCTCTATCCCCGAGATCAGAAATCATCCATGGTTCTTGAAGAATCTCCCAGC
TGACCTTATGGATGATAGCAAGATGAGCAGCCAGTACGAGGAGCCCGAACAGCCAATGCAG
AGCATGGATGAGATCATGCAGATACTGGCAGAGGCGACCATACCAGCAGCTGGGTCTGGTG
GAATCAACCAGTTCTTGAATGATGGCCTTGACCTCGATGATGACATGGAGGACCTTGATTC
AGACCCCGATCTTGACGTGGAAAGCAGTGGGGAGATAGTATACGCTATGTGA

**SEQ ID NO 44: *Oryza sativa* SAPK10, deduced protein sequence, BAD18006**

MDRAALTVGPGMDMPIMHDGDRYELVRDIGSGNFGVARLMRSRADGQLVAVKYIERGDKID
ENVQREIINHRSLRHPNIIRFKEVILTPTHLAIVMEYASGGELFERICNAGRFSEDEARFF
FQQLISGVSYCHSMQVCHRDLKLENTLLDGSTAPRLKICDFGYSKSSVLHSQPKSTVGTPA
YIAPEVLLKKEYDGKIADVWSCGVTLYVMLVGAYPFEDPDEPKNFRKTIQRILGVQYSIPD
YVHISPECRDLIARIFVANPATRISIPEIRNHPWFLKNLPADLMDDSKMSSQYEEPEQPMQ
SMDEIMQILAEATIPAAGSGGINQFLNDGLDLDDDMEDLDSDPDLDVESSGEIVYAM

**SEQ ID NO 45: *Brassica napus* BSK1 mRNA**

ATGGAGAAGTACGAGCTGGTGAAAGACATAGGAGCTGGGAATTTCGGAGTGGCGAGGCTCA
TGAAGGTCAAAGACTCTAAGGAGCTCGTTGCCATGAAGTACATCGAGCGTGGTCCCAAGAT
TGATGAGAACGTGGCAAGAGAGATTTATAATCACAGATCGCTTCGCCATCCTAATATTATC
CGCTTTAAGGAGGTGGTGTTGACTCCGACTCATCTTGCTATTGCCATGGAGTATGCTGCTG
GTGGTGAACTTTTCGAGCGTATATGCGGTGCTGGAAGATTCAGTGAGGATGAGGCGAGATA
CTTCTTCCAGCAGCTTATATCAGGTGTTAGCTATTGCCATGCTATGCAAATATGCCATAGA

## FIGURE 3 (continued)

GATCTGAAGCTCGAGAATACACTCCTTGATGGAAGTCCTGCTCCACGTCTCAAAATCTGTG
ATTTTGGTTATTCCAAGTCCTCTCTACTGCACTCGAGGCCTAAATCAACTGTTGGAACTCC
AGCATATATTGCACCTGAGGTCCTCTCTCGGAGAGAATATGATGGCAAGATGGCTGATGTA
TGGTCCTGTGGTGTTACTCTTTATGTCATGCTTGTTGGAGCATACCCTTTTGAAGACCAGG
AAGACCCCAAAAACTTCAGGAAAACAATACAAAAAATCATGGCTGTTCAGTACAAGATCCC
GGACTACGTCCACATCTCACAAGATTGCAAACATCTCCTTTCCCGTATATTTGTGGCCAAC
TCACTCAAGAGGATAACCATTGCGGAAATCAAGAAACACCCATGGTTCACGAAGAACTTGC
CAAGGGAGCTCACAGAGACAGCTCAAGCTGCATATTTCAAGAAAGAGAATCCAACCTTCTC
CGCCCAGACCGCTGAAGAGATCATGAAGATAGTGGATGACGCCAAAACGCCTCCGCCTGTT
TCCCGTTCCATTGGAGGTTTTGGCTGGGGAGGAGAGGGAGATTTAGAGGGGAAAGAGGAAG
AGGAGGTGGATGAAGAGGAGGTTGAGGAAGAGGAAGACGAAGAAGATGAATATGATAAGAC
TGTAAAGGAAGTACACGCAAGCGGAGAAGTGAGAATCAGTTGA

**SEQ ID NO 46: *Brassica napus* BSK1, deduced protein sequence, AAA33003**
MEKYELVKDIGAGNFGVARLMKVKDSKELVAMKYIERGPKIDENVAREIYNHRSLRHPNII
RFKEVVLTPTHLAIAMEYAAGGELFERICGAGRFSEDEARYFFQQLISGVSYCHAMQICHR
DLKLENTLLDGSPAPRLKICDFGYSKSSLLHSRPKSTVGTPAYIAPEVLSRREYDGKMADV
WSCGVTLYVMLVGAYPFEDQEDPKNFRKTIQKIMAVQYKIPDYVHISQDCKHLLSRIFVAN
SLKRITIAEIKKHPWFTKNLPRELTETAQAAYFKKENPTFSAQTAEEIMKIVDDAKTPPPV
SRSIGGFGWGGEGDLEGKEEEEVDEEEVEEEEDEEDEYDKTVKEVHASGEVRIS

**SEQ ID NO 47: *Brassica napus* BSK2 mRNA**
ATGGAGAAGTACGAGCTGGTGAAAGACATAGGCGCTGGGAATTTCGGAGTGGCGAGGCTCA
TGAAGGTCAAAAACTCTAAAGAGCTTGTTGCCATGAAGTACATCGAGCGTGGTCCCAAGAT
TGATGAGAATGTGGCAAGAGAGATCATTAATCACAGATCGCTTCGTCATCCTAATATTATC
CGTTTTAAGGAGGTTGTGTTGACTCCAACTCATCTTGCTATTGCCATGGAATATGCTGCTG
GTGGTGAATTATTCGAGCGTATATGCAGTGCTGGAAGATTCAGTGAGGATGAGGCGAGATA
CTTCTTCCAGCAGCTTATATCAGGTGTTAGCTATTGCCATGCTATGCAAATATGCCATAGA
GATCTGAAGCTCGAGAACACACTCCTGGATGGAAGTCCTGCTCCACGTCTCAAAATCTGTG
ATTTTGGTTATTCCAAGTCCTCTCTACTGCACTCGAGGCCCAAATCCACAGTTGGAACTCC
AGCATATATTGCACCTGAGGTCCTTTCTCGGAGAGAGTATGATGGCAAGATGGCTGATGTA
TGGTCTTGTGGTGTAACTCTTTATGTCATGCTTGTTGGAGCCTACCCATTCGAAGACCAGG
AAGACCCAAAGAACTTCAGGAAAACAATACAAAAAATCATGGCTGTTCAGTACAAGATCCC
GGACTACGTCCACATCTCACAGGATTGCAAACACCTCCTTTCCCGTATATTTGTTGCCAAT
TCACTCAAGAGGATAACCATTGCAGAAATCAAGAAACACCCATGGTTCCTGAAGAACCTGC
CAAGGGAGCTCACAGAGACAGCTCAAGCTGCATATTTCAAGAAAGAGAATCCAACCTTCTC
CCCGCAGACCGCTGAAGAGATCATGAAGATAGTGGATGACGCCAAAACGCCTCCGCCTGTT
TCCAGATCCATTGGAGGGTTTGGCTGGGGAGGAAAGGGAGACGAAGAGGAAGAAGAAGTGG
ATGAAGAGGAGGTGGTGGAGGAAGAGGAAGACGAAGAAGATGAATATGATAAGACTGTAAA
GGAAGCACACGCAAGTGGAGAAGTGTGA

**FIGURE 3 (continued)**

**SEQ ID NO 48: *Brassica napus* BSK2, deduced protein sequence, AAA33004**

MEKYELVKDIGAGNFGVARLMKVKNSKELVAMKYIERGPKIDENVAREIINHRSLRHPNII
RFKEVVLTPTHLAIAMEYAAGGELFERICSAGRFSEDEARYFFQQLISGVSYCHAMQICHR
DLKLENTLLDGSPAPRLKICDFGYSKSSLLHSRPKSTVGTPAYIAPEVLSRREYDGKMADV
WSCGVTLYVMLVGAYPFEDQEDPKNFRKTIQKIMAVQYKIPDYVHISQDCKHLLSRIFVAN
SLKRITIAEIKKHPWFLKNLPRELTETAQAAYFKKENPTFSPQTAEEIMKIVDDAKTPPPV
SRSIGGFGWGGKGDEEEEEVDEEEVVEEEEDEEDEYDKTVKEAHASGEV

**SEQ ID NO 49: *Glycine max* SPK-3 mRNA**

ATGGATAAGTATGAGGCTGTCAAGGATTTGGGAGCTGGGAATTTTGGGGTGGCTAGGCTCA
TGAGGAACAAGGAGACCAAGGAGCTTGTTGCCATGAAATACATCGAGCGTGGCCAAAAGAT
TGATGAGAATGTGGCAAGAGAGATTATCAACCACAGATCCCTTCGGCACCCCAATATAATT
CGCTTCAAGGAGGTGGTTTTGACCCCCACCCATTTGGCCATAGTGATGGAGTATGCGGCTG
GAGGAGAGCTCTTTGAGAGGATATGCAATGCTGGCAGGTTCAGTGAAGATGAGGCTAGATA
TTTCTTTCAGCAGCTGATTTCTGGTGTACATTACTGTCATGCCATGCAAATATGTCACAGA
GATTTGAAGCTAGAAAATACCCTTTTAGATGGAAGCCCTGCACCCCGCCTGAAAATTTGTG
ACTTTGGTTATTCCAAGTCATCATTACTTCATTCACGGCCAAAATCAACTGTTGGAACTCC
AGCTTATATAGCACCAGAGGTTCTTTCCAGGAGGGAGTATGATGGCAAGTTGGCTGATGTA
TGGTCATGTGGAGTGACTCTTTATGTCATGCTGGTTGGAGCTTATCCCTTTGAGGATCAGG
ATGACCCTAGGAATTTTAGGAAAACAATTCAGCGTATAATGGCTGTTCAATACAAAATCCC
TGATTATGTTCACATATCTCAAGACTGCAGACACCTCCTTTCTCGTATATTTGTAGCAAAT
CCATTAAGGAGGATCTCTCTTAAGGAAATTAAGAGCCACCCATGGTTTTTAAAGAATCTTC
CAAGAGAGCTGACTGAATCAGCTCAAGCTGTCTATTACCAGAGAGGCAATCCAAGCTTTTC
AATTCAAAGTGTGGAGGAGATCATGAAGATTGTGGGAGAGGCAAGGGACCCTCCTCCAGTA
TCTAGACCTGTCAAAGGTTTTGGCTGGGATGGCGAAGAAGATGAAGGGGAAGAAGACGTGG
AGGAAGAGGAGGACGAAGAAGACGAGTATGACAAGAGGGTCAAAGAGGTTCATGCAAGTGG
AGAATTTCAAATCAGTTAA

**SEQ ID NO 50: *Glycine max* SPK-3, deduced protein sequence, AAB68961**

MDKYEAVKDLGAGNFGVARLMRNKETKELVAMKYIERGQKIDENVAREIINHRSLRHPNII
RFKEVVLTPTHLAIVMEYAAGGELFERICNAGRFSEDEARYFFQQLISGVHYCHAMQICHR
DLKLENTLLDGSPAPRLKICDFGYSKSSLLHSRPKSTVGTPAYIAPEVLSRREYDGKLADV
WSCGVTLYVMLVGAYPFEDQDDPRNFRKTIQRIMAVQYKIPDYVHISQDCRHLLSRIFVAN
PLRRISLKEIKSHPWFLKNLPRELTESAQAVYYQRGNPSFSIQSVEEIMKIVGEARDPPPV
SRPVKGFGWDGEEDEGEEDVEEEEDEEDEYDKRVKEVHASGEFQIS

**SEQ ID NO 51: *Glycine max* SPK-4 mRNA**

ATGGATAAGTACGAGGCTGTTAAGGATTTGGGAGCTGGCAATTTTGGGGTGGCTAGGCTCA
TGAGGAACAAGGTCACCAAGGAGCTTGTAGCCATGAAATACATCGAGCGTGGCCCCAAGAT
TGATGAGAACGTGGCAAGGGAGATTATGAACCACAGGTCCCTTCGGCATCCCAATATAATT
CGTTACAAGGAGGTGGTTTTGACTCCCACACATTTAGCAATAGTGATGGAGTATGCAGCAG
GAGGAGAGCTCTTTGAGAGGATATGCAGTGCTGGCAGGTTCAGTGAAGATGAGGCTAGATA
TTTCTTTCAGCAGCTGATTTCCGGTGTTCATTTCTGTCATACCATGCAAATATGCCACAGA

**FIGURE 3 (continued)**

GATTTGAAGCTAGAAAATACCCTTCTAGATGGAAGTCCTGCACCTCGGTTGAAAATTTGTG
ACTTCGGTTATTCCAAGTCATCTTTGCTGCACTCACGACCCAAATCAACAGTTGGAACACC
AGCTTACATAGCACCGGAAGTTCTTTCTAGGCGAGAGTATGACGGAAAGTTGGCTGATGTA
TGGTCATGTGCGGTGACTCTTTATGTCATGCTGGTTGGAGCATATCCCTTTGAGGACCAGG
ATGACCCTAGGAATTTTAGGAAAACAATTCAGCGTATAATGGCTGTTCAATACAAAATCCC
TGATTATGTTCACATATCTCAAGATTGTAGGCACCTCCTCTCTCGTATATTTGTTGCAAAT
CCATTGAGGAGAATTACTATTAAGGAAATTAAGAATCACCCATGGTTTTTGAGGAATCTTC
CAAGGGAGCTAACTGAATCTGCTCAAGCTATCTATTACCAGAGAGACAGCCCAAACTTTCA
CCTTCAAAGTGTGGATGAGATAATGAAAATTGTAGGAGAGGCAAGAAATCCACCTCCAGTA
TCTAGGGCTCTCAAAGGTTTTGGCTGGGAAGGTGAAGAAGATTTGGATGAAGAAGTGGAGG
AAGAAGAGGATGAAGATGAGTATGATAAGAGGGTCAAAGAGGTTCATGCAAGTGGCGAATT
TCAAATTAGTTAA

**SEQ ID NO 52: *Glycine max* SPK-4, deduced protein sequence, AAB68962**
MDKYEAVKDLGAGNFGVARLMRNKVTKELVAMKYIERGPKIDENVAREIMNHRSLRHPNII
RYKEVVLTPTHLAIVMEYAAGGELFERICSAGRFSEDEARYFFQQLISGVHFCHTMQICHR
DLKLENTLLDGSPAPRLKICDFGYSKSSLLHSRPKSTVGTPAYIAPEVLSRREYDGKLADV
WSCAVTLYVMLVGAYPFEDQDDPRNFRKTIQRIMAVQYKIPDYVHISQDCRHLLSRIFVAN
PLRRITIKEIKNHPWFLRNLPRELTESAQAIYYQRDSPNFHLQSVDEIMKIVGEARNPPPV
SRALKGFGWEGEEDLDEEVEEEEDEDEYDKRVKEVHASGEFQIS

**SEQ ID NO 53: *Nicotiana tabacum* OSAK mRNA**
ATGGATAAATACGAGCTTGTGAAAGATATAGGGTCAGGGAATTTTGGTGTGGCAAGGCTGA
TGAGGCACAAGGAAACCAAAGAACTTGTGGCAATGAAATACATTGAGAGAGGACATAAGAT
TGATGAGAATGTAGCAAGGGAGATCATTAATCATAGATCGCTTCGGCATCCAAACATAATT
CGATTCAAGGAGGTGTTAGTGACTCCCACTCATCTTGCCATTGTTATGGAATATGCAGCTG
GTGGAGAACTGTTTGAGCGCATTTGCAATGCAGGAAGGTTTAGTGAAGATGAGGCTAGGTA
CTTTTTCCAGCAGCTTATTTCAGGAGTTCACTACTGTCACAACATGCAAATATGCCATAGA
GATTTGAAGCTGGAGAATACGCTTCTTGATGGAAGTCCAGCTCCACGCTTGAAGATATGTG
ATTTTGGATACTCAAAGTCGTCCCTGTTGCATTCGAGGCCAAAATCAACTGTTGGGACTCC
AGCTTATATTGCTCCTGAGGTCCTATCAAGAAGAGAATATGATGGCAAGCTGGCTGATGTT
TGGTCATGCGGGGTGACACTTTATGTGATGCTGGTTGGGGCATATCCTTTTGAAGATCAGG
AGGATCCGAAGAATTTTAGGAAAACTATTCAACGAATAATGGCGGTACAGTACAAGATTCC
CGACTATGTTCACATATCACAAGATTGTAGGCACCTTCTCTCTCGGATATTTGTTGCTAAT
CCAGCAAGGAGGATCACAATCAAAGAAATCAAGTCTCACCCATGGTTTTTGAAGAATTTGC
CGAGGGAATTAACAGAAGCAGCACAGGCAGCTTATTACAGAAGAGAAAACCCAACATTTTC
ACTTCAGAGTGTTGAGGAGATCATGAAAATTGTGGAAGAGGCAAAGACTCCCGCTCCAGCT
TCCCGTTCAGTCTCAGGCTTTGGCTGGGGAGGAGAAGAAGAAGAAGAGGAGAAGGAAGGAG
ATGTAGAAGAAGAGGAAGAGGATGAAGAAGAAGAAGACGAATATGAAAAGCAAGTGAAGCA
GGCACATGAAAGCGGAGAAGTTCGTCTCACCTAA

**FIGURE 3 (continued)**

**SEQ ID NO 54: *Nicotiana tabacum* OSAK, deduced protein sequence, AAL89456**
MDKYELVKDIGSGNFGVARLMRHKETKELVAMKYIERGHKIDENVAREIINHRSLRHPNII
RFKEVLVTPTHLAIVMEYAAGGELFERICNAGRFSEDEARYFFQQLISGVHYCHNMQICHR
DLKLENTLLDGSPAPRLKICDFGYSKSSLLHSRPKSTVGTPAYIAPEVLSRREYDGKLADV
WSCGVTLYVMLVGAYPFEDQEDPKNFRKTIQRIMAVQYKIPDYVHISQDCRHLLSRIFVAN
PARRITIKEIKSHPWFLKNLPRELTEAAQAAYYRRENPTFSLQSVEEIMKIVEEAKTPAPA
SRSVSGFGWGGEEEEEEKEGDVEEEEEDEEEEDEYEKQVKQAHESGEVRLT

**SEQ ID NO 55: rice GOS2 promoter**
aatccgaaaagtttctgcaccgttttcaccccctaactaacaatatagggaacgtgtgcta
aatataaaatgagaccttatatatgtagcgctgataactagaactatgcaagaaaaactca
tccacctactttagtggcaatcgggctaaataaaaaagagtcgctacactagtttcgtttt
ccttagtaattaagtgggaaaatgaaatcattattgcttagaatatacgttcacatctctg
tcatgaagttaaattattcgaggtagccataattgtcatcaaactcttcttgaataaaaaa
atctttctagctgaactcaatgggtaaagagagagatttttttaaaaaaatagaatgaag
atattctgaacgtattggcaaagatttaaacatataattatataattttatagtttgtgca
ttcgtcatatcgcacatcattaaggacatgtcttactccatcccaatttttatttagtaat
taaagacaattgacttatttttattatttatctttttttcgattagatgcaaggtacttacg
cacacactttgtgctcatgtgcatgtgtgagtgcacctcctcaatacacgttcaactagca
acacatctctaatatcactcgcctatttaatacatttaggtagcaatatctgaattcaagc
actccaccatcaccagaccacttttaataatatctaaaatacaaaaaataatttttacagaa
tagcatgaaaagtatgaaacgaactatttaggtttttcacatacaaaaaaaaaaaagaattt
tgctcgtgcgcgagcgccaatctcccatattgggcacacaggcaacaacagagtggctgcc
cacagaacaacccacaaaaaacgatgatctaacggaggacagcaagtccgcaacaaccttt
taacagcaggctttgcggccaggagagaggaggagaggcaaagaaaaccaagcatcctcct
cctcccatctataaattcctccccccttttcccctctctatataggaggcatccaagccaa
gaagagggagagcaccaaggacacgcgactagcagaagccgagcgaccgccttcttcgatc
catatcttccggtcgagttcttggtcgatctcttccctcctccacctcctcctcacagggt
atgtgcccttcggttgttcttggatttattgttctaggttgtgtagtacgggcgttgatgt
taggaaaggggatctgtatctgtgatgattcctgttcttggatttgggatagaggggttct
tgatgttgcatgttatcggttcggtttgattagtagtatggttttcaatcgtctggagagc
tctatggaaatgaaatggtttagggtacggaatcttgcgattttgtgagtaccttttgttt
gaggtaaaatcagagcaccggtgattttgcttggtgtaataaaagtacggttgtttggtcc
tcgattctggtagtgatgcttctcgatttgacgaagctatcctttgtttattccctattga
acaaaaataatccaactttgaagacggtcccgttgatgagattgaatgattgattcttaag
cctgtccaaaatttcgcagctggcttgtttagatacagtagtccccatcacgaaattcatg
gaaacagttataatcctcaggaacaggggattccctgttcttccgatttgctttagtccca
gaattttttttcccaaatatcttaaaaagtcactttctggttcagttcaatgaattgattg
ctacaaataatgcttttatagcgttatcctagctgtagttcagttaataggtaatacccct
atagtttagtcaggagaagaacttatccgatttctgatctccattttaattatatgaaat
gaactgtagcataagcagtattcatttggattattttttttattagctctcaccccttcat
tattctgagctgaaagtctggcatgaactgtcctcaattttgttttcaaattcacatcgat
tatctatgcattatcctcttgtatctacctgtagaagtttctttttggttattccttgact
gcttgattacagaaagaaatttatgaagctgtaatcgggatagttatactgcttgttctta
tgattcatttcctttgtgcagttcttggtgtagcttgccactttcaccagcaaagttc

**FIGURE 3 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9805760 A
- WO 0102541 A, Assmann and Li
- US 5811238 A
- US 6395547 A
- US 5565350 A, Kmiec
- US 9303868 W, Zarling
- EP 1198985 A1
- EP 04103421 A
- US 60589765 A


### Non-patent literature cited in the description


- **Anderberg ; Walker-Simmons.** *Proc. Natl. Acad. Sci. USA,* 1992, vol. 89, 10183-10187
- **Park et al.** *Plant Molecular Biology,* 1993, vol. 22, 615-624
- **Mustilli et al.** *Plant Cell,* 2002, vol. 14, 3089-3099
- **Kobyashi et al.** *Plant Cell,* 2004, vol. 16, 1163-1177
- **Schultz et al.** *Proc. Natl. Acad. Sci. USA,* 1998, vol. 95, 5857-5864
- **Letunic et al.** *Nucleic Acids Res,* 2002, vol. 30, 242-244, http://smart.embl-heidelberg.de
- **Mulder et al.** *Nucl. Acids. Res.,* 2003, vol. 31, 315-318, http://www.ebi.ac.uk/interpro
- A generalized profile syntax for biomolecular sequences motifs and its function in automatic sequence interpretation. **Bucher ; Bairoch.** ISMB-94; Proceedings 2nd International Conference on Intelligent Systems for Molecular Biology. AAAIPress, 1994, 53-61
- **Hulo et al.** *Nucl. Acids. Res.,* 2004, vol. 32, D134-D137, http://www.expasy.org/prosite
- **Bateman et al.** *Nucleic Acids Research,* 2002, vol. 30 (1), 276-280
- **Needleman ; Wunsch.** *J. Mol. Biol.,* 1970, vol. 48, 443-453
- **Smith ; Waterman.** *Advances in Applied Mathematics,* 1981, vol. 2, 482-489
- **Altschul, S.F. ; Gish, W. ; Miller, W. ; Myers, E.W. ; Lipman, D.J.** *J. Mol. Biol.,* 1990, vol. 215, 403-410
- **W. R. Pearson ; D. J. Lipman.** *Proc.Natl.Acad.Sci. USA,* 1988, vol. 85, 2444-2448
- **Ausubel et al.** Current Protocols in Molecular Biology. Current Protocols, 1994, vol. 1, 2
- *Biochimica et Biophysica Acta,* 1996, vol. 1314, 191-225
- **He et al.** *Science,* 2000, vol. 288, 2360-2363
- **Meinkoth ; Wahl.** *Anal. Biochem.,* 1994, vol. 138, 267-284
- **Sambrook et al.** Molecular Cloning: a laboratory manual. Cold Spring Harbor Laboratory Press, 2001
- Current Protocols in Molecular Biology. John Wiley & Sons, 1989
- **Hayashi et al.** *Science,* 1992, vol. 258, 1350-1353
- **Redei ; Koncz.** Methods in Arabidopsis Research. World Scientific, 1992, 16-82
- **Feldmann.** Arabidopsis. Cold Spring Harbor Laboratory Press, 1994, 137-172
- **Lightner ; Caspar.** Methods on Molecular Biology. Humana Press, 1998, vol. 82, 91-104
- **McCallum.** *Nature Biotechnol.,* 2000, vol. 18, 455-457
- **Stemple.** *Nature Rev. Genet.,* 2004, vol. 5, 145-150
- **Ausubel.** Current Protocols in Molecular Biology. Wiley
- **Castle et al.** *Science,* 2004, vol. 304 (5674), 1151-4
- **Offringa et al.** *EMBO J.,* 1990, vol. 9, 3077-3084
- **Terada.** *Nature Biotechnol.,* 2002, vol. 20, 1030-1034
- **Iida ; Terada.** *Curr. Opin. Biotechnol.,* 2004, vol. 15, 132-138
- **Creighton.** Proteins. W.H. Freeman and Company, 1984
- **Buchman ; Berg.** *Mol. Cell Biol.,* 1988, vol. 8, 4395-4405
- **Callis et al.** *Genes Dev.,* 1987, vol. 1, 1183-1200
- The Maize Handbook. Springer, 1994
- **Wu et al.** *J Biochem,* 1998, vol. 123 (3), 386-91
- **McElroy et al.** *Plant Cell,* 1990, vol. 2, 163-171
- **Odell et al.** *Nature,* 1985, vol. 313, 810-812

- **Nilsson et al.** *Physiol. Plant.,* 1997, vol. 100, 456-462
- **de Pater et al.** *Plant J,* November 1992, vol. 2 (6), 837-44
- **Christensen et al.** *Plant Mol. Biol.,* 1992, vol. 18, 675-689
- **Buchholz et al.** *Plant Mol Biol.,* 1994, vol. 25 (5), 837-43
- **Lepetit et al.** *Mol. Gen. Genet.,* 1992, vol. 231, 276-285
- **An et al.** *Plant J.,* 1996, vol. 10 (1), 107-121
- **Krens et al.** *Nature,* 1982, vol. 296, 72-74
- **Negrutiu et al.** *Plant Mol. Biol.,* 1987, vol. 8, 363-373
- **Shillito et al.** *Bio/Technol,* 1985, vol. 3, 1099-1102
- **Crossway et al.** *Mol. Gen. Genet.,* 1986, vol. 202, 179-185
- **Klein et al.** *Nature,* 1987, vol. 327, 70
- **Aldemita ; Hodges.** *Planta,* 1996, vol. 199, 612-617
- **Chan et al.** *Plant Mol. Biol.,* 1993, vol. 22, 491-506
- **Hiei et al.** *Plant J.,* 1994, vol. 6, 271-282
- **Ishida et al.** *Nature Biotechnol.,* 1996, vol. 14, 745-50
- **Frame et al.** *Plant Physiol.,* 2002, vol. 129, 13-22
- **Lander et al.** *Genomics,* 1987, vol. 1, 174-181
- **Botstein et al.** *Am. J. Hum. Genet.,* 1980, vol. 32, 314-331
- **Bematzky ; Tanksley.** *Plant Mol. Biol. Reporter,* 1986, vol. 4, 37-41
- **Hoheisel et al.** Nonmammalian Genomic Analysis: A Practical Guide. Academic press, 1996, 319-346
- **Trask.** *Trends Genet.,* 1991, vol. 7, 149-154
- **Laan et al.** *Genome Res.,* 1995, vol. 5, 13-20
- **Kazazian.** *J. Lab. Clin. Med.,* 1989, vol. 11, 95-96
- **Sheffield et al.** *Genomics,* 1993, vol. 16, 325-332
- **Landegren et al.** *Science,* 1988, vol. 241, 1077-1080
- **Sokolov.** *Nucleic Acid Res.,* 1990, vol. 18, 3671
- **Walter et al.** *Nat. Genet.,* 1997, vol. 7, 22-28
- **Dear ; Cook.** *Nucleic Acid Res.,* 1989, vol. 17, 6795-6807
- **Sambrook.** Molecular Cloning: a laboratory manual. Cold Spring Harbor Laboratory Press, 2001
- Current Protocols in Molecular Biology. Current Protocols, 1994, vol. 1, 2
- **R.D.D. Croy.** Plant Molecular Biology Labfax. BIOS Scientific Publications Ltd (UK) and Blackwell Scientific Publications (UK), 1993